# EUROPEAN PATENT APPLICATION

(11) **EP 3 868 893 A1**
(43) Date of publication of application: **25.08.2021**
(21) Application number: 20158577.5
(22) Date of filing: 20.02.2020
(51) Int. Cl.: C12Q 1/68, B82Y 5/00, B82Y 30/00, A61K 47/54

(54) **PROGRAMMABLE SHELLS FOR VIRUS ENCAPSULATION**

(71) Applicant: Technische Universität München, 80333 München (DE)
(72) Inventor: Dietz, Hendrik, 85540 Haar (DE)
(74) Representative: Virnekäs, Bernhard

(57) **Abstract**

The present invention relates to a macromolecule-based nanostructure, such as a DNA-based nanostructure, for encapsulating a virus or viral particle, to a composition comprising a virus or viral particle encapsulated by such a macromolecule-based nanostructure according to the present invention, and to a method for encapsulating a virus or viral particle by using such a macromolecule-based nanostructure

## Description

### FIELD OF THE INVENTION

The present invention relates to a macromolecule-based nanostructure, such as a DNA-based nanostructure, for encapsulating a virus or viral particle, to a composition comprising a virus or viral particle encapsulated by such a macromolecule-based nanostructure according to the present invention, and to a method for encapsulating a virus or viral particle by using such a macromolecule-based nanostructure.

### BACKGROUND OF THE INVENTION

Viral infections cause millions of deaths per year globally, enormous suffering and morbidity, and impose huge drains on societies and economies in health care costs, lost work time, and other less easily measured burdens such as mental health issues associated with loss of parents, children, and care givers or stigmatization. Climate change and global migration are projected to increase the threat of viral outbreaks because vectors spread to regions that so far were too cold for them to survive. The burden of virus infections will further increase due to habitat encroachment by humans, urbanization and megacities with increasing population density, increasing travel not only locally but also far distance, and numerous other drivers of disease emergence (1). Viruses are the pathogen class most likely to adapt to new environmental conditions because of their short generation time and genetic variability allowing rapid evolution (2). For the majority of viral diseases (∼70% of current WHO-listed viruses), no effective treatment is available. The few existing antiviral therapies are almost exclusively targeted to a specific virus and do not allow application against a newly emerging pathogen. In addition, antiviral therapy typically faces the challenge that it must be started very soon after infection to be effective, before the viral load gets too high and caused disease symptoms. Emerging virus threats require a rapid response, but broadly applicable ready-to-use antivirals do not exist.

In this context, it is useful to first consider how current antiviral therapies work. Existing antiviral drugs target either virus-specific proteins, mostly polymerases, or essential virus or cellular structures that enable virus replication and spread. The major targetable steps in a virus replication cycle are (1) virus particles docking to the cell membrane of host cells; (2) uptake into the host cell; (3) release of the virus capsid into the cytoplasm and transport of the viral genome to the replication spot; (4) synthesis of viral nucleic acids and proteins and posttranslational processing of viral proteins; (5) assembly of virus components into new viral particles; (6) release of the newly formed viruses from the infected cell. Most clinically available antivirals are polymerase-inhibitors that are specific for a given viral enzyme. Examples include acyclovir (3), active against herpes simplex and varizella zoster virus; tenofovir, active against hepatitis B virus (HBV) and HIV and sofosbuvir, active against hepatitis C virus (HCV). Examples for drugs targeting different stages of the virus life cycle are: enfuvirtide (4), which inhibits HIV fusion (stage 2); amantadine (5), which inhibits influenza A virus uncoating (stage 3); or the neuraminidase inhibitor oseltamivir (6), which interferes with influenza virus release from host cells (stage 6) (6). These drugs, however, can only act when a virus is replicating or spreading but cannot kill or neutralize it. None of these antivirals is broadly applicable.

Recently, a star-shaped designer DNA nanostructure has been presented as a template to display multiple binding motifs with spatial pattern-recognition properties, which confers sensing and viral inhibitory capabilities for a dengue (DENV) viral surface (56). While it is stated that the molecular-platform design strategy could be adapted to detect and combat other disease-causing pathogens by generating the requisite ligand patterns on customized DNA nanoarchitectures, it is also stated that a defined DNA nanoarchitecture with precise, multivalent spatial pattern-recognizing properties is required, since without the optimal shape identity to keep the required hairpin interactions from forming, sensing and inhibitory abilities suffer. Thus, this proposal does not appear to provide a generically applicable approach for targeting different or yet uncharacterized virus particles, such as needed in case of the emergence of a new virus, as seen in these days with the Coronavirus. Additionally, the rather open structure of the nanostructures bears the danger that viral capsid proteins may still be able to protrude, to be immunologically active and/or to trigger the viral infection process.

Thus, while different strategies for the treatment of viral infections have been developed or suggested up to date, there is still a need for the development of a concept of a generic antiviral drug platform for targeting a variety of viral pathogens. In particular, a concept would be highly desirous that does not rely on prior detailed knowledge about genetics and properties of the target virus.

### SUMMARY OF THE INVENTION

It is an object of the present invention to provide constructs that enable the encapsulation of a virus or viral particle. The solution to that problem, i.e. the use of macromolecular building blocks, such as DNA-based nanostructures, has not yet been taught or suggested by the prior art.

Therefore, in one aspect, the disclosure provides a macromolecule-based nanostructure encasing a cavity with a diameter of at least 20 nm for encapsulating a virus or viral particle.

In a particular embodiment, the disclosure provides a macromolecule-based nanostructure, which is a DNA-based nanostructure.

In another aspect, the disclosure provides a composition comprising a virus or viral particle encapsulated by a macromolecule-based nanostructure according to the present invention.

In yet another aspect, the disclosure provides a method for encapsulating a virus or viral particle, comprising the steps of: providing a macromolecule-based nanostructure according to the present invention, and contacting said macromolecule-based nanostructure with a medium comprising, or suspected to comprise, said virus or viral particle.

The disclosure contemplates all combinations of any one or more of the foregoing aspects and/or embodiments, as well as combinations with any one or more of the embodiments set forth in the detailed description and examples.

Other features, objects, and advantages of the compositions and methods herein will be apparent from the description and drawings, and from the claims.

### BRIEF DESCRIPTION OF THE DRAWINGS

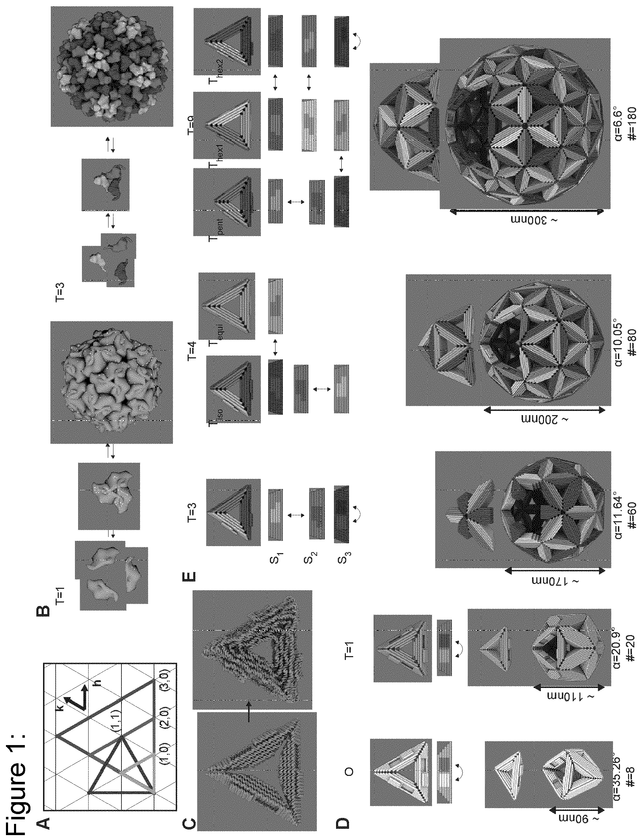

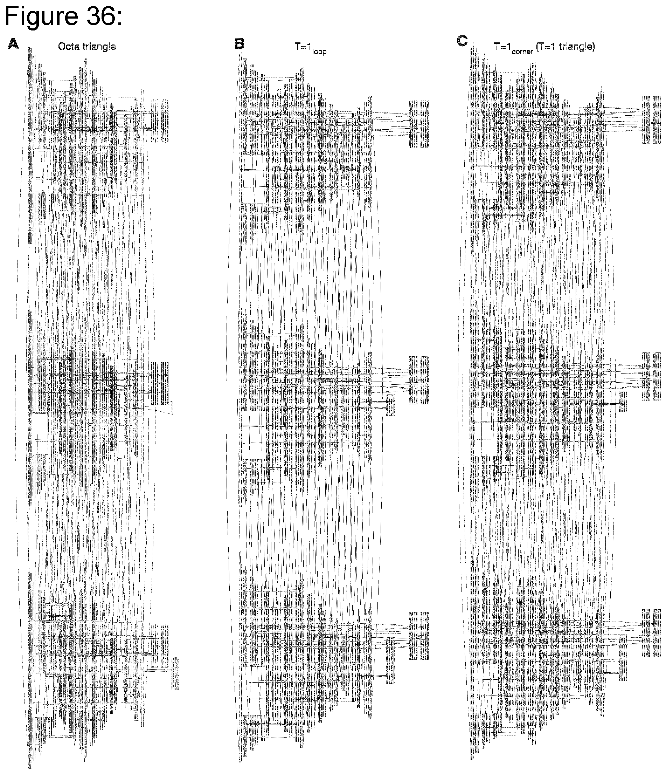

**Figure 1** shows the design principles. (A) Triangulation number of icosahedral shells. Each colored triangle represents one of the 20 faces forming an icosahedron. The small triangles represent the triangular building blocks. (h,k) indicates the location of pentamers within a shell. (B) Natural icosahedral virus shells. Left, T=1 shell built out of identical subunits (MVM) (22). Right, T=3 shell built out of multiple copies of three different subunits (CCMV) (23). (C) ENRG-MD simulation (38) of an exemplary shell subunit design prototype at the start (left) and end (right) of the simulation. (D and E) Cylindrical model of DNA-origami triangles and the corresponding shells. The sides of the triangles are modified with protrusions (light) and recesses (dark). The arrows indicate shape-complementary sides. For each shell design, one of its 20 icosahedral faces has been displaced (see (A)). α is the bevel angle of the sides, # the number of DNA-origami triangles building the shell (design details see Figs. 36 to 39).

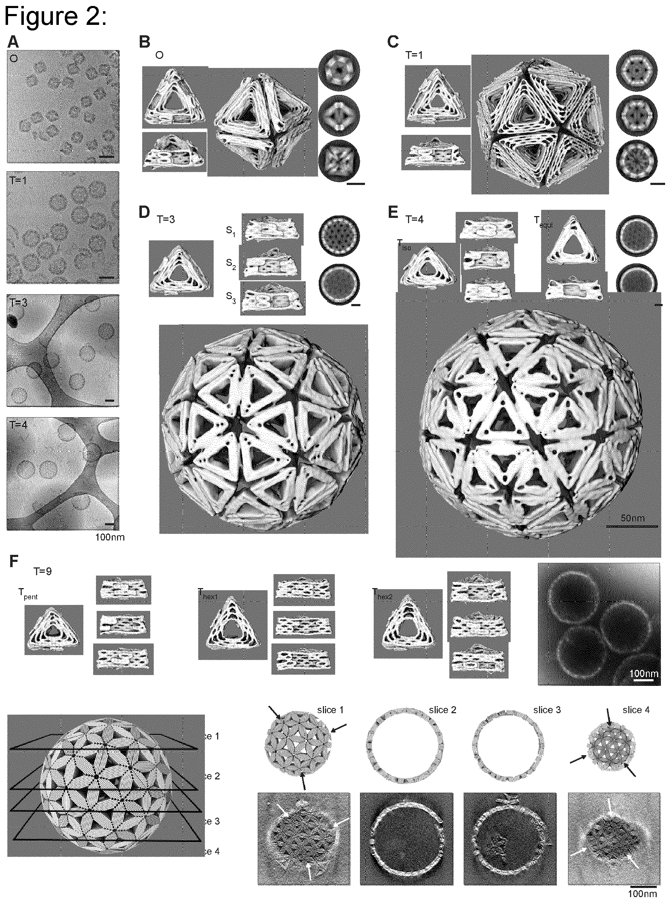

**Figure 2** shows the structures of shells and of shell subunits. (A) Cryo-EM micrographs of assembled shells in free-standing ice (O, T=1) and on lacey carbon grids with carbon support (T=3, T=4). (B to E) Cryo-EM reconstructions of shell subunits and fully assembled shells (octahedron to T=4 shells). The two-dimensional class averages show assembled shells from different orientations. (F) EM validation of the T=9 shell design. Top left, cryo-EM reconstructions of the three triangles assembling into a T=9 shell. Top right, negatively stained EM micrograph of assembled shells. Bottom, comparison of slices through a model shell to slices of a tomogram calculated from an EM tilt series. The arrows indicate the positions of pentamers within the shell.

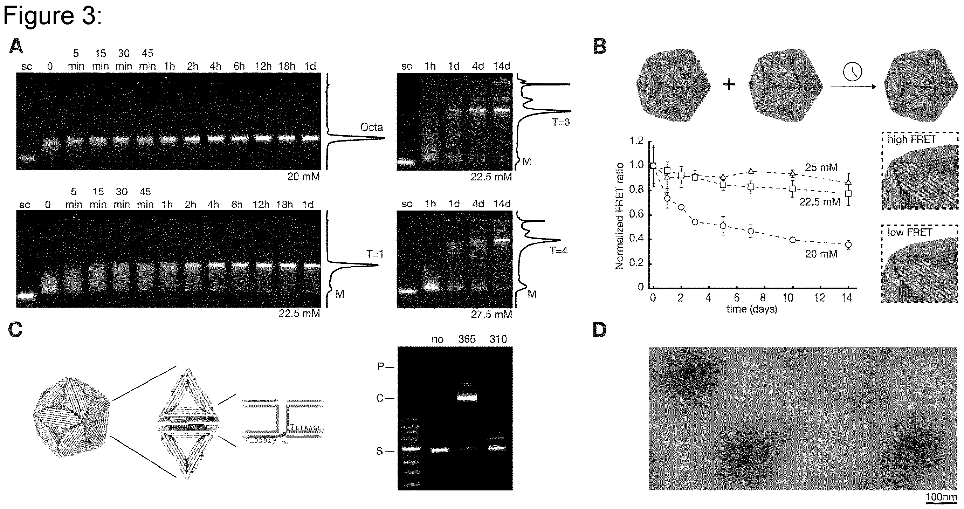

**Figure 3** shows shell yield and stability. (A) Laser-scanned fluorescent images of 0.5% agarose gels showing the assembly of octahedra, T=1, T=3 and T=4 shells at 40°C with a monomer concentration of 5 nM at different time points. Solid lines give cross-sectional lane intensity profiles from the 1d samples. (B) Triangle exchange experiments. Cyan: FRET-pair labeled T=1 shells. Orange: unlabeled shells. Symbols give FRET signals measured vs time of incubation in the presence of the indicated concentrations of Mg²⁺. Errors bars are SEM of duplicate measurements (design details see Fig. 44). (C) Left: schematics of covalent stabilization of shells using CNVK moieties placed at base-pair stacking sites. Right: Laser-scanned fluorescent image of 0.5% agarose gel on which shell samples were run under shell-destabilizing conditions (low salt, 5 mM Mg²⁺). From left to right: Marker lane, non-illuminated T=1 shell (disassembles), sample illuminated for 30 min with 365 nm light (stays intact), 365-illuminated sample additionally illuminated with 310 nm light for 1 min to reverse the covalent bonding (disassembles). S=subunits. C=Complete shells. P=pocket. (D) Negative-staining TEM image of octahedral shells coated with a 1:1 mixture of oligolysine and oligolysine-PEG and incubated for 1 h in 55 % mouse serum.

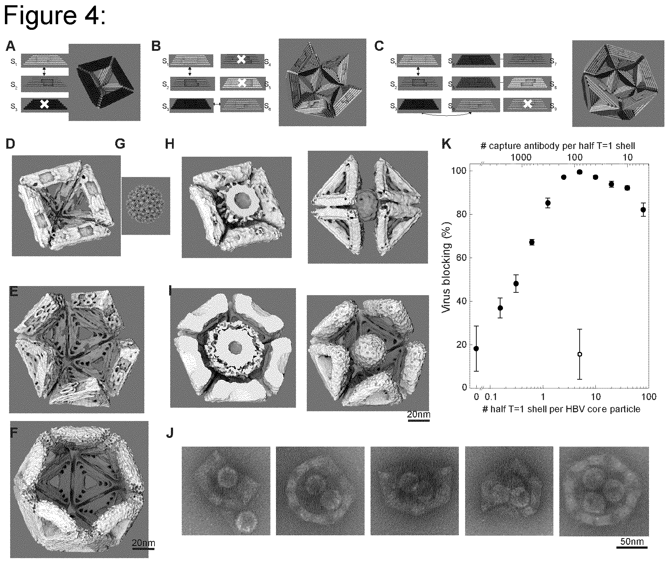

**Figure 4** shows the programming of partial shell assembly and trapping of a viral capsid (here: hepatitis B virus core particles (HBV). (A-C) Cylindrical models of DNA origami triangles and corresponding partial shells. The sides of the triangles are modified with protrusions and recesses. The arrows indicate shape-complementary sides. White crosses indicate deactivated interaction sites. (D-F) Cryo-EM 3D reconstructions of half-octahedral (D), half T=1 shells (E) and T=1 shells omitting a pentagon vertex (F). Arrows indicate density stemming from anchor sites for mounting virus-binding moieties. (G) Cryo-EM reconstruction of a HBV core particle (Electron Microscopy Data Bank identifier EMD-0271). (H) Left: Cut through the octahedral-DNA shell cryo EM map with the HBV core particle trapped. The density around the HBV core particle is attributed to the layer of antibodies connecting the HBV core particles to the octahedral shell. Right: Cryo-EM reconstruction of two octahedral half-shells coordinating a trapped hepatitis-B virus particle. (I) Left: Cut through the map similar as in (H). The electron density thresholds differ, which makes the HBV core particle look thicker in the T=1 half shell compared to when trapped in the half octahedron. Right: Cryo-EM reconstruction of half T=1 shell engulfing a HBV core particle. (J) Negative stain TEM images of T=1 shell with a missing pentagon vertex engulfing up to three HBV core particles. (K) In vitro virus blocking ELISA experiments of 2.5 pM HBV core particle incubated with pre-assembled mixtures of 1 nM oligonucleotide-conjugated capture antibody and various concentrations of half T=1 shells. Errors bars are standard deviation of triplicate measurements.

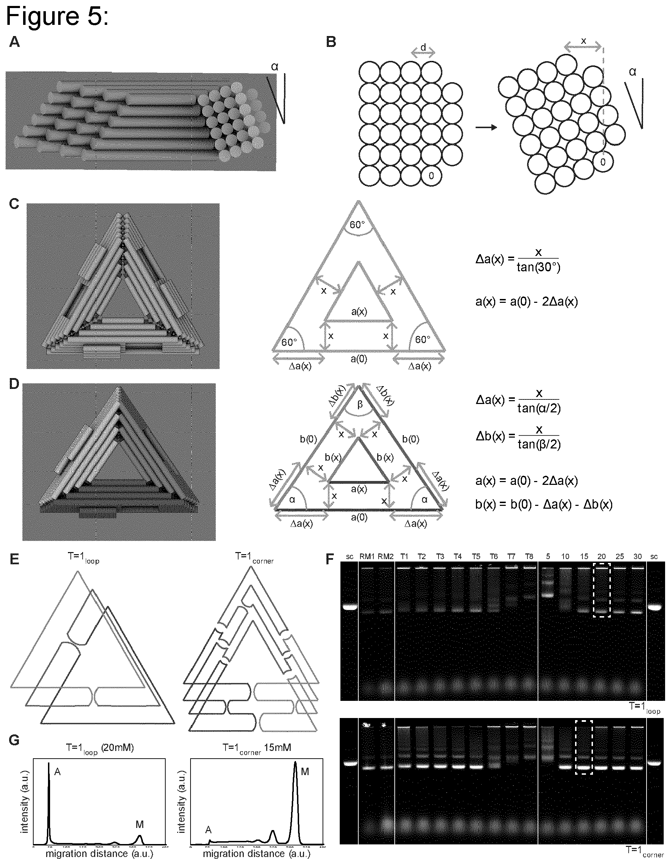

**Figure 5** shows design principle of triangular subunits. (A) Schematics of T=1 triangle designs with a bevel angle alpha. (B) Cross-section of a triangles side consisting of 4x6 helices in square-lattice packing without (left) and with (right) a bevel angle. The side is turned around the longest helix indicated by '0'. d is the distance between the center of two neighboring helices (2.6 nm) and x the radial distance of any helix to helix '0'. To transform nm in base pairs we used a rise of 0.34 nm per base pair. (C and D) Schematic illustrating the calculation of helix lengths. Left, cylindrical model of a triangle. Middle, schematics of the lengths a(x) and b(x) of different helices within the triangle depending on the distance x to helix '0'. Right, formulas to calculate the length differences of individual helices. (E) Scaffold routing through the triangular structure. Left, The scaffold forms a loop around the whole triangle before connecting to the next layer. Right, each corner is built by a certain scaffold domain (different blues) and is only connected by one cross-over to a neighboring corner (red). (F) 1.5% agarose gel containing 0.5xTBE buffer and 5.5 mM MgCl₂ showing initial folding screens according to (46). (G) Integrated lane profiles of the two lanes indicated by the dotted squares in (B). T=1ₗₒₒₚ forms a lot of aggregates and less well formed triangles. Changing the scaffold routing to T=1_{corner} clearly improves the folding yield. Subsequently, all triangles presented in this work are designed with the improved scaffold routing of T=1corner. A: malformed aggregates, M: correctly folded monomers.

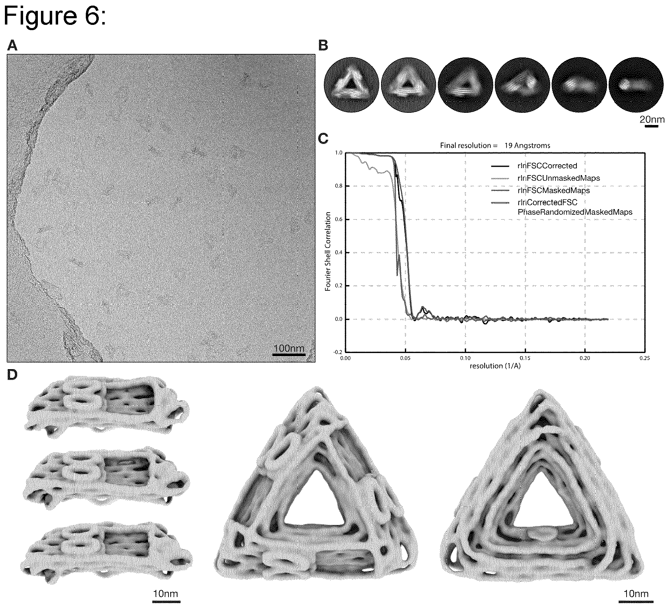

**Figure 6** shows cryo-EM reconstruction of the octahedron triangle. (A) Cryo-EM micrograph of the octahedron triangle in free-standing ice. (B) Two dimensional class averages showing different orientations. (C) Graph showing different FSC curves which were used for resolution estimation. (D) Electron density map shown from different viewing angles.

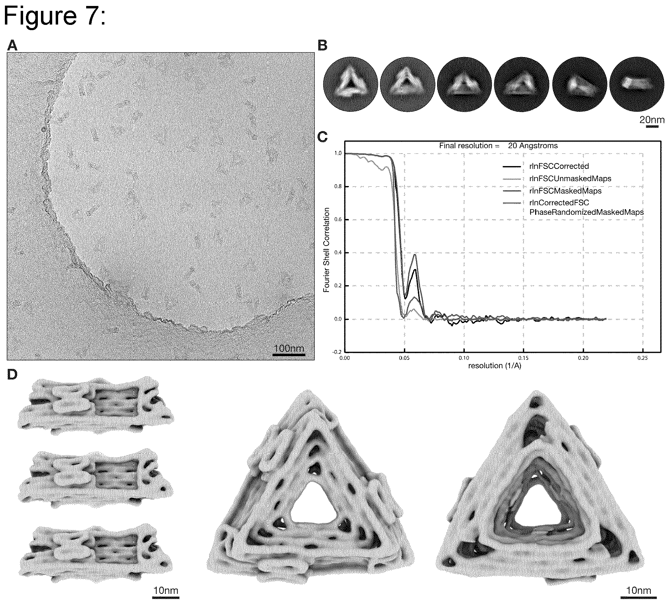

**Figure 7** shows cryo-EM reconstruction of the T=1 triangle. (A) Cryo-EM micrograph of the T=1 triangle in free-standing ice. (B) Two dimensional class averages showing different orientations. (C) Graph showing different FSC curves which were used for resolution estimation. (D) Electron density map shown from different viewing angles.

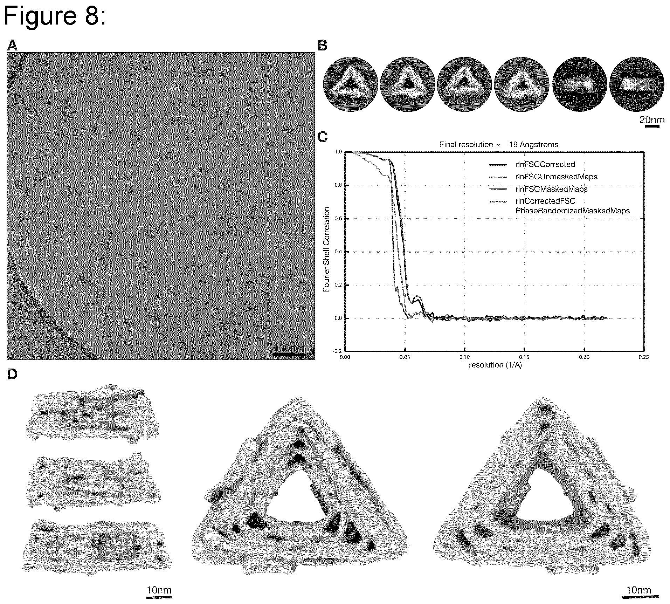

**Figure 8** shows cryo-EM reconstruction of the T=3 triangle. (A) Cryo-EM micrograph of the T=3 triangle in free-standing ice. (B) Two dimensional class averages showing different orientations. (C) Graph showing different FSC curves which were used for resolution estimation. (D) Electron density map shown from different viewing angles.

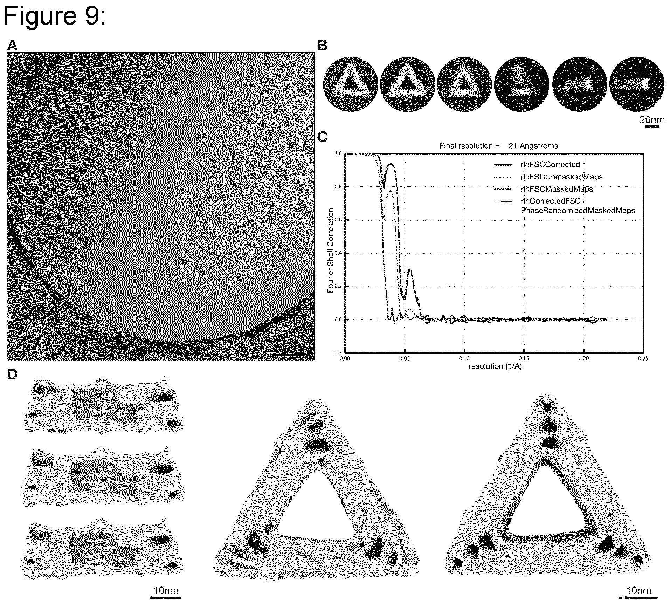

**Figure 9** shows cryo-EM reconstruction of the T=4 triangle T_{equi}. (A) Cryo-EM micrograph of the T_{equi} triangle in free-standing ice. (B) Two dimensional class averages showing different orientations. (C) Graph showing different FSC curves which were used for resolution estimation. (D) Electron density map shown from different viewing angles.

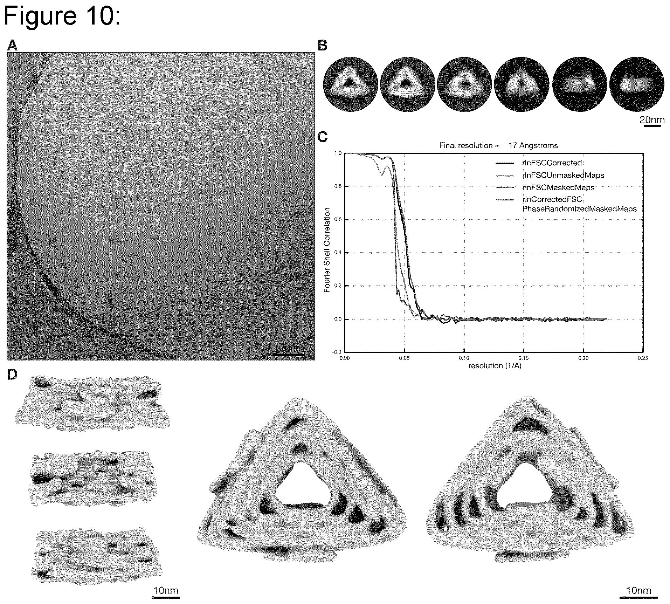

**Figure 10** shows Cryo-EM reconstruction of the T=4 triangle Tᵢₛₒ. (A) Cryo-EM micrograph of the Tᵢₛₒ triangle in free-standing ice. (B) Two dimensional class averages showing different orientations. (C) Graph showing different FSC curves which were used for resolution estimation. (D) Electron density map shown from different viewing angles.

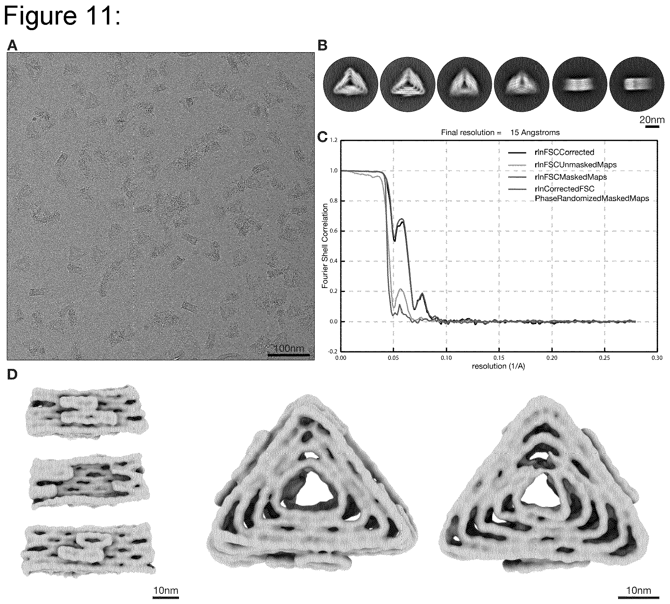

**Figure 11** shows Cryo-EM reconstruction of the T=9 triangle Tₚₑₙₜ. (A) Cryo-EM micrograph of the Tₚₑₙₜ triangle in free-standing ice. (B) Two dimensional class averages showing different orientations. (C) Graph showing different FSC curves which were used for resolution estimation. (D) Electron density map shown from different viewing angles.

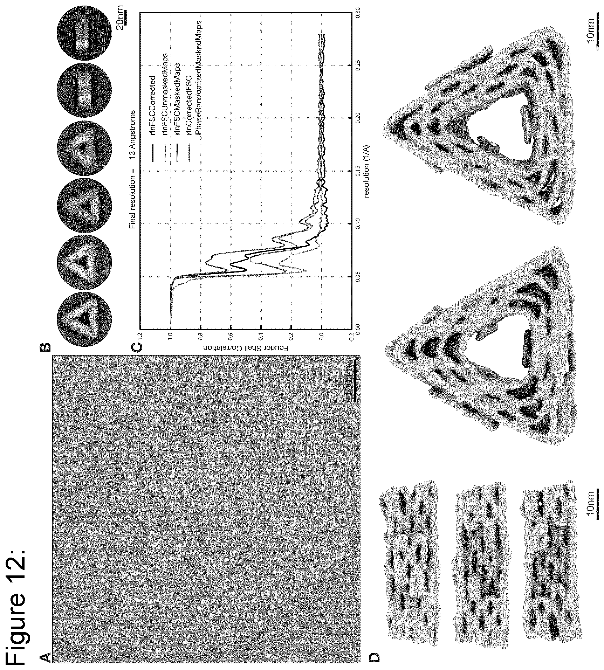

**Figure 12** shows Cryo-EM reconstruction of the T=9 triangle Tₕₑₓ₁. (A) Cryo-EM micrograph of the Tₕₑₓ₁ triangle in free-standing ice. (B) Two dimensional class averages showing different orientations. (C) Graph showing different FSC curves which were used for resolution estimation. (D) Electron density map shown from different viewing angles.

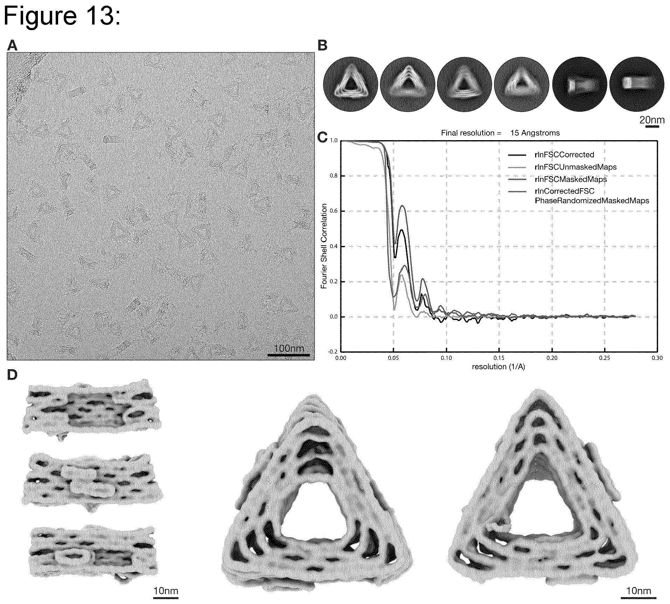

**Figure 13** shows Cryo-EM reconstruction of the T=9 triangle Tₕₑₓ₂. (A) Cryo-EM micrograph of the Tₕₑₓ₂ triangle in free-standing ice. (B) Two dimensional class averages showing different orientations. (C) Graph showing different FSC curves which were used for resolution estimation. (D) Electron density map shown from different viewing angles.

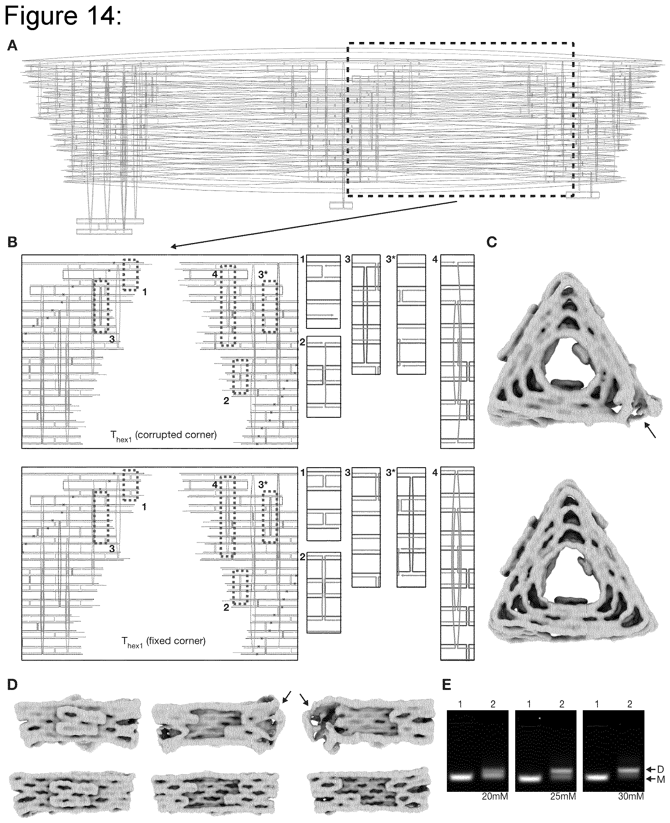

**Figure 14** shows Correction for folding defects in Tₕₑₓ₁. (A) Design diagram of the Tₕₑₓ₁ triangle with fixed corner. (B) Zoom-in on the location of the corrupted corner showing the differences between the two versions in the design diagram. (C and D) Cryo-EM map of the two Tₕₑₓ₁ triangle versions. The arrows indicate the location of the corrupted corner. (E) 1.5% agarose gels with different MgCl₂ concentrations showing the dimerization of Tₚₑₙₜ and Tₕₑₓ₁ triangles. All sides not involved in the binding of the two triangles were passivated with polyT extensions at the stacking contacts. While the corrupted corner prevents the two triangles to bind to each other because of missing stacking contacts, the triangle with fixed corners binds to Tₚₑₙₜ and assembles into dimers. Lane 1: Tₚₑₙₜ + Tₕₑₓ₁ (corrupted corner), Lane 2: Tₚₑₙₜ + Tₕₑₓ₁ (fixed corner). D: dimer, M: monomer.

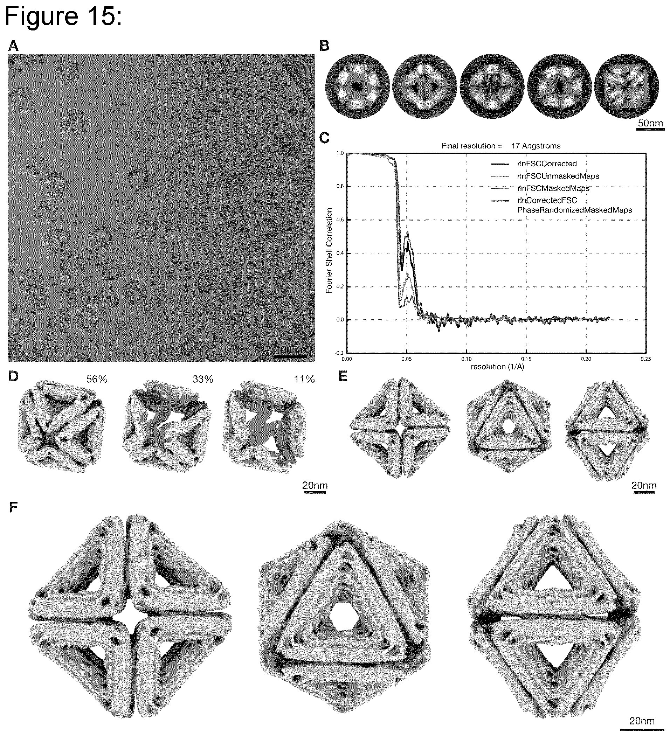

**Figure 15** shows Cryo-EM reconstruction of the assembled octahedron at 17.5 mM MgCl₂. (A) Cryo-EM micrograph of the octahedron in free-standing ice at 17.5 mM MgCl₂. (B) Two dimensional class averages showing different orientations. (C) Graph showing different FSC curves which were used for resolution estimation. (D) Classes after 3d classification in relion3. (E) Refinement without using any symmetry (C1). (F) Electron density map shown from the 4-fold, 3-fold and 2-fold symmetry axis (left to right).

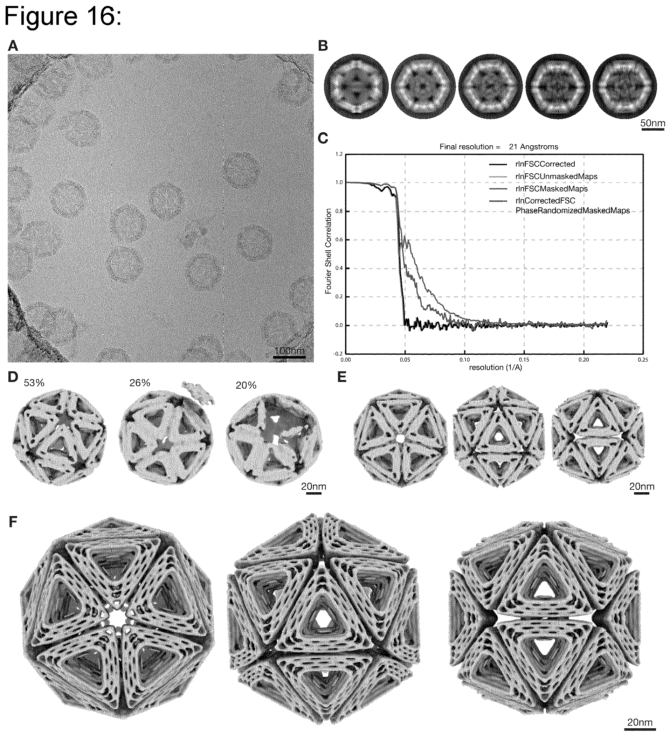

**Figure 16** shows Cryo-EM reconstruction of the T=1 shell at 20 mM MgCl₂. (A) Cryo-EM micrograph of the T=1 shell in free-standing ice. (B) Two dimensional class averages showing different orientations. (C) Graph showing different FSC curves which were used for resolution estimation. (D) Classes after 3d classification in relion3. (E) Refinement without using any symmetry (C1). (F) Electron density map shown from the 5-fold, 3-fold and 2-fold symmetry axis (left to right).

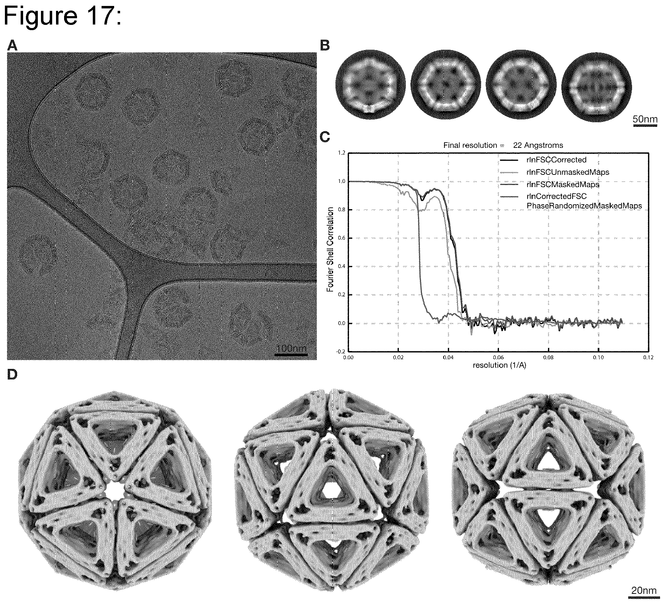

**Figure 17** shows Cryo-EM reconstruction of the T=1 shell at 25 mM MgCl₂. (A) Cryo-EM micrograph of T=1 shells on a lacey carbon grid with carbon support. (B) Two dimensional class averages showing different orientations. (C) Graph showing different FSC curves which were used for resolution estimation. (D) Electron density map shown from the 5-fold, 3-fold and 2-fold symmetry axis (left to right, respectively).

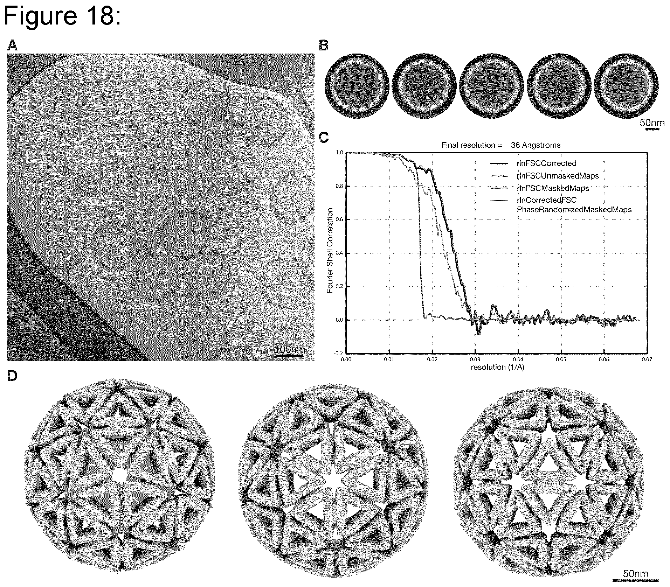

**Figure 18** shows Cryo-EM reconstruction of the T=3 shell at 20 mM MgCl₂. (A) Cryo-EM micrograph of T=3 shells on a lacey carbon grid with carbon support. (B) Two dimensional class averages showing different orientations. (C) Graph showing different FSC curves which were used for resolution estimation. (D) Electron density map shown from the 5-fold, 3-fold and 2-fold symmetry axis (left to right).

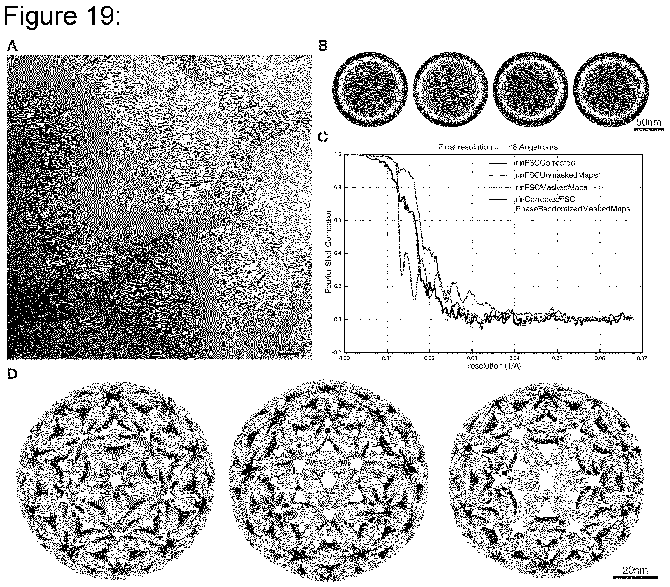

**Figure 19** shows cryo-EM reconstruction of the T=4 shell at 25 mM MgCl₂. (A) Cryo-EM micrograph of T=4 shells on a lacey carbon grid with carbon support. (B) Two dimensional class averages showing different orientations. (C) Graph showing different FSC curves which were used for resolution estimation. (D) Electron density map shown from the 5-fold, 3-fold and 2-fold symmetry axis (left to right).

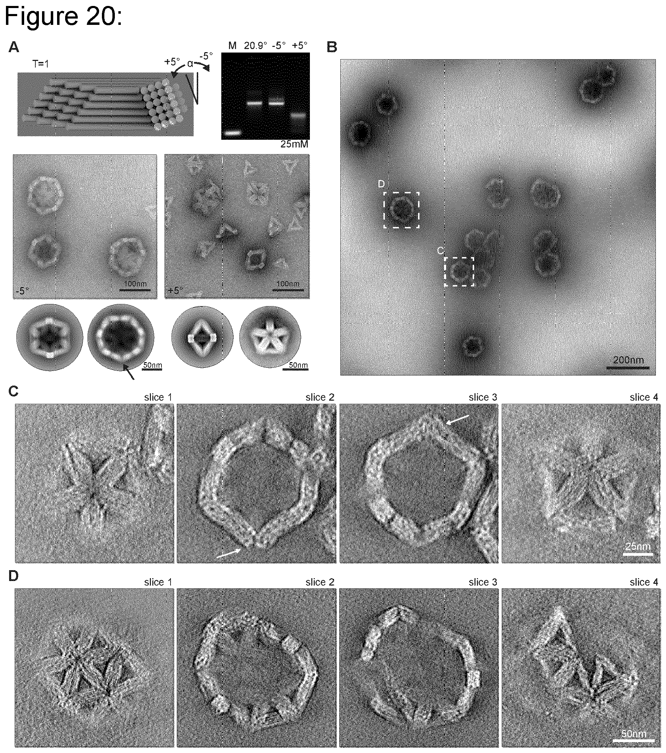

**Figure 20** shows negative stain EM tomograms of the T=1 triangle with -5° bevel angle. (A) Top left: Schematics of T=1 triangle designs with a modified bevel angle by 5° in both directions (original bevel angle 20.9°). Top right: Laser-scanned image of an agarose gel on which shell assembly reactions of all three design variants were electrophoresed. Bottom: exemplary negative-staining TEM micrographs and two-dimensional class averages of shell assembly products. (B) Negative stain TEM image of assembled T=1 triangles with a decreased bevel angle by -5° assembled at 35 mM MgCl₂. The dotted squares indicate the assemblies whose tomograms are shown in (C) and (D). (C) Four slices of a tomogram of an assembled T=1 shell. The white arrows indicate locations of stress release that accumulates because of the wrong bevel angle. (D) Four slices of a tomogram of a not fully closed assembly.

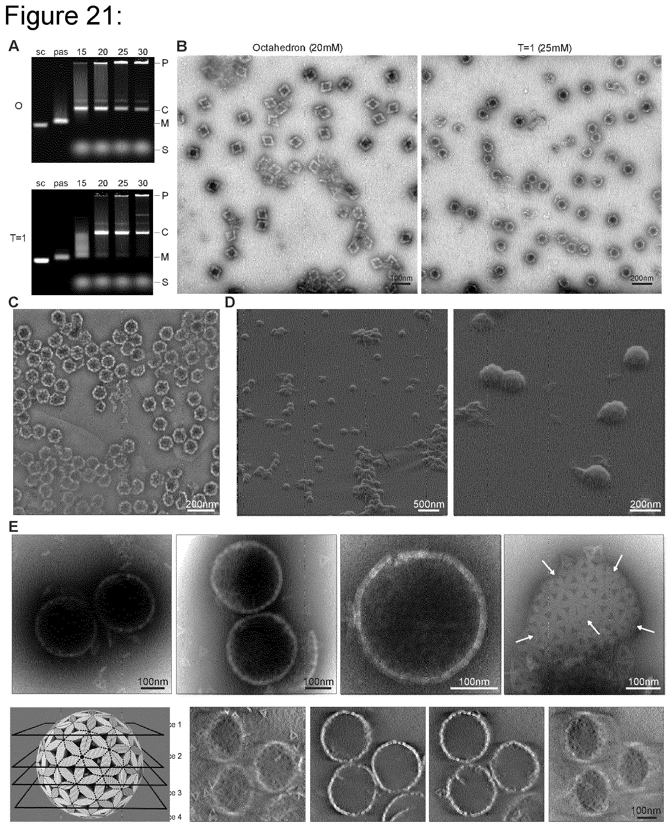

**Figure 21** shows TEM and HIM data of T=1, T=3 and T=9 shells. (A) 0.5% agarose gels containing 0.5x TBE buffer and 20 mM MgCl₂. The gels ran for 1.5 h at 90 V bias voltage. The buffer was exchanged after 45 min. While the octahedron triangles assemble at all tested MgCl₂ concentrations into shells during the triangle folding reaction, the T=1 triangles only assemble at 20mM MgCl₂ or higher into closed shells. There are staple strands in solution because the triangles are folded with a four times excess of staple over scaffold strands and not purified before loading them to the agarose gel. The folding reaction mixtures contained different amounts of MgCl₂ (15-30 mM) and were subjected to a thermal annealing ramp from 60°C to 45°C decreasing the temperature by 1°C per hour. sc: scaffold reference, pas: passivated triangle, 15-30: MgCl₂ concentration in mM present in the folding reaction, P: pocket, C: shell, M: monomer, S: excess staples. (B) Negative stain TEM images of octahedra and T=1 shells assembled during the folding reaction of the triangles at 20 and 25 mM MgCl₂, respectively. Single stranded excess staple strands are still visible. (C) Negative stain TEM image of assembled T=1 triangles assembled at 25 mM MgCl₂. (D) HIM images of T=3 shells coated with a 5 nm layer of AuPd. (E) Negative stain TEM images of assembled T=9 shells. Top: exemplary images of fully or partially assembled shells. The white arrows indicate the right locations of pentamers within the assembly. Bottom: Four slices of a tomogram showing three fully assembled T=9 shells.

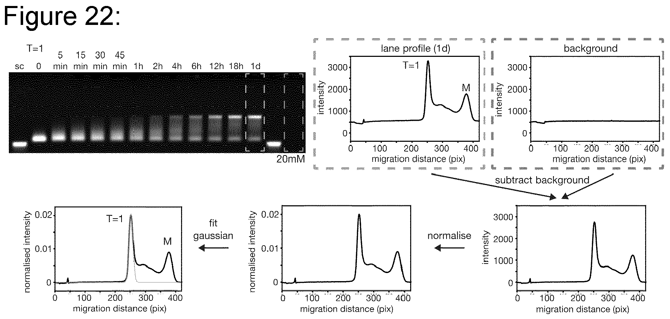

**Figure 22** shows exemplary procedure for extracting complete shell yield from agarose gels (done with Igor Pro 7). We extracted the lane profiles of the lanes containing the sample (orange) as well as of an empty lane (blue) to get the background signal. Subsequently, we subtracted the background, normalized the graph and fitted a Gaussian to the shell peak. The area underneath the Gaussian curve is the completed shell yield.

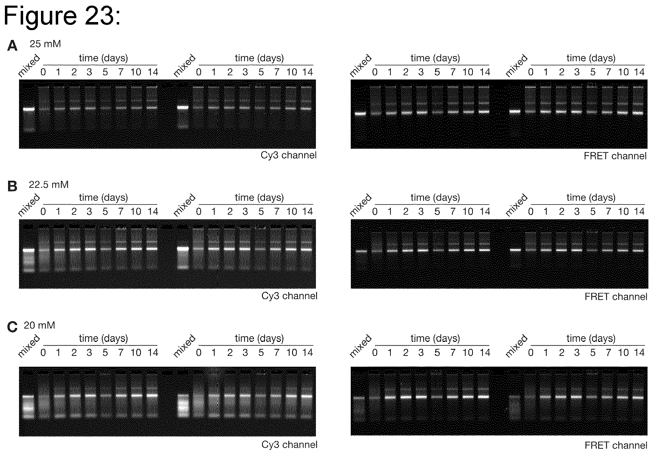

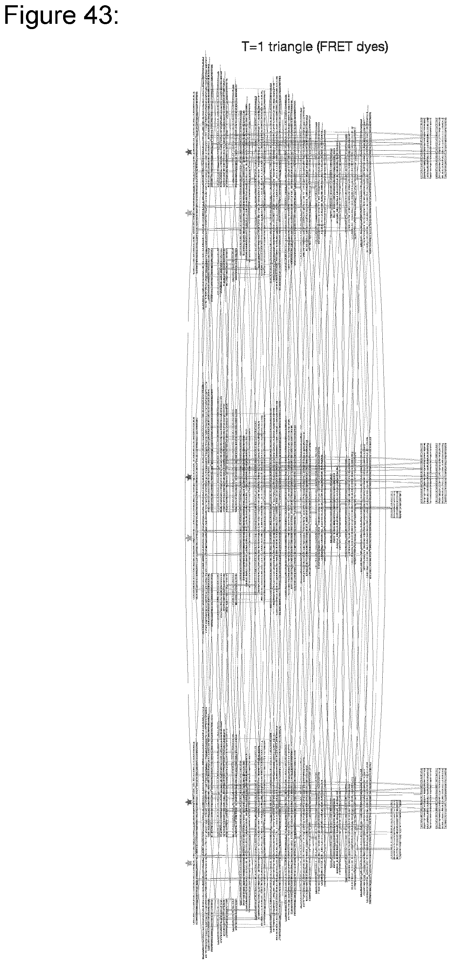

**Figure 23** shows subunit exchange experiments. Agarose gels used for extracting the FRET ratio of the subunit exchange experiments. The gels were run at 25 mM (A), 22.5 mM (B) and 20 mM MgCl₂ (C). All gels are 0.5% agarose gels containing 0.5x TBE buffer with different MgCl₂ concentrations and ran for 1.5 h at 90 V bias voltage. The buffer was exchanged after 45 min. The same gel was scanned in a Cy3 (left) and FRET (right) channel. Triangles with Cy3 and Cy5 labeled oligonucleotides at appropriate locations (see Fig. 43) and unlabeled triangles were assembled separately at 25 mM MgCl₂ before mixing the assembled shells and adjusting the MgCl₂ concentration. The mixtures were incubated up to 14 days at 40°C. At indicated time points aliquots were taken, frozen in liquid nitrogen and stored at -20°C. As reference for fully exchanged subunits, labeled and unlabeled triangles were assembled in a 1:1 ratio at 25 mM MgCl₂ (mixed).

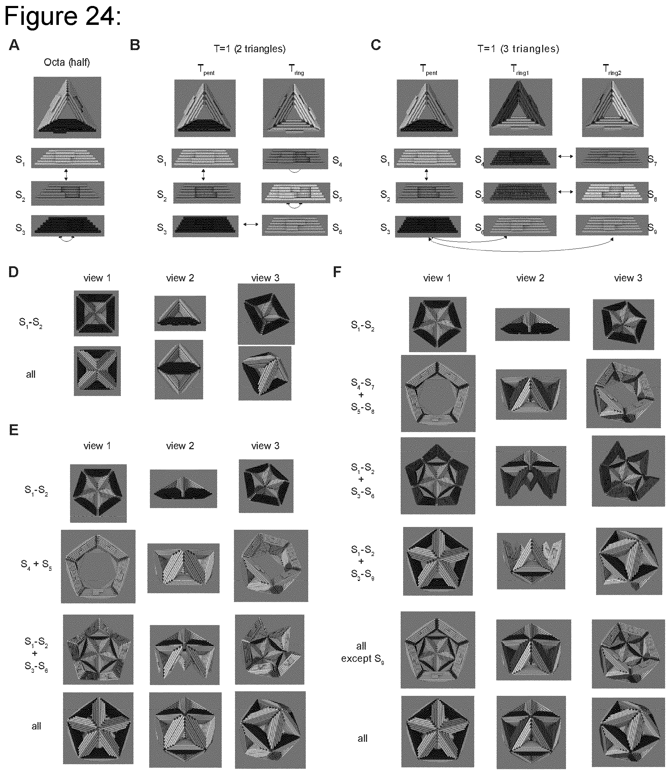

**Figure 24** shows design principle of triangular subunits for half shells. Cylindrical model of DNA-origami triangles assembling into half octahedra (A+D), half T=1 shells (B+E) or a T=1 shells with a missing pentagonal vertex (C+F). The sides of the triangles are modified with protrusions (light) and recesses (dark). The arrows indicate shape-complementary sides. Depending on the activation of certain sided the triangles form different kind of assemblies. All sides not involved in the binding of the triangles were passivated with polyT extensions at the stacking contacts.

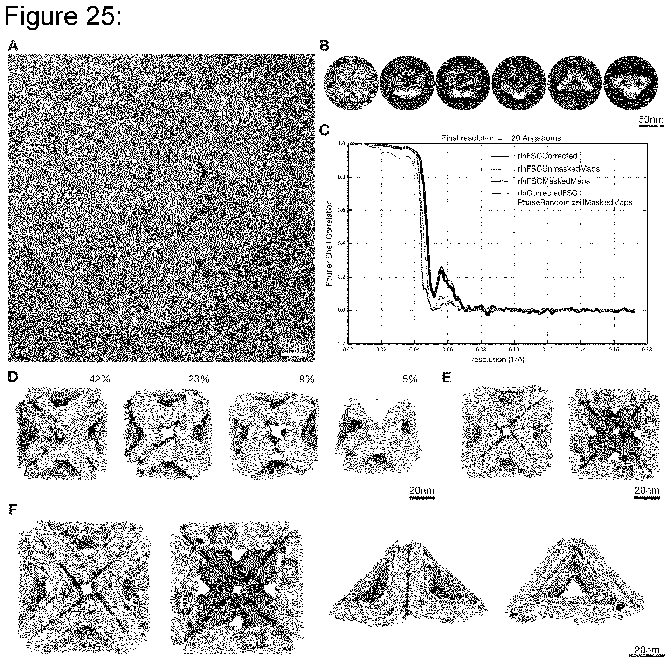

**Figure** 25 shows cryo-EM reconstruction of the half Octahedron shell. (A) Cryo-EM micrograph of the half Octahedron shell in free-standing ice. (B) Two dimensional class averages showing different orientations. (C) Graph showing different FSC curves which were used for resolution estimation. (D) Classes after 3d classification in relion3. (E) Refinement without using any symmetry (C1). (F) Electron density map of the half Octahedron shell reconstructed with C4 symmetry.

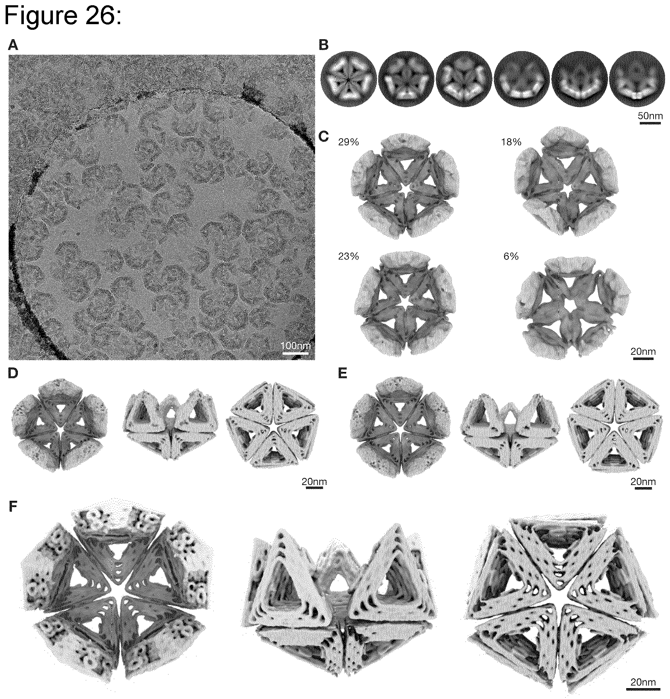

**Figure 26** shows cryo-EM reconstruction of the half T=1 shell. (A) Cryo-EM micrograph of the half T=1 shell in free-standing ice. (B) Two dimensional class averages showing different orientations. (C) Classes after 3d classification in relion3. (D) Refinement without using any symmetry (C1). (E) Refinement with using C5 symmetry. (F) Multibody refinement using six bodies indicated by the colors in relion3.

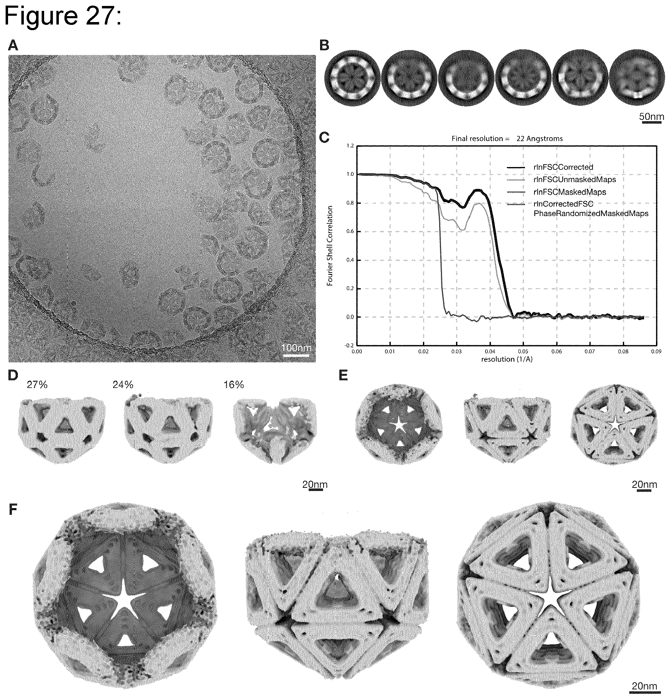

**Figure 27** shows cryo-EM reconstruction of the T=1 shell with a missing pentagon vertex. (A) Cryo-EM micrograph of T=1 shell with a missing pentagon vertex in free-standing ice. (B) Two dimensional class averages showing different orientations. (C) Graph showing different FSC curves which were used for resolution estimation. (D) Classes after 3d classification in relion3. (E) Refinement without using any symmetry (C1). (F) Electron density map of the T=1 shell missing one pentagon vertex reconstructed with C5 symmetry.

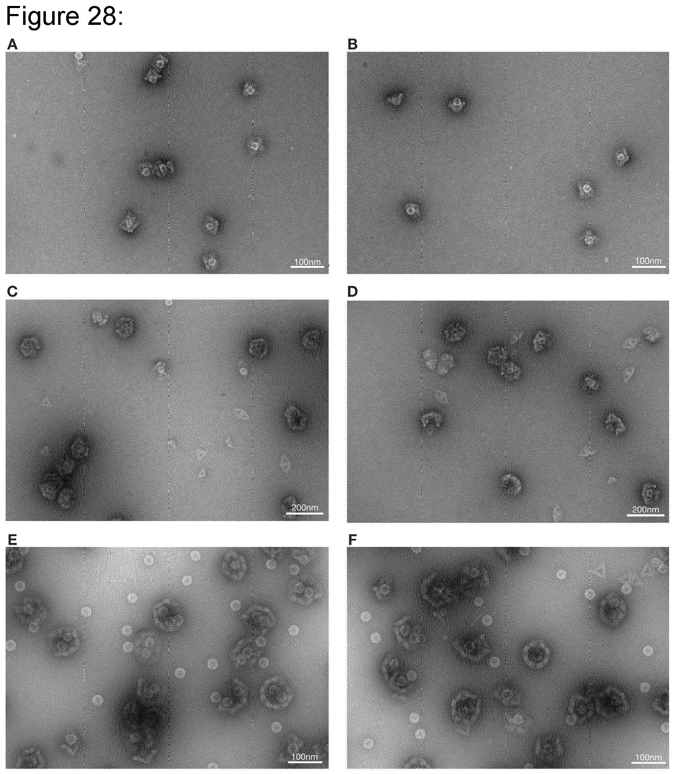

**Figure 28** shows negative stain EM images of encapsulated HBV core particles using a half octahedron (A), a half T'=1 shell (B) and a T=1 shell with a missing pentagonal vertex (C). All shells were assembled at 30 mM MgCl₂, incubated with the DNA modified 17H7 antibody overnight and subsequently incubated with the HBV particles for 1-4h or overnight.

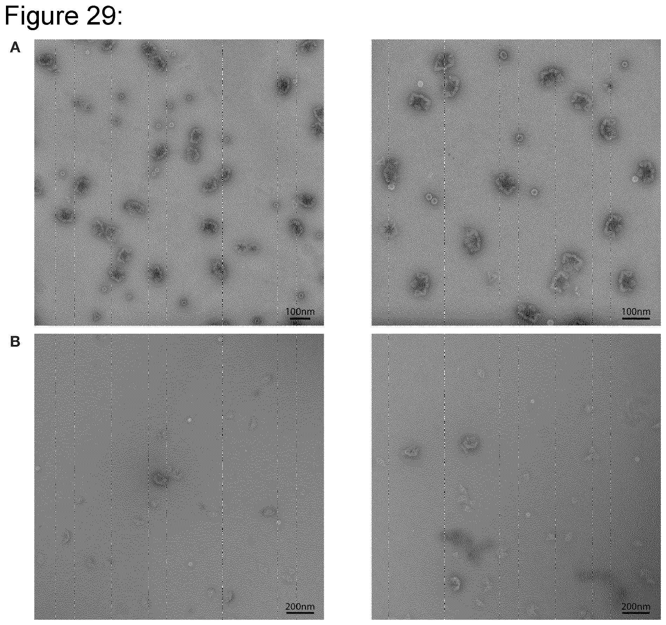

**Figure 29** shows negative control of HBV binding. (A) Negative stain images of HBV core particles and half T=1 shells without the presence of 17H7 antibodies. (B) Negative stain images of half T=1 shells equipped with 4D06 HBsAg antibodies and HBV core particles.

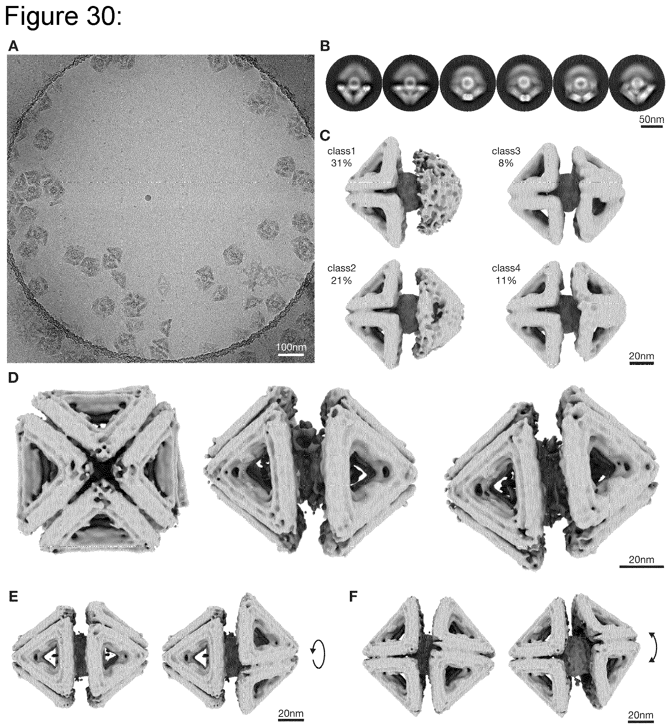

**Figure 30** shows cryo-EM reconstruction of the half Octahedron shell with a trapped HBV core particle. (A) Cryo-EM micrograph of the half Octahedron shell with HBV core particles in free-standing ice. (B) Two dimensional class averages showing different orientations. (C) Classes after 3d classification in relion3. One half of class1 and class2 is not well defined because of the averaging of multiple conformation of the second half shell. (D) Multibody refinement with three bodies (the two half shells and the HBV core particle) using the particles in class3 and class4 in (C). (E) First eigenvector of the multibody refinement showing a rotational movement of the second half shell. (F) Second eigenvector of the multibody refinement showing a sliding movement of the second half shell.

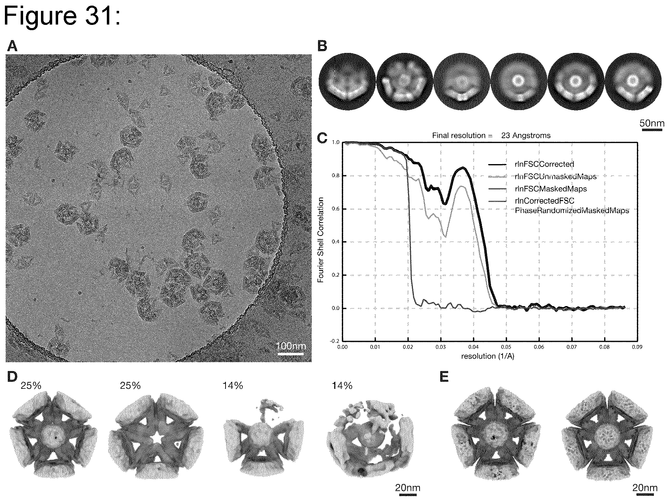

**Figure 31** shows cryo-EM reconstruction of the half T=1 shell with a trapped HBV core particle. (A) Cryo-EM micrograph of the half T=1 shell with a trapped HBV core particle in free-standing ice. (B) Two dimensional class averages showing different orientations. (C) Graph showing different FSC curves which were used for resolution estimation. (D) Classes after 3d classification in relion3. (E) Refinement without (left) and with (right) using C5 symmetry.

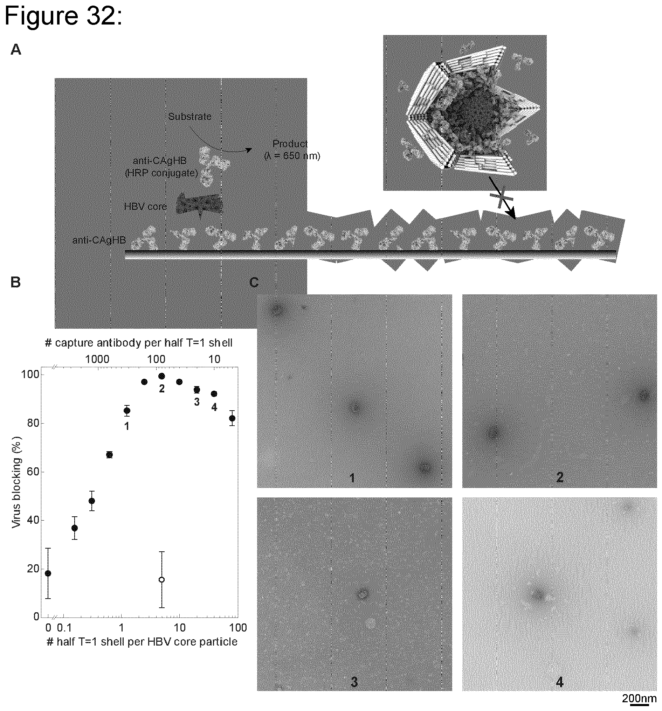

**Figure 32** shows in vitro virus blocking ELISA. (A) Schematics of the in vitro blocking ELISA. The HBV core particle by itself can bind to the immobilized CAgHB antibodies and can be visualized by a subsequent flushing of an anti-CAgHB conjugated with a horseradish peroxidase (HRP). When the HBV core particle is engulfed by a half T=1 particle, the shell sterically occludes the core particle from binding to the surface and the signal of the HRP is reduced. (B) In vitro virus blocking ELISA experiments of 2.5 pM HBV core particle incubated with pre-assembled mixtures of 1 nM oligonucleotide-conjugated capture antibody and various concentrations of half T=1 shells. Errors bars are standard deviation of triplicate measurements. (C) Negative stain TEM images of selected samples from the in vitro blocking ELISA indicated by numbers in (B). The half T=1 shell concentration increases: (1) 12.5 pM, (2) 3.1 pM, (3) 0.78 pM, (4) 0.39 pM.

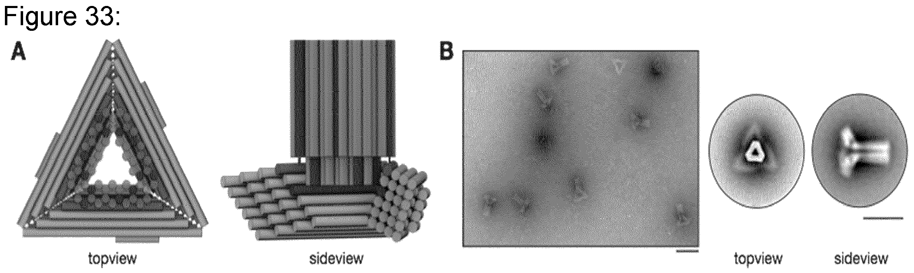

**Figure 33** shows design strategy T=1 triangle and a triangular brick structure. (A) Schematic representation of T=1 triangle dimerization (shown in orange) with the protruding brick (shown in blue) in top and side view. Each cylinder represents a double stranded DNA helix. The cylinders colored in red inside the triangle and in dark blue inside the tail mark those helices connecting the two objects. The thin lines between the two structures indicate single stranded DNA sticky ends extending from the triangle to the brick-like structure. (B) Field of view of a negative stain TEM image of the dimerized structures (left) and 2D class averages of the dimer in top and side view (right).

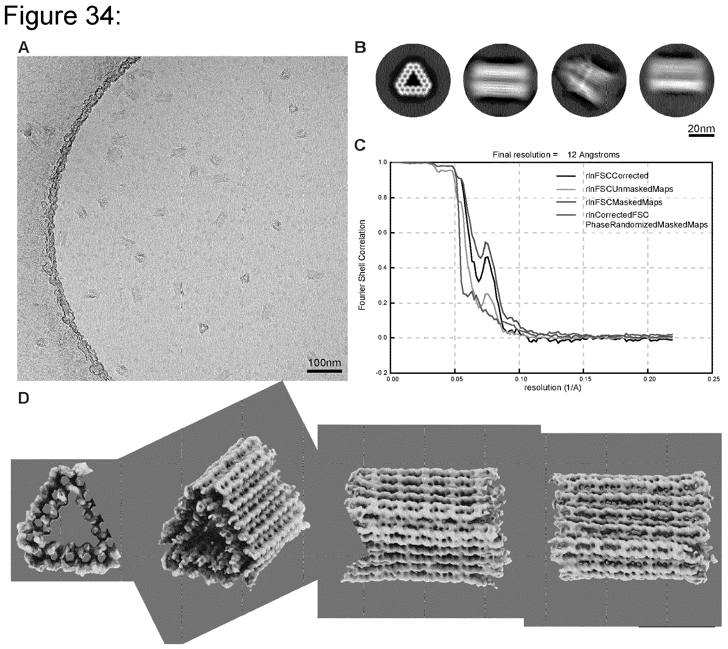

**Figure 34** shows cryo-EM reconstruction of the triangular brick. (A) Cryo-EM micrograph of the triangular brick in free-standing ice at 5 mM MgCl₂. (B) Two dimensional class averages showing different orientations. (C) Graph showing different FSC curves which were used for resolution estimation. (D) Electron density map shown from different viewing angles.

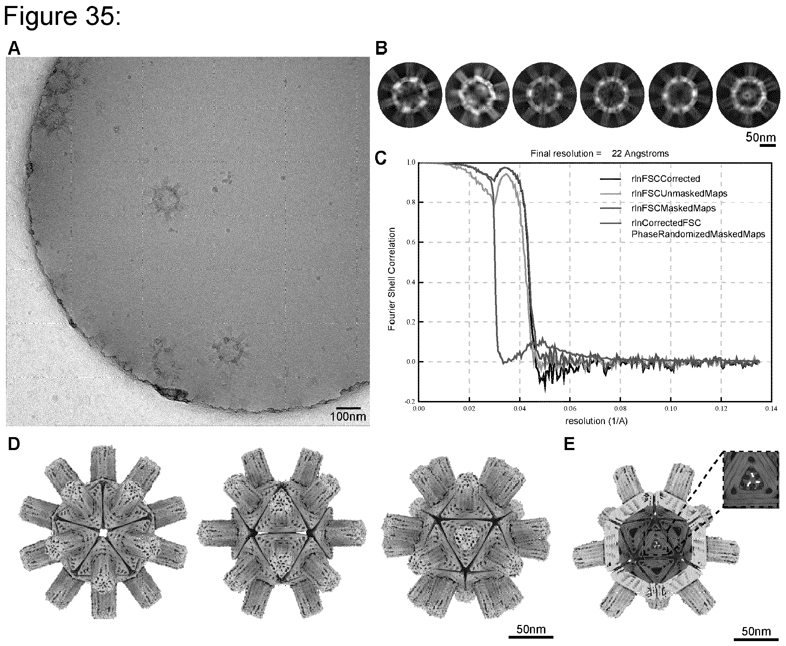

**Figure 35** shows cryo-EM reconstruction of the spiky shell at 22.5 mM MgCl₂. (A) Cryo-EM micrograph of the spiky shell in free-standing ice at 22.5 mM MgCl₂. (B) Two dimensional class averages showing different orientations. (C) Graph showing different FSC curves which were used for resolution estimation. (D) Electron density map shown from the 5-fold, 2-fold and 3-fold symmetry axis (left to right, respectively). (E) Cut through the density map shown in (D).
**Figure 36** shows design diagram of Octa and T=1 triangles prepared with caDNAno v0.1. Oligonucleotides that connect two different sides have five additional single-stranded thymidines at the corner.

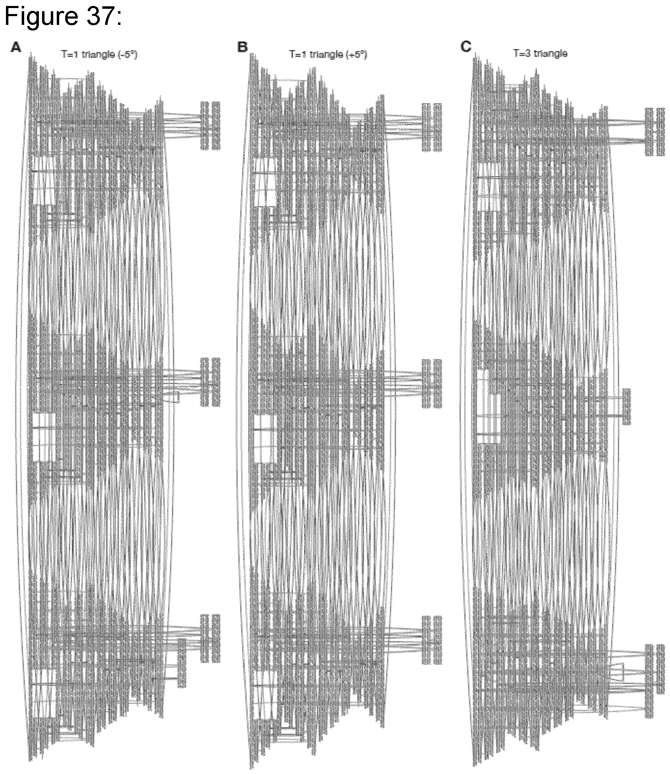

**Figure 37** shows design diagram of T=1 triangles with altered bevel angle and T=3 triangle prepared with caDNAno v0.1. Oligonucleotides that connect two different sides have five additional single-stranded thymidines at the corner.

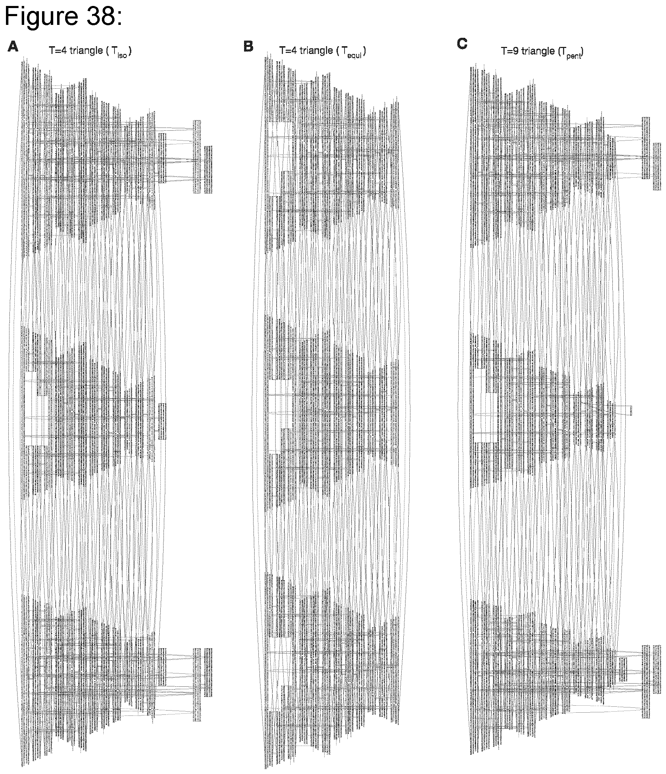

**Figure 38** shows design diagram of T=4 triangles and Tₚₑₙₜ prepared with caDNAno v0.1. Oligonucleotides that connect two different sides have five additional single-stranded thymidines at the corner.

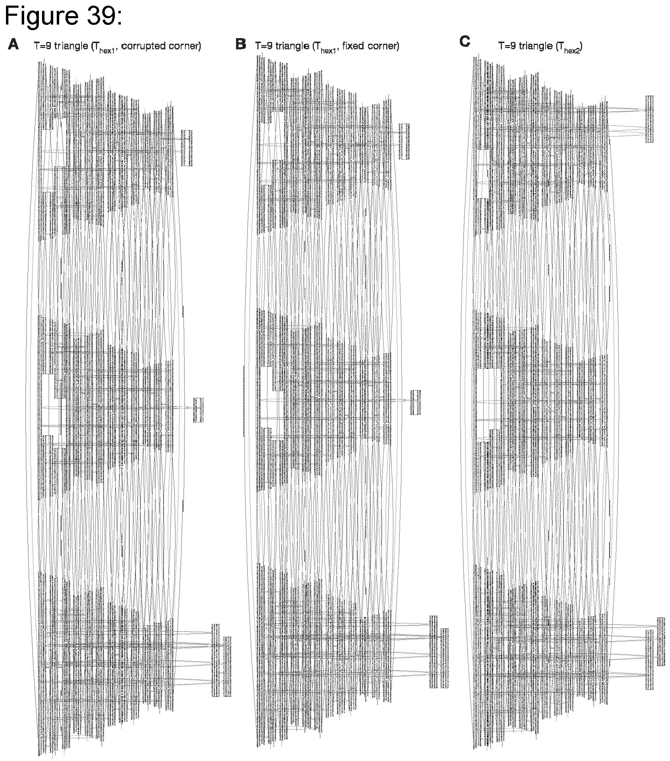

**Figure 39** shows design diagram of T=9 triangles Tₕₑₓ₁ and Tₕₑₓ₂ prepared with caDNAno v0.1. Oligonucleotides that connect two different sides have five additional single-stranded thymidines at the corner.

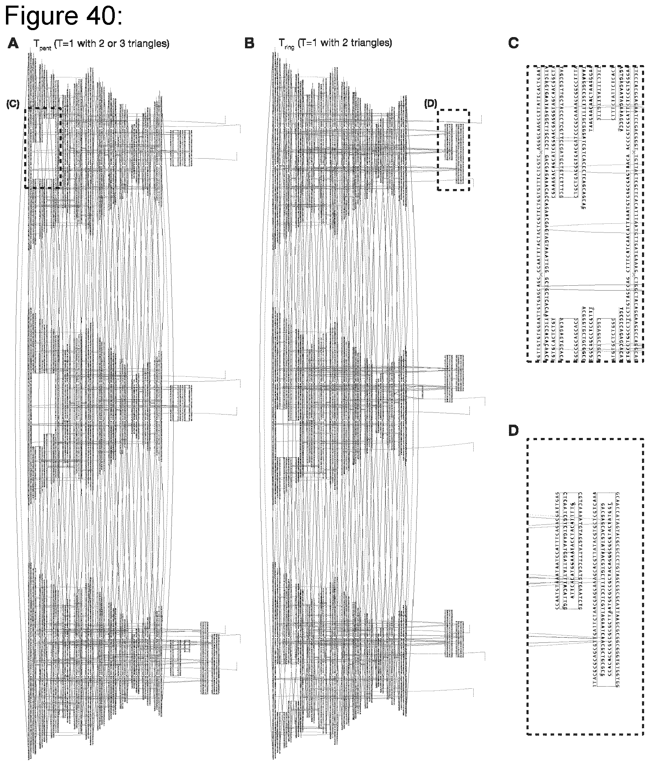

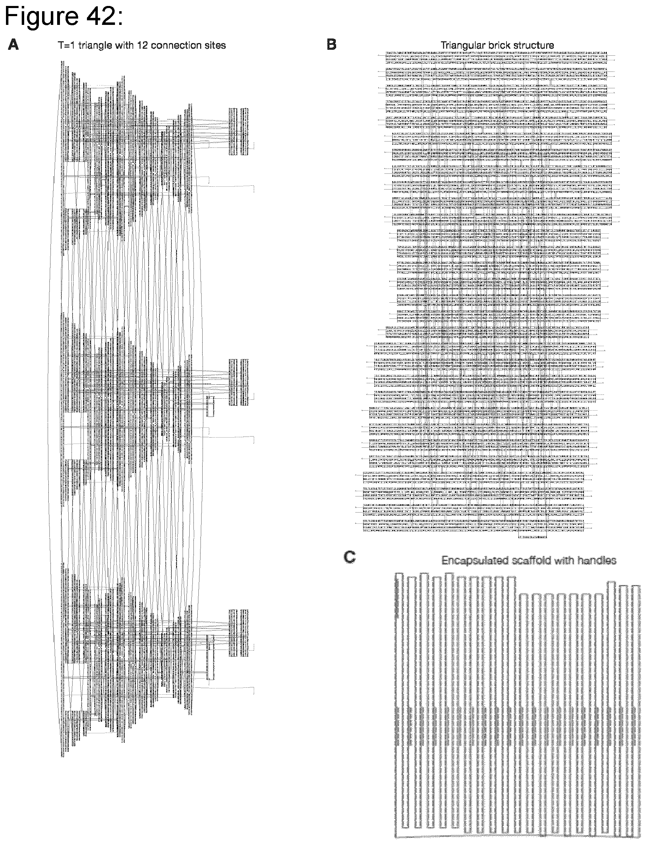

**Figure 40** shows design diagram of half-shell T=1 triangles prepared with caDNAno v0.1. (A)+(B) The Tₚₑₙₜ triangle can be used to assemble half T=1 shells together with the T_{ring} triangle or to assemble T=1 shells missing a pentagon vertex together with T_{ring1} and T_{ring2} triangles (Fig. 42). Oligonucleotides that connect two different sides have five additional single-stranded thymidines at the corner. Every side is equipped with three handles for attaching the antibodies (seq.: GCAGTAGAGTAGGTAGAGATTAGGCA-oligonucleotide). (C)+(D) Zoom on the recess and protrusion of the Tₚₑₙₜ and T_{ring} triangle in (A) and (B). Six oligos building the recess were modified with overhangs complementary to parts of the scaffold in the protrusion to increase the binding strength.

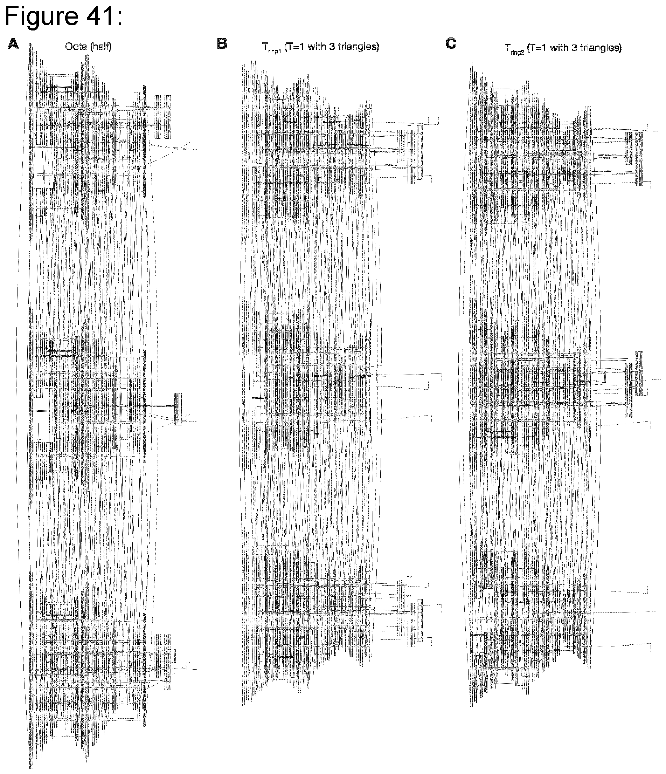

**Figure 41** shows design diagram of Octa and T=1 half-shell triangles prepared with caDNAno v0.1. Oligonucleotides that connect two different sides have five additional single-stranded thymidines at the corner. Every side is equipped with three handles for attaching the antibodies (seq.: GCAGTAGAGTAGGTAGAGATTAGGCA-oligo). The T_{ring1} and T_{ring2} triangles can assemble T=1 shells missing a pentagon vertex together with Tₚₑₙₜ triangle (Fig. 40).
**Figure 42** shows design diagrams for modified T=1 shells prepared with caDNAno v0.1. (A) T=1 triangle with protruding oligonucleotides. (B) Triangular brick structure with connections sites. (C) Single-stranded scaffold with 20 handles for triangle (grey) and one handle for Cy5 (red) attachment.
**Figure 43** shows a design diagram for a T=1 triangle with modifications prepared with caDNAno v0.1. T=1 triangle with Cy3 (green star) and Cy5 (red star) modifications on the 5' end.

### DETAILED DESCRIPTION OF THE INVENTION

The present disclosure provides constructs that enable the encapsulation of a virus or viral particle.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

Therefore, in one aspect, the disclosure provides a macromolecule-based nanostructure encasing a cavity with a diameter of at least 20 nm for encapsulating a virus or viral particle.

In the context of the present disclosure, the term "macromolecule-based nanostructure" refers to a nanostructure that is formed by the a set of macromolecules, wherein the macromolecules are selected from DNA, RNA, proteins and hybrids thereof, in particular hybrids selected from DNA-RNA hybrids and DNA-protein hybrids, including DNA-(protein linker)-DNA hybrids.

In the context of the present disclosure, the term "DNA" refers to deoxyribonucleic acid composed of a single-strand of monomeric units called nucleotides, wherein each nucleotide is composed of a nitrogen-containing nucleobase, a 2-deoxyribose sugar moiety, and a phosphate group, wherein the individual nucleotides are linked in the single-strand by a phosphate group linking the OH group in position 5' of a 2-deoxyribose sugar moiety to the OH group in 3' of a neighboring 2-deoxyribose sugar moiety. In particular embodiments, the nitrogen-containing nucleobases are independently selected from cytosine [C], guanine [G], adenine [A] and thymine [T]. In particular embodiments, one or more of the nucleobases are non-canonical bases, in particular a non-canonical base selected from the list of: a modified adenosine, in particular N6-carbamoyl-methyladenine or N6-methyadenine; a modified guanine, in particular 7-deazaguanine or 7-methylguanine; a modified cytosine, N4-methylcytosine, 5-carboxylcytosine, 5-formylcytosine, 5-glycosylhydroxymethylcytosine, 5-hydroxycytosine, or 5-methylcytosine; a modified thymidine, in particular α-glutamyl thymidine or α-putrescinyl thymine; a uracil or a modification thereof, in particular uracil, base J, 5-dihydroxypentauracil; or 5-hydroxymethyldeoxyuracil; deoxyarchaeosine and 2,6-diaminopurine. A stretch of a single-strand of DNA may interact with a complementary stretch of DNA by interaction of complementary nucleobases, wherein cytosine and guanine, and adenine and thymine, are complementary to each other, respectively by forming two (A/T) and three (G/C) hydrogen bonds between the nucleobases. Two single-strands of DNA may be fully complementary to each other, as in the case of genomic DNA, or may be partially complementary to each other, including situations, where one single-strand of DNA is partially complementary to two or more other single-stranded DNA strands. The interaction of two complementary single-stranded DNA sequences results in the formation of a double-stranded DNA double helix.

As is well known, DNA has evolved in nature as carrier of the genetic information encoding proteins. DNA further includes non-coding regions that include regions having regulatory functions. Thus, any DNA-based application usually critically depends on the specific DNA sequence and is almost always only enabled by naming the specific DNA sequence. In contrast, in the context of the present invention, such coding and/or regulatory functions do not play any role and may or may not be present, since the underlying DNA sequences are solely designed and selected in a way that the desired arrangement of double-helical subunits is formed. Thus, in one embodiment any form of a long single-stranded DNA sequence, whether naturally occurring DNA (such as the DNA of a bacteriophage) or synthetically produced DNA may be selected as template, and a set of short single-stranded DNA sequences may be designed, wherein each sequence is complementary to one or more different parts of the template and thus forms one or more double-helical sections. Collectively, all such double-helical sections created by interaction of the full set of short single-stranded DNA sequences with the template, then form the desired three-dimensional arrangement. Starting from a given single-stranded template sequence, the design of a set of complementary can be set up using known techniques, such as, for example, the methods described for the synthesis of megadalton-scale discrete objects with structurally well-defined 3D shapes (18, 24-35). In particular, iterative design with caDNAno (37) paired with elastic-network-guided molecular dynamics simulations (38) can be used.

In addition to the interaction of complementary nucleobases of different stretches of single-stranded DNA via hydrogen bonds, additional interactions between different DNA strands are possible, including the stacking interactions between the blunt ends of two double-stranded DNA helices (36), thus enabling the design and the formation of complex DNA-based nanostructures via the shape-complementarity of double-helical subunits. Thus, two three-dimensional arrangements formed in accordance with the previous paragraph, may interact with each other by stacking interactions between double-helical subunits present on the two three-dimensional arrangements, including specific interactions between two three-dimensional arrangements having complementary protrusions and recessions (or knobs and holes), as shown, for example, in Figs. 7-13D.

In the context of the present disclosure, the term "RNA" refers to ribonucleic acid composed of a single-strand of monomeric units called nucleotides, wherein each nucleotide is composed of a nitrogen-containing nucleobase, a ribose sugar moiety, and a phosphate group, wherein the individual nucleotides are linked in the single-strand by a phosphate group linking the OH group in position 5' of a ribose sugar moiety to the OH group in 3' of a neighboring ribose sugar moiety. In particular embodiments, the nitrogen-containing nucleobases are independently selected from cytosine [C], guanine [G], adenine [A] and uracil [U]. In particular embodiments, one or more of the nucleobases are non-canonical bases, in particular a non-canonical base selected from the list of: pseudouridine, ribothymidine, and inosine. Unlike DNA, RNA is most often in a single-stranded form, but the formation of double-stranded forms is possible by interaction of complementary nucleobases, wherein cytosine and guanine, and adenine and uracil, are complementary to each other, respectively by forming two (A/U) and three (G/C) hydrogen bonds between the nucleobases. In a particular embodiment, the disclosure provides a macromolecule-based nanostructure, which is a RNA-based nanostructure.

In a particular embodiment, said cavity has a diameter of at least 50 nm, at least 100 nm, at least 150 nm, at least 200 nm or at least 250 nm.

In particular embodiments, said cavity has a diameter of at most 1,000 nm.

In the context of the present invention, the term "diameter" refers to the diameter of the smallest circle that is encompassed by the surface of the macromolecule-based nanostructure. For the sake of clarity, in the case of a macromolecule-based nanostructure in the form of a capsule (or spherocylinder), the diameter is the diameter of the hemispherical ends and/or the diameter of the cylindrical central part.

In a particular embodiment, the macromolecule-based nanostructure has a molecular mass of at least 1 MDa, particularly at least 10 MDa, particularly at least 20 MDa, more particularly at least 30 MDa. In other particular embodiments, the DNA-based nanostructure has a molecular mass of at least 50 MDa, at least 80 MDa, at least 100 MDa, at least 200 MDa, or at least 500 MDa. In particular embodiments, the DNA-based nanostructure has a molecular mass of at most 1,500 MDa.

In particular embodiments, the ratio between the numerical value of the molecular mass of the macromolecule-based nanostructure (in MDa) and the numerical value of the volume of the cavity encased by said macromolecule-based nanostructure (in nm³) is less than 10,000, particularly less than 9,000. In particular embodiments said ratio has a value of between 1,000 and 10,000, particularly between 2,000 and 9,000. For example, in the case of certain octahedral nanostructures, where the molecular mass is about 40 MDa, and where the encased volume is about 113,000 nm³, said ratio is about 2,800.

In particular embodiments, the ratio between the outer surface area of the macromolecule-based nanostructure covered by the macromolecules forming said macromolecule-based nanostructure and the outer surface area not covered by said macromolecules (excluding the area of the opening of a macromolecule-based nanostructure in the form of a shell) is at least 1, in particular at least 2, in particular at least 4, in particular at least 6, in particular at least 8. In other particular embodiments, the ratio is at least 10. In particular embodiments, the ratio is between 1 and 20, in particular between 2 and 18, between 4 and 16, between 6 and 14, and more particularly between 8 and 12. For example, in a case, where the macromolecule-based nanostructure is a shell in the form of a half sphere, only the area of the curved surface, but not that of the opening, i.e. the area of the flat face of the half sphere, is used for calculating said ratio.

In a particular embodiment, the present invention relates to a DNA-based nanostructure.

In a particular embodiment, the DNA-based nanostructure is formed by self-assembling DNA-based building blocks.

In a particular embodiment, each of said self-assembling DNA-based building blocks is formed by a single-stranded DNA template strand and a set of oligonucleotides complementary to said single-stranded DNA template, wherein each of said oligonucleotides is either complementary to one contiguous DNA sequence stretch or to at least two non-contiguous DNA sequence stretches on said single-stranded DNA template.

In a particular embodiment, the molecular weight of each self-assembling DNA-based building block is between 4.5 and 5.5 MDa.

In a particular embodiment, the each self-assembling DNA-based building block comprises between 7,500 and 8,500 base pairs.

In a particular embodiment, the DNA-based nanostructure consists of between 4 and 180 of such self-assembling DNA-based building blocks.

In particular embodiments, said single-stranded DNA template is single-stranded DNA of filamentous bacteriophage, or is derived from single-stranded DNA of filamentous bacteriophage.

In the context of the present invention, the term "filamentous bacteriophage" refers to a type of bacteriophage, or virus of bacteria, which is characterized by its filament-like shape that usually contains a genome of circular single-stranded DNA and infects Gram-negative bacteria. Filamentous phage includes Ff phage, such as M13, f1 and fd1 phage, and Pf1 phage.

In particular embodiments, said single-stranded DNA template has a sequence selected from SEQ ID NO: 1 (M13 8064) and 2 (M13 7249) (see Table 1). In particular embodiments, said single-stranded DNA is circular.

In the context of the present invention, a single-stranded DNA template that is "derived from single-stranded DNA of filamentous bacteriophage" refers to a DNA construct that is derived from a naturally occurring of published DNA sequence of a filamentous bacteriophage by one or more of: (i) opening of the circular structure to a linear sequence; (ii) deletion of one or more nucleotides; (iii) insertion of one or more nucleotides; (iii) substitution of one or more nucleotides; (iv) addition of one or more nucleotides; and (v) modification of one or more nucleotides. While any such variation might have detrimental, or at least rather unpredictable, effects on bacteriophage biology, its infectivity and its ability to propagate, such effects do not play any role in the context of the present invention, since, as already mentioned above, said single-stranded DNA template is only used as naked template without any requirement for having any functional property, and all structural aspects, such as the correct formation the three-dimensional shape of said self-assembling DNA-based building blocks, are implemented by the proper choice of said set of complementary oligonucleotides.

In particular embodiments, said single-stranded DNA template has at least 80 %, particularly at least 90 %, more particularly at least 95 %, sequence identity to the sequence of a naturally occurring or published sequence of a filamentous bacteriophage, in particular to a M13, f1 or fd1 phage, in particular to a sequence selected from SEQ ID NO: 1 (M13 8064) and 2 (M13 7249).

In a particular embodiment, the DNA-based nanostructure is a closed three-dimensional geometric shape, in particular a closed three-dimensional geometric shape selected from a sphere, a spherocylinder, and a polyhedron, in particular a tetrahedron, an octahedron or an icosahedron, formed in situ from said self-assembling DNA-based building blocks in the presence of said virus or viral particle to be encapsulated.

In another particular embodiment, the DNA-based nanostructure is a shell with an opening for accessing said cavity.

In the context of the present invention, the term "shell" refers to a structure that is a part of a closed three-dimensional geometric shape, in particular a closed three-dimensional geometric shape selected from a sphere, a spherocylinder, and a polyhedron, in particular a tetrahedron or an octahedron,

In yet another particular embodiment, the DNA-based nanostructure is a combination of a first shell and a second shell with an opening to access a first and a second inner cavity, respectively, wherein said first and said second inner cavity together form said cavity.

In a particular embodiment, said first and said second shells are connected by at least one linker.

In particular embodiments, said linker is a linker selected from a DNA linker, an RNA linker, a polypeptide linker, a protein linker and a chemical linker.

In the context of the present invention, the term "DNA linker" refers to a linker formed from DNA, wherein the sequence of said DNA linker is not complementary to the DNA of said single-stranded DNA template or to any of said set of oligonucleotides complementary to said single-stranded DNA template, wherein said DNA linker is linked at one terminus to a DNA sequence forming a self-assembling DNA-based building block of said first shell, and at the other terminus to a DNA sequence forming a self-assembling DNA-based building block of said second shell.

In the context of the present invention, the term "polypeptide linker" refers to a linker formed from at least 2, particularly at least 5, at least 10, or at least 20 amino acid residues linked by peptide bonds, wherein said polypeptide has no tertiary or quaternary structure, and wherein said polypeptide linker is linked at one terminus to a DNA sequence forming a self-assembling DNA-based building block of said first shell, and at the other terminus to a DNA sequence forming a self-assembling DNA-based building block of said second shell.

In the context of the present invention, the term "protein linker" refers to a linker formed from at least 20, particularly at least 50, at least 100, at least 200 amino acid residues, at least 500 amino acid residues, or at least 1,000 amino acid residues, particularly less than 1,500 amino acid residues linked by peptide bonds, wherein said polypeptide has tertiary and/or quaternary structure, and wherein said protein linker is linked at one terminus to a DNA sequence forming a self-assembling DNA-based building block of said first shell, and at the other terminus to a DNA sequence forming a self-assembling DNA-based building block of said second shell. In particular embodiments, said protein linker is covalently attached to said DNA sequences. In particular other embodiments, said protein linker is non-covalently attached to said DNA sequences, in particular wherein said protein linker is an antibody-based protein linker, in particular selected from a diabody and a full antibody, including an IgG antibody.

In the context of the present invention, the term "chemical linker" refers to a continuous chain of between 1 and 30 atoms (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 atoms; thus, in the context of the present invention, the term "between" is used so that the borders mentioned are included) in its backbone, i.e. the length of the linker is defined as the shortest connection as measured by the number of atoms or bonds between the two DNA sequences linked by said chemical linker. In the context of the present invention, a chemical linker preferably is an C₁₋₂₀-alkylene, C₁₋₂₀-heteroalkylene, C₂-₂₀-alkenylene, C₂₋₂₀-heteroalkenylene, C₂₋₂₀-alkynylene, C₂₋₂₀-heteroalkynylene, cycloalkylene, heterocycloalkylene, arylene, heteroarylene, aralkylene, or a heteroaralkylene group, which may optionally be substituted. The linker may contain one or more structural elements such as carboxamide, ester, ether, thioether, disulfide, urea, thiourea, hydrocarbon moieties and the like. The linker may also contain combinations of two or more of these structural elements. Each one of these structural elements may be present in the linker more than once, e.g. twice, three times, four times, five times, or six times. In some embodiments the linker may comprise a disulfide bond. It is understood that the linker has to be attached either in a single step or in two or more subsequent steps to the two DNA sequences linked by said chemical linker. To that end the linker to be will carry two groups, preferably at a proximal and distal end, which can (i) form a covalent bond to a group present in one of the two DNA sequences to be linked, or (ii) which is or can be activated to form a covalent bond with one of the two DNA sequences.

In a particular embodiment, the DNA-based nanostructure is based on an icosahedral structure.

In a particular embodiment, each of said self-assembling DNA-based building blocks is a prismoid.

In the context of the present invention, the term "prismoid" refers to a polyhedron, wherein all vertices lie in two parallel planes.

In particular embodiments, said prismoid is a triangular prismoid. In other embodiments, said prismoid is a rectangular prismoid.

In particular embodiments, the DNA-based nanostructure is based on a mixture of a triangular and a rectangular prismoid.

In a particular embodiment, the present invention relates to a DNA-based nanostructure,
wherein each said triangular, or said rectangular prismoid, is formed by m triangular, or rectangular, respectively, planes, wherein m is an integer independently selected from 4, 5, 6, 7 and 8, in particular independently selected from 5, 6 and 7, more particularly wherein said integer is 6.
wherein the three, or four, respectively, edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4,
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes, and
wherein at least two of the three, or four, respectively, side trapezoids comprises a specific pattern of recesses and/or extrusions formed by missing or additional DNA double helical stretches for specific interaction with a complementary pattern on the side trapezoid of another one of said self-assembling DNA-based building blocks.

In a particular embodiment, the average length of each of the n stretches of DNA double helices in the m planes of a triangular, or rectangular, respectively, prismoid is between 80 and 200 base pairs.

In particular embodiments, said triangular prismoid is a triangular frustum. In particular embodiments, said rectangular prismoid is a rectangular frustum.

In the context of the present invention, the term "triangular frustum" refers to a three-dimensional geometric shape in the form of a triangular pyramid, and the term "rectangular frustum" refers to a three-dimensional geometric shape in the form of a rectangular pyramid, where the tip of the pyramid has been removed resulting in a plane on the top parallel to the basis of the pyramid.

In a particular embodiment, for at least part of said self-assembling DNA-based building blocks the length of at least one edge of each of said m planes is decreasing from the first to the m^{th} plane, so that a bevel angle results between planes perpendicular to said first plane and the trapezoid plane formed by said m edges (see Fig. 5). In particular embodiments, all three, or four, respectively, trapezoid planes exhibit a bevel angle.

In a particular embodiment, a bevel angle is between 16° and 26°, particularly between 18° and 24°, more particularly between 20° and 22°, most particularly about 20.9°.

In a particular embodiment, said DNA-based nanostructure comprises at least one set of self-assembling DNA-based building blocks, wherein all three, or four, respectively, side trapezoids comprises a specific pattern of recesses and/or extrusions formed by missing or additional DNA double helical stretches for specific interaction with a complementary pattern on the side trapezoid of another one of said self-assembling DNA-based building blocks.

In a particular embodiment, particularly in the case of a DNA-based nanostructure closed three-dimensional geometric shape, all said self-assembling DNA-based building blocks are identical.

In a particular embodiment, said DNA-based nanostructure comprises two or more sets of self-assembling DNA-based building blocks.

In a particular embodiment, said DNA-based nanostructure is rod-shaped.

In particular embodiments, said DNA-based nanostructure comprises two or more sets of self-assembling DNA-based building blocks.

In particular such embodiment, said rod-shaped DNA-based nanostructure comprises at least a first and a second set of self-assembling DNA-based building blocks, wherein said first and set second set differ at least with respect to the bevel angles. In a particular embodiment, at least one set consists of self-assembling DNA-based building blocks exhibiting only two bevel angles. In a particular embodiment, said at least one set consists of rectangular frusta, which comprise a bevel angle on each of two opposing trapezoids.

In a particular embodiment, the side trapezoids forming the rim of said shell, or of said first and second shell, respectively, do not comprises a specific pattern of recesses and/or extrusions formed by missing or additional DNA double helical stretches for specific interaction with a complementary pattern on the side trapezoid of another one of said self-assembling DNA-based building blocks.

In a particular embodiment, said DNA-based nanostructure is a shell selected from
(i) a half octahedron (Fig. 4A), which consists of a set of four copies of a triangular frustum, wherein the base-pair stacking contacts on one of the triangular edges of the triangular frustum are inactivated by either strand shortening or by adding unpaired thymidines (Fig. 4A, see Fig. 24A,D);
(ii) a half T=1 shell (Fig. 4B), which consists of two sets of in each case five copies of two different triangular frusta, wherein the five copies of the first set form a closed pentamer, and the five copies of the second set dock onto the edges of said pentamer (Fig. 4B, see Fig. 24B,E); and
(iii) a "trap" T=1 shell with a missing pentagon vertex (Fig. 4C), which consists of three sets of in each case five copies of three different triangular frusta, wherein the five copies of the first set form a closed pentamer, the five copies of the second set dock onto the edges of said pentamer the five copies of the second set dock onto the edges of said pentamer, and the five copies of the third set dock into the gaps between the five copies of said second set (Fig. 4C, see Fig. 24C,F).

In a particular embodiment, the present invention relates to a DNA-based nanostructure further comprising one or more types of DNA brick constructs, each type of such DNA brick constructs being characterized by one or more interaction sites for specific interaction by edge-to-edge stacking contacts with one or more complementary interaction sites present on the plane of said triangular, or rectangular, respectively, prismoid on the outer surface of said DNA-based nanostructure, wherein said DNA brick constructs cover the free space between the three, or four, respectively, edges of said plane (see Fig. 33).

In a particular embodiment, the present invention relates to a DNA-based nanostructure further comprising one or more cross-linkages within one of said triangular, or rectangular, respectively, prismoids, and/or between two of said triangular, or rectangular, respectively, prismoids.

In the context of the present invention, the term "cross-linkage" refers to any permanent or intermittent linkage within one of said triangular, or rectangular, respectively, prismoids, and/or between two of said triangular, or rectangular, respectively, prismoids. Any such linkage may be achieved *a priori* by linking two of the oligonucleotides being used for forming the self-assembling DNA-based building blocks prior to the assembly, or *a priori,* e. g. by chemically or photochemically adding linkages between different parts of the three-dimensional nanostructure. Permanent linkages may, for example, be created by photochemically cross-linking T residues appropriately positioned in the structure under formation of covalent cyclobutane pyrimidine dimer (CPD) bonds (41), and intermittent linkages may, for example, be created by photochemically cross-linking the blunt ends of two double-helical subunits between a 3-cyanovinylcarbazole (cnvK) moiety positioned at a first blunt end and a thymine residue (T) positioned at the other blunt end (40).

In a particular embodiment, the present invention relates to a macromolecule-based nanostructure further comprising at least one moiety specifically interacting with said virus or viral particle.

In particular embodiments, said macromolecule-based nanostructure is a DNA-based nanostructure in accordance with the present invention, wherein said at least one moiety is linked to one of said triangular, or rectangular, respectively, prismoids forming the DNA-based nanostructure in a way that said at least one moiety is located on the inside of said DNA-based nanostructure and is pointing into the cavity formed by said DNA-based nanostructure.

In particular embodiments, said at least one moiety is specifically interacting with said virus or viral particle by being able to specifically bind to said virus or virus particle. In particular embodiments, said at least one moiety is an antibody or a binding moiety based on an antibody comprising at least an antigen-binding site of an antibody, in particular at least a VH domain of an antibody or at least a combination of a VH and a VL domain of an antibody.

In particular embodiments, said at least one moiety is specifically interacting with said virus or viral particle by being able to bind to inactivate said virus or virus particle.

In another aspect, the disclosure provides a composition comprising a virus or viral particle encapsulated by a macromolecule-based nanostructure according to the present invention.

In particular embodiments, said composition is formed in a process of removing said virus or viral particle from a medium containing said virus or viral particle. In particular other embodiments, said composition is formed in a process of incorporating said virus or viral particle as cargo in said macromolecule-based nanostructure.

In another aspect, the disclosure provides a composition comprising a cargo different from a virus or viral particle, where said cargo, such as a complex macromolecule, is encapsulated by a macromolecule-based nanostructure according to the present invention

In yet another aspect, the disclosure provides a method for encapsulating a virus or viral particle, comprising the steps of: providing a macromolecule-based nanostructure according to the present invention, and contacting said macromolecule-based nanostructure with a medium comprising, or suspected to comprise, said virus or viral particle.

In particular embodiments, said method is for removing said virus or viral particle from said medium. In particular embodiment, said method is for encapsulating said virus or viral particle in order to transport said virus or viral particle.

In yet another aspect, the disclosure provides a method for encapsulating a cargo different from a virus or viral particle, such as a complex macromolecule, comprising the steps of: providing a macromolecule-based nanostructure according to the present invention, and contacting said macromolecule-based nanostructure with a medium comprising, or suspected to comprise, said cargo.

**TABLE 1: Sequence listing.**

| **SEQ ID NO:** | **Description** | **Sequence / Details** |
|---|---|---|
| | **Templates** | |
| 1 | M13 8064 | GGCAATGACCTGATAGCCTTTGTAGATCTCTCAAAAATAGCTACCCTCTCCGGCATTAATTTATCAGCT AGAACGGTTGAATATCATATTGATGGTGATTTGACTGTCTCCGGCCTTTCTCACCCTTTTGAATCTTTA CCTACACATTACTCAGGCATTGCATTTAAAATATATGAGGGTTCTAAAAATTTTTATCCTTGCGTTGAAA TAAAGGCTTCTCCCGCAAAAGTATTACAGGGTCATAATGTTTTTGGTACAACCGATTTAGCTTTATGCT CTGAGGCTTTATTGCTTAATTTTGCTAATTCTTTGCCTTGCCTGTATGATTTATTGGATGTTAATGCTAC TACTATTAGTAGAATTGATGCCACCTTTTCAGCTCGCGCCCCAAATGAAAATATAGCTAAACAGGTTAT TGACCATTTGCGAAATGTATCTAATGGTCAAACTAAATCTACTCGTTCGCAGAATTGGGAATCAACTGT TATATGGAATGAAACTTCCAGACACCGTACTTTAGTTGCATATTTAAAACATGTTGAGCTACAGCATTA TATTCAGCAATTAAGCTCTAAGCCATCCGCAAAAATGACCTCTTATCAAAAGGAGCAATTAAAGGTACT CTCTAATCCTGACCTGTTGGAGTTTGCTTCCGGTCTGGTTCGCTTTGAAGCTCGAATTAAAACGCGAT ATTTGAAGTCTTTCGGGCTTCCTCTTAATCTTTTTGATGCAATCCGCTTTGCTTCTGACTATAATAGTCA GGGTAAAGACCTGATTTTTGATTTATGGTCATTCTCGTTTTCTGAACTGTTTAAAGCATTTGAGGGGGA TTCAATGAATATTTATGACGATTCCGCAGTATTGGACGCTATCCAGTCTAAACATTTTACTATTACCCC CTCTGGCAAAACTTCTTTTGCAAAAGCCTCTCGCTATTTTGGTTTTTATCGTCGTCTGGTAAACGAGGG TTATGATAGTGTTGCTCTTACTATGCCTCGTAATTCCTTTTGGCGTTATGTATCTGCATTAGTTGAATGT GGTATTCCTAAATCTCAACTGATGAATCTTTCTACCTGTAATAATGTTGTTCCGTTAGTTCGTTTTATTA ACGTAGATTTTTCTTCCCAACGTCCTGACTGGTATAATGAGCCAGTTCTTAAAATCGCATAAGGTAATT CACAATGATTAAAGTTGAAATTAAACCATCTCAAGCCCAATTTACTACTCGTTCTGGTGTTTCTCGTCA GGGCAAGCCTTATTCACTGAATGAGCAGCTTTGTTACGTTGATTTGGGTAATGAATATCCGGTTCTTG TCAAGATTACTCTTGATGAAGGTCAGCCAGCCTATGCGCCTGGTCTGTACACCGTTCATCTGTCCTCT TTCAAAGTTGGTCAGTTCGGTTCCCTTATGATTGACCGTCTGCGCCTCGTTCCGGCTAAGTAACATGG AGCAGGTCGCGGATTTCGACACAATTTATCAGGCGATGATACAAATCTCCGTTGTACTTTGTTTCGCG CTTGGTATAATCGCTGGGGGTCAAAGATGAGTGTTTTAGTGTATTCTTTTGCCTCTTTCGTTTTAGGTT GGTGCCTTCGTAGTGGCATTACGTATTTTACCCGTTTAATGGAAACTTCCTCATGAAAAAGTCTTTAGT CCTCAAAGCCTCTGTAGCCGTTGCTACCCTCGTTCCGATGCTGTCTTTCGCTGCTGAGGGTGACGAT CCCGCAAAAGCGGCCTTTAACTCCCTGCAAGCCTCAGCGACCGAATATATCGGTTATGCGTGGGCGA TGGTTGTTGTCATTGTCGGCGCAACTATCGGTATCAAGCTGTTTAAGAAATTCACCTCGAAAGCAAGC TGATAAACCGATACAATTAAAGGCTCCTTTTGGAGCCTTTTTTTTGGAGATTTTCAACGTGAAAAAATT ATTATTCGCAATTCCTTTAGTTGTTCCTTTCTATTCTCACTCCGCTGAAACTGTTGAAAGTTGTTTAGCA AAATCCCATACAGAAAATTCATTTACTAACGTCTGGAAAGACGACAAAACTTTAGATCGTTACGCTAAC |
| | | TATGAGGGCTGTCTGTGGAATGCTACAGGCGTTGTAGTTTGTACTGGTGACGAAACTCAGTGTTACG GTACATGGGTTCCTATTGGGCTTGCTATCCCTGAAAATGAGGGTGGTGGCTCTGAGGGTGGCGGTTC TGAGGGTGGCGGTTCTGAGGGTGGCGGTACTAAACCTCCTGAGTACGGTGATACACCTATTCCGGG CTATACTTATATCAACCCTCTCGACGGCACTTATCCGCCTGGTACTGAGCAAAACCCCGCTAATCCTA ATCCTTCTCTTGAGGAGTCTCAGCCTCTTAATACTTTCATGTTTCAGAATAATAGGTTCCGAAATAGGC AGGGGGCATTAACTGTTTATACGGGCACTGTTACTCAAGGCACTGACCCCGTTAAAACTTATTACCAG TACACTCCTGTATCATCAAAAGCCATGTATGACGCTTACTGGAACGGTAAATTCAGAGACTGCGCTTT CCATTCTGGCTTTAATGAGGATTTATTTGTTTGTGAATATCAAGGCCAATCGTCTGACCTGCCTCAACC TCCTGTCAATGCTGGCGGCGGCTCTGGTGGTGGTTCTGGTGGCGGCTCTGAGGGTGGTGGCTCTGA GGGTGGCGGTTCTGAGGGTGGCGGCTCTGAGGGAGGCGGTTCCGGTGGTGGCTCTGGTTCCGGTG ATTTTGATTATGAAAAGATGGCAAACGCTAATAAGGGGGCTATGACCGAAAATGCCGATGAAAACGC GCTACAGTCTGACGCTAAAGGCAAACTTGATTCTGTCGCTACTGATTACGGTGCTGCTATCGATGGTT TCATTGGTGACGTTTCCGGCCTTGCTAATGGTAATGGTGCTACTGGTGATTTTGCTGGCTCTAATTCC CAAATGGCTCAAGTCGGTGACGGTGATAATTCACCTTTAATGAATAATTTCCGTCAATATTTACCTTCC CTCCCTCAATCGGTTGAATGTCGCCCTTTTGTCTTTGGCGCTGGTAAACCATATGAATTTTCTATTGAT TGTGACAAAATAAACTTATTCCGTGGTGTCTTTGCGTTTCTTTTATATGTTGCCACCTTTATGTATGTAT TTTCTACGTTTGCTAACATACTGCGTAATAAGGAGTCTTAATCATGCCAGTTCTTTTGGGTATTCCGTT ATTATTGCGTTTCCTCGGTTTCCTTCTGGTAACTTTGTTCGGCTATCTGCTTACTTTTCTTAAAAAGGG CTTCGGTAAGATAGCTATTGCTATTTCATTGTTTCTTGCTCTTATTATTGGGCTTAACTCAATTCTTGTG GGTTATCTCTCTGATATTAGCGCTCAATTACCCTCTGACTTTGTTCAGGGTGTTCAGTTAATTCTCCCG TCTAATGCGCTTCCCTGTTTTTATGTTATTCTCTCTGTAAAGGCTGCTATTTTCATTTTTGACGTTAAAC AAAAAATCGTTTCTTATTTGGATTGGGATAAATAATATGGCTGTTTATTTTGTAACTGGCAAATTAGGCT CTGGAAAGACGCTCGTTAGCGTTGGTAAGATTCAGGATAAAATTGTAGCTGGGTGCAAAATAGCAACT AATCTTGATTTAAGGCTTCAAAACCTCCCGCAAGTCGGGAGGTTCGCTAAAACGCCTCGCGTTCTTAG AATACCGGATAAGCCTTCTATATCTGATTTGCTTGCTATTGGGCGCGGTAATGATTCCTACGATGAAA ATAAAAACGGCTTGCTTGTTCTCGATGAGTGCGGTACTTGGTTTAATACCCGTTCTTGGAATGATAAG GAAAGACAGCCGATTATTGATTGGTTTCTACATGCTCGTAAATTAGGATGGGATATTATTTTTCTTGTT CAGGACTTATCTATTGTTGATAAACAGGCGCGTTCTGCATTAGCTGAACATGTTGTTTATTGTCGTCGT CTGGACAGAATTACTTTACCTTTTGTCGGTACTTTATATTCTCTTATTACTGGCTCGAAAATGCCTCTG CCTAAATTACATGTTGGCGTTGTTAAATATGGCGATTCTCAATTAAGCCCTACTGTTGAGCGTTGGCTT TATACTGGTAAGAATTTGTATAACGCATATGATACTAAACAGGCTTTTTCTAGTAATTATGATTCCGGT GTTTATTCTTATTTAACGCCTTATTTATCACACGGTCGGTATTTCAAACCATTAAATTTAGGTCAGAAGA TGAAATTAACTAAAATATATTTGAAAAAGTTTTCTCGCGTTCTTTGTCTTGCGATTGGATTTGCATCAGC ATTTACATATAGTTATATAACCCAACCTAAGCCGGAGGTTAAAAAGGTAGTCTCTCAGACCTATGATTT TGATAAATTCACTATTGACTCTTCTCAGCGTCTTAATCTAAGCTATCGCTATGTTTTCAAGGATTCTAAG |
| | | GGAAAATTAATTAATAGCGACGATTTACAGAAGCAAGGTTATTCACTCACATATATTGATTTATGTACT GTTTCCATTAAAAAAGGTAATTCAAATGAAATTGTTAAATGTAATTAATTTTGTTTTCTTGATGTTTGTTT CATCATCTTCTTTTGCTCAGGTAATTGAAATGAATAATTCGCCTCTGCGCGATTTTGTAACTTGGTATT CAAAGCAATCAGGCGAATCCGTTATTGTTTCTCCCGATGTAAAAGGTACTGTTACTGTATATTCATCTG ACGTTAAACCTGAAAATCTACGCAATTTCTTTATTTCTGTTTTACGTGCAAATAATTTTGATATGGTAGG TTCTAACCCTTCCATTATTCAGAAGTATAATCCAAACAATCAGGATTATATTGATGAATTGCCATCATCT GATAATCAGGAATATGATGATAATTCCGCTCCTTCTGGTGGTTTCTTTGTTCCGCAAAATGATAATGTT ACTCAAACTTTTAAAATTAATAACGTTCGGGCAAAGGATTTAATACGAGTTGTCGAATTGTTTGTAAAG TCTAATACTTCTAAATCCTCAAATGTATTATCTATTGACGGCTCTAATCTATTAGTTGTTAGTGCTCCTA AAGATATTTTAGATAACCTTCCTCAATTCCTTTCAACTGTTGATTTGCCAACTGACCAGATATTGATTGA GGGTTTGATATTTGAGGTTCAGCAAGGTGATGCTTTAGATTTTTCATTTGCTGCTGGCTCTCAGCGTG GCACTGTTGCAGGCGGTGTTAATACTGACCGCCTCACCTCTGTTTTATCTTCTGCTGGTGGTTCGTTC GGTATTTTTAATGGCGATGTTTTAGGGCTATCAGTTCGCGCATTAAAGACTAATAGCCATTCAAAAATA TTGTCTGTGCCACGTATTCTTACGCTTTCAGGTCAGAAGGGTTCTATCTCTGTTGGCCAGAATGTCCC TTTTATTACTGGTCGTGTGACTGGTGAATCTGCCAATGTAAATAATCCATTTCAGACGATTGAGCGTCA AAATGTAGGTATTTCCATGAGCGTTTTTCCTGTTGCAATGGCTGGCGGTAATATTGTTCTGGATATTAC CAGCAAGGCCGATAGTTTGAGTTCTTCTACTCAGGCAAGTGATGTTATTACTAATCAAAGAAGTATTG CTACAACGGTTAATTTGCGTGATGGACAGACTCTTTTACTCGGTGGCCTCACTGATTATAAAAACACTT CTCAGGATTCTGGCGTACCGTTCCTGTCTAAAATCCCTTTAATCGGCCTCCTGTTTAGCTCCCGCTCT GATTCTAACGAGGAAAGCACGTTATACGTGCTCGTCAAAGCAACCATAGTACGCGCCCTGTAGCGGC GCATTAAGCGCGGCGGGTGTGGTGGTTACGCGCAGCGTGACCGCTACACTTGCCAGCGCCCTAGCG CCCGCTCCTTTCGCTTTCTTCCCTTCCTTTCTCGCCACGTTCGCCGGCTTTCCCCGTCAAGCTCTAAA TCGGGGGCTCCCTTTAGGGTTCCGATTTAGTGCTTTACGGCACCTCGACCCCAAAAAACTTGATTTGG GTGATGGTTCACGTAGTGGGCCATCGCCCTGATAGACGGTTTTTCGCCCTTTGACGTTGGAGTCCAC GTTCTTTAATAGTGGACTCTTGTTCCAAACTGGAACAACACTCAACCCTATCTCGGGCTATTCTTTTGA TTTATAAGGGATTTTGCCGATTTCGGAACCACCATCAAACAGGATTTTCGCCTGCTGGGGCAAACCAG CGTGGACCGCTTGCTGCAACTCTCTCAGGGCCAGGCGGTGAAGGGCAATCAGCTGTTGCCCGTCTC ACTGGTGAAAAGAAAAACCACCCTGGCGCCCAATACGCAAACCGCCTCTCCCCGCGCGTTGGCCGA TTCATTAATGCAGCTGGCACGACAGGTTTCCCGACTGGAAAGCGGGCAGTGAGCGCAACGCAATTAA TGTGAGTTAGCTCACTCATTAGGCACCCCAGGCTTTACACTTTATGCTTCCGGCTCGTATGTTGTGTG GAATTGTGAGCGGATAACAATTTCACACAGGAAACAGCTATGACCATGATTACGAATTCGAGCTCGGT ACCCGGGGATCCTCAACTGTGAGGAGGCTCACGGACGCGAAGAACAGGCACGCGTGCTGGCAGAA ACCCCCGGTATGACCGTGAAAACGGCCCGCCGCATTCTGGCCGCAGCACCACAGAGTGCACAGGCG CGCAGTGACACTGCGCTGGATCGTCTGATGCAGGGGGCACCGGCACCGCTGGCTGCAGGTAACCC GGCATCTGATGCCGTTAACGATTTGCTGAACACACCAGTGTAAGGGATGTTTATGACGAGCAAAGAAA |
| | | CCTTTACCCATTACCAGCCGCAGGGCAACAGTGACCCGGCTCATACCGCAACCGCGCCCGGCGGAT TGAGTGCGAAAGCGCCTGCAATGACCCCGCTGATGCTGGACACCTCCAGCCGTAAGCTGGTTGCGT GGGATGGCACCACCGACGGTGCTGCCGTTGGCATTCTTGCGGTTGCTGCTGACCAGACCAGCACCA CGCTGACGTTCTACAAGTCCGGCACGTTCCGTTATGAGGATGTGCTCTGGCCGGAGGCTGCCAGCG ACGAGACGAAAAAACGGACCGCGTTTGCCGGAACGGCAATCAGCATCGTTTAACTTTACCCTTCATC ACTAAAGGCCGCCTGTGCGGCTTTTTTTACGGGATTTTTTTATGTCGATGTACACAACCGCCCAACTG CTGGCGGCAAATGAGCAGAAATTTAAGTTTGATCCGCTGTTTCTGCGTCTCTTTTTCCGTGAGAGCTA TCCCTTCACCACGGAGAAAGTCTATCTCTCACAAATTCCGGGACTGGTAAACATGGCGCTGTACGTTT CGCCGATTGTTTCCGGTGAGGTTATCCGTTCCCGTGGCGGCTCCACCTCTGAAAGCTTGGCACTGGC CGTCGTTTTACAACGTCGTGACTGGGAAAACCCTGGCGTTACCCAACTTAATCGCCTTGCAGCACATC CCCCTTTCGCCAGCTGGCGTAATAGCGAAGAGGCCCGCACCGATCGCCCTTCCCAACAGTTGCGCA GCCTGAATGGCGAATGGCGCTTTGCCTGGTTTCCGGCACCAGAAGCGGTGCCGGAAAGCTGGCTGG AGTGCGATCTTCCTGAGGCCGATACTGTCGTCGTCCCCTCAAACTGGCAGATGCACGGTTACGATGC GCCCATCTACACCAACGTGACCTATCCCATTACGGTCAATCCGCCGTTTGTTCCCACGGAGAATCCG ACGGGTTGTTACTCGCTCACATTTAATGTTGATGAAAGCTGGCTACAGGAAGGCCAGACGCGAATTAT TTTTGATGGCGTTCCTATTGGTTAAAAAATGAGCTGATTTAACAAAAATTTAATGCGAATTTTAACAAAA TATTAACGTTTACAATTTAAATATTTGCTTATACAATCTTCCTGTTTTTGGGGCTTTTCTGATTATCAACC GGGGTACATATGATTGACATGCTAGTTTTACGATTACCGTTCATCGATTCTCTTGTTTGCTCCAGACTC TCA |
| 2 | M13 7249 | TGATAGACGGTTTTTCGCCCTTTGACGTTGGAGTCCACGTTCTTTAATAGTGGACTCTTGTTCCAAACT GGAACAACACTCAACCCTATCTCGGGCTATTCTTTTGATTTATAAGGGATTTTGCCGATTTCGGAACCA CCATCAAACAGGATTTTCGCCTGCTGGGGCAAACCAGCGTGGACCGCTTGCTGCAACTCTCTCAGGG CCAGGCGGTGAAGGGCAATCAGCTGTTGCCCGTCTCACTGGTGAAAAGAAAAACCACCCTGGCGCC CAATACGCAAACCGCCTCTCCCCGCGCGTTGGCCGATTCATTAATGCAGCTGGCACGACAGGTTTCC CGACTGGAAAGCGGGCAGTGAGCGCAACGCAATTAATGTGAGTTAGCTCACTCATTAGGCACCCCAG GCTTTACACTTTATGCTTCCGGCTCGTATGTTGTGTGGAATTGTGAGCGGATAACAATTTCACACAGG AAACAGCTATGACCATGATTACGAATTCGAGCTCGGTACCCGGGGATCCTCTAGAGTCGACCTGCAG GCATGCAAGCTTGGCACTGGCCGTCGTTTTACAACGTCGTGACTGGGAAAACCCTGGCGTTACCCAA CTTAATCGCCTTGCAGCACATCCCCCTTTCGCCAGCTGGCGTAATAGCGAAGAGGCCCGCACCGATC GCCCTTCCCAACAGTTGCGCAGCCTGAATGGCGAATGGCGCTTTGCCTGGTTTCCGGCACCAGAAG CGGTGCCGGAAAGCTGGCTGGAGTGCGATCTTCCTGAGGCCGATACTGTCGTCGTCCCCTCAAACT GGCAGATGCACGGTTACGATGCGCCCATCTACACCAACGTGACCTATCCCATTACGGTCAATCCGCC GTTTGTTCCCACGGAGAATCCGACGGGTTGTTACTCGCTCACATTTAATGTTGATGAAAGCTGGCTAC AGGAAGGCCAGACGCGAATTATTTTTGATGGCGTTCCTATTGGTTAAAAAATGAGCTGATTTAACAAA AATTTAATGCGAATTTTAACAAAATATTAACGTTTACAATTTAAATATTTGCTTATACAATCTTCCTGTTT |
| | | TTGGGGCTTTTCTGATTATCAACCGGGGTACATATGATTGACATGCTAGTTTTACGATTACCGTTCATC GATTCTCTTGTTTGCTCCAGACTCTCAGGCAATGACCTGATAGCCTTTGTAGATCTCTCAAAAATAGCT ACCCTCTCCGGCATTAATTTATCAGCTAGAACGGTTGAATATCATATTGATGGTGATTTGACTGTCTCC GGCCTTTCTCACCCTTTTGAATCTTTACCTACACATTACTCAGGCATTGCATTTAAAATATATGAGGGT TCTAAAAATTTTTATCCTTGCGTTGAAATAAAGGCTTCTCCCGCAAAAGTATTACAGGGTCATAATGTT TTTGGTACAACCGATTTAGCTTTATGCTCTGAGGCTTTATTGCTTAATTTTGCTAATTCTTTGCCTTGCC TGTATGATTTATTGGATGTTAATGCTACTACTATTAGTAGAATTGATGCCACCTTTTCAGCTCGCGCCC CAAATGAAAATATAGCTAAACAGGTTATTGACCATTTGCGAAATGTATCTAATGGTCAAACTAAATCTA CTCGTTCGCAGAATTGGGAATCAACTGTTATATGGAATGAAACTTCCAGACACCGTACTTTAGTTGCA TATTTAAAACATGTTGAGCTACAGCATTATATTCAGCAATTAAGCTCTAAGCCATCCGCAAAAATGACC TCTTATCAAAAGGAGCAATTAAAGGTACTCTCTAATCCTGACCTGTTGGAGTTTGCTTCCGGTCTGGTT CGCTTTGAAGCTCGAATTAAAACGCGATATTTGAAGTCTTTCGGGCTTCCTCTTAATCTTTTTGATGCA ATCCGCTTTGCTTCTGACTATAATAGTCAGGGTAAAGACCTGATTTTTGATTTATGGTCATTCTCGTTTT CTGAACTGTTTAAAGCATTTGAGGGGGATTCAATGAATATTTATGACGATTCCGCAGTATTGGACGCT ATCCAGTCTAAACATTTTACTATTACCCCCTCTGGCAAAACTTCTTTTGCAAAAGCCTCTCGCTATTTT GGTTTTTATCGTCGTCTGGTAAACGAGGGTTATGATAGTGTTGCTCTTACTATGCCTCGTAATTCCTTT TGGCGTTATGTATCTGCATTAGTTGAATGTGGTATTCCTAAATCTCAACTGATGAATCTTTCTACCTGT AATAATGTTGTTCCGTTAGTTCGTTTTATTAACGTAGATTTTTCTTCCCAACGTCCTGACTGGTATAATG AGCCAGTTCTTAAAATCGCATAAGGTAATTCACAATGATTAAAGTTGAAATTAAACCATCTCAAGCCCA ATTTACTACTCGTTCTGGTGTTTCTCGTCAGGGCAAGCCTTATTCACTGAATGAGCAGCTTTGTTACGT TGATTTGGGTAATGAATATCCGGTTCTTGTCAAGATTACTCTTGATGAAGGTCAGCCAGCCTATGCGC CTGGTCTGTACACCGTTCATCTGTCCTCTTTCAAAGTTGGTCAGTTCGGTTCCCTTATGATTGACCGT CTGCGCCTCGTTCCGGCTAAGTAACATGGAGCAGGTCGCGGATTTCGACACAATTTATCAGGCGATG ATACAAATCTCCGTTGTACTTTGTTTCGCGCTTGGTATAATCGCTGGGGGTCAAAGATGAGTGTTTTA GTGTATTCTTTTGCCTCTTTCGTTTTAGGTTGGTGCCTTCGTAGTGGCATTACGTATTTTACCCGTTTA ATGGAAACTTCCTCATGAAAAAGTCTTTAGTCCTCAAAGCCTCTGTAGCCGTTGCTACCCTCGTTCCG ATGCTGTCTTTCGCTGCTGAGGGTGACGATCCCGCAAAAGCGGCCTTTAACTCCCTGCAAGCCTCAG CGACCGAATATATCGGTTATGCGTGGGCGATGGTTGTTGTCATTGTCGGCGCAACTATCGGTATCAA GCTGTTTAAGAAATTCACCTCGAAAGCAAGCTGATAAACCGATACAATTAAAGGCTCCTTTTGGAGCC TTTTTTTTGGAGATTTTCAACGTGAAAAAATTATTATTCGCAATTCCTTTAGTTGTTCCTTTCTATTCTCA CTCCGCTGAAACTGTTGAAAGTTGTTTAGCAAAATCCCATACAGAAAATTCATTTACTAACGTCTGGAA AGACGACAAAACTTTAGATCGTTACGCTAACTATGAGGGCTGTCTGTGGAATGCTACAGGCGTTGTAG TTTGTACTGGTGACGAAACTCAGTGTTACGGTACATGGGTTCCTATTGGGCTTGCTATCCCTGAAAAT GAGGGTGGTGGCTCTGAGGGTGGCGGTTCTGAGGGTGGCGGTTCTGAGGGTGGCGGTACTAAACC TCCTGAGTACGGTGATACACCTATTCCGGGCTATACTTATATCAACCCTCTCGACGGCACTTATCCGC |
| | | CTGGTACTGAGCAAAACCCCGCTAATCCTAATCCTTCTCTTGAGGAGTCTCAGCCTCTTAATACTTTCA TGTTTCAGAATAATAGGTTCCGAAATAGGCAGGGGGCATTAACTGTTTATACGGGCACTGTTACTCAA GGCACTGACCCCGTTAAAACTTATTACCAGTACACTCCTGTATCATCAAAAGCCATGTATGACGCTTA CTGGAACGGTAAATTCAGAGACTGCGCTTTCCATTCTGGCTTTAATGAGGATTTATTTGTTTGTGAATA TCAAGGCCAATCGTCTGACCTGCCTCAACCTCCTGTCAATGCTGGCGGCGGCTCTGGTGGTGGTTCT GGTGGCGGCTCTGAGGGTGGTGGCTCTGAGGGTGGCGGTTCTGAGGGTGGCGGCTCTGAGGGAGG CGGTTCCGGTGGTGGCTCTGGTTCCGGTGATTTTGATTATGAAAAGATGGCAAACGCTAATAAGGGG GCTATGACCGAAAATGCCGATGAAAACGCGCTACAGTCTGACGCTAAAGGCAAACTTGATTCTGTCG CTACTGATTACGGTGCTGCTATCGATGGTTTCATTGGTGACGTTTCCGGCCTTGCTAATGGTAATGGT GCTACTGGTGATTTTGCTGGCTCTAATTCCCAAATGGCTCAAGTCGGTGACGGTGATAATTCACCTTT AATGAATAATTTCCGTCAATATTTACCTTCCCTCCCTCAATCGGTTGAATGTCGCCCTTTTGTCTTTGG CGCTGGTAAACCATATGAATTTTCTATTGATTGTGACAAAATAAACTTATTCCGTGGTGTCTTTGCGTT TCTTTTATATGTTGCCACCTTTATGTATGTATTTTCTACGTTTGCTAACATACTGCGTAATAAGGAGTCT TAATCATGCCAGTTCTTTTGGGTATTCCGTTATTATTGCGTTTCCTCGGTTTCCTTCTGGTAACTTTGTT CGGCTATCTGCTTACTTTTCTTAAAAAGGGCTTCGGTAAGATAGCTATTGCTATTTCATTGTTTCTTGC TCTTATTATTGGGCTTAACTCAATTCTTGTGGGTTATCTCTCTGATATTAGCGCTCAATTACCCTCTGA CTTTGTTCAGGGTGTTCAGTTAATTCTCCCGTCTAATGCGCTTCCCTGTTTTTATGTTATTCTCTCTGTA AAGGCTGCTATTTTCATTTTTGACGTTAAACAAAAAATCGTTTCTTATTTGGATTGGGATAAATAATATG GCTGTTTATTTTGTAACTGGCAAATTAGGCTCTGGAAAGACGCTCGTTAGCGTTGGTAAGATTCAGGA TAAAATTGTAGCTGGGTGCAAAATAGCAACTAATCTTGATTTAAGGCTTCAAAACCTCCCGCAAGTCG GGAGGTTCGCTAAAACGCCTCGCGTTCTTAGAATACCGGATAAGCCTTCTATATCTGATTTGCTTGCT ATTGGGCGCGGTAATGATTCCTACGATGAAAATAAAAACGGCTTGCTTGTTCTCGATGAGTGCGGTAC TTGGTTTAATACCCGTTCTTGGAATGATAAGGAAAGACAGCCGATTATTGATTGGTTTCTACATGCTCG TAAATTAGGATGGGATATTATTTTTCTTGTTCAGGACTTATCTATTGTTGATAAACAGGCGCGTTCTGC ATTAGCTGAACATGTTGTTTATTGTCGTCGTCTGGACAGAATTACTTTACCTTTTGTCGGTACTTTATAT TCTCTTATTACTGGCTCGAAAATGCCTCTGCCTAAATTACATGTTGGCGTTGTTAAATATGGCGATTCT CAATTAAGCCCTACTGTTGAGCGTTGGCTTTATACTGGTAAGAATTTGTATAACGCATATGATACTAAA CAGGCTTTTTCTAGTAATTATGATTCCGGTGTTTATTCTTATTTAACGCCTTATTTATCACACGGTCGGT ATTTCAAACCATTAAATTTAGGTCAGAAGATGAAATTAACTAAAATATATTTGAAAAAGTTTTCTCGCGT TCTTTGTCTTGCGATTGGATTTGCATCAGCATTTACATATAGTTATATAACCCAACCTAAGCCGGAGGT TAAAAAGGTAGTCTCTCAGACCTATGATTTTGATAAATTCACTATTGACTCTTCTCAGCGTCTTAATCTA AGCTATCGCTATGTTTTCAAGGATTCTAAGGGAAAATTAATTAATAGCGACGATTTACAGAAGCAAGGT TATTCACTCACATATATTGATTTATGTACTGTTTCCATTAAAAAAGGTAATTCAAATGAAATTGTTAAATG TAATTAATTTTGTTTTCTTGATGTTTGTTTCATCATCTTCTTTTGCTCAGGTAATTGAAATGAATAATTCG CCTCTGCGCGATTTTGTAACTTGGTATTCAAAGCAATCAGGCGAATCCGTTATTGTTTCTCCCGATGTA |
| | | AAAGGTACTGTTACTGTATATTCATCTGACGTTAAACCTGAAAATCTACGCAATTTCTTTATTTCTGTTT TACGTGCAAATAATTTTGATATGGTAGGTTCTAACCCTTCCATTATTCAGAAGTATAATCCAAACAATCA GGATTATATTGATGAATTGCCATCATCTGATAATCAGGAATATGATGATAATTCCGCTCCTTCTGGTGG TTTCTTTGTTCCGCAAAATGATAATGTTACTCAAACTTTTAAAATTAATAACGTTCGGGCAAAGGATTTA ATACGAGTTGTCGAATTGTTTGTAAAGTCTAATACTTCTAAATCCTCAAATGTATTATCTATTGACGGCT CTAATCTATTAGTTGTTAGTGCTCCTAAAGATATTTTAGATAACCTTCCTCAATTCCTTTCAACTGTTGA TTTGCCAACTGACCAGATATTGATTGAGGGTTTGATATTTGAGGTTCAGCAAGGTGATGCTTTAGATTT TTCATTTGCTGCTGGCTCTCAGCGTGGCACTGTTGCAGGCGGTGTTAATACTGACCGCCTCACCTCT GTTTTATCTTCTGCTGGTGGTTCGTTCGGTATTTTTAATGGCGATGTTTTAGGGCTATCAGTTCGCGCA TTAAAGACTAATAGCCATTCAAAAATATTGTCTGTGCCACGTATTCTTACGCTTTCAGGTCAGAAGGGT TCTATCTCTGTTGGCCAGAATGTCCCTTTTATTACTGGTCGTGTGACTGGTGAATCTGCCAATGTAAAT AATCCATTTCAGACGATTGAGCGTCAAAATGTAGGTATTTCCATGAGCGTTTTTCCTGTTGCAATGGCT GGCGGTAATATTGTTCTGGATATTACCAGCAAGGCCGATAGTTTGAGTTCTTCTACTCAGGCAAGTGA TGTTATTACTAATCAAAGAAGTATTGCTACAACGGTTAATTTGCGTGATGGACAGACTCTTTTACTCGG TGGCCTCACTGATTATAAAAACACTTCTCAGGATTCTGGCGTACCGTTCCTGTCTAAAATCCCTTTAAT CGGCCTCCTGTTTAGCTCCCGCTCTGATTCTAACGAGGAAAGCACGTTATACGTGCTCGTCAAAGCA ACCATAGTACGCGCCCTGTAGCGGCGCATTAAGCGCGGCGGGTGTGGTGGTTACGCGCAGCGTGAC CGCTACACTTGCCAGCGCCCTAGCGCCCGCTCCTTTCGCTTTCTTCCCTTCCTTTCTCGCCACGTTCG CCGGCTTTCCCCGTCAAGCTCTAAATCGGGGGCTCCCTTTAGGGTTCCGATTTAGTGCTTTACGGCA CCTCGACCCCAAAAAACTTGATTTGGGTGATGGTTCACGTAGTGGGCCATCGCCC |
| | | |

| | **T**_**octa** | |
|---|---|---|
| 3 to 154 | core | Core_1 to core_152 (see sequence listing) |
| 155 to 160 | side1_recess | side1_recess_1 to side1_recess_6 (see sequence listing) |
| 161 to 168 | side1_protrusion | side1_protrusion_1 to side1_ protrusion _8 (see sequence |
| | | listing) |
| 169 to 174 | side2_recess | side2_recess_1 to side2_recess_6 (see sequence listing) |
| 175 to 182 | side2_protrusion | side2_protrusion_1 to side2_ protrusion _8 (see sequence listing) |
| 183 to 188 | side3_recess | side3_recess_1 to side3_recess_6 (see sequence listing) |
| 189 to 196 | side3_protrusion | side3_protrusion_1 to side3_ protrusion _8 (see sequence listing) |

| | **T=1** | |
|---|---|---|
| 197 to 351 | core | core_1 to core_155 (see sequence listing) |
| 352 to 358 | side1_recess | side1_recess_1 to side1_recess_7 (see sequence listing) |
| 359 to 365 | side1_protrusion | side1_protrusion_1 to side1_ protrusion _7 (see sequence |
| | | listing) |
| 366 to 372 | side2_recess | side2_recess_1 to side2_recess_7 (see sequence listing) |
| 373 to 379 | side2_protrusion | side2_protrusion_1 to side2_ protrusion _7 (see sequence listing) |
| 380 to 386 | side3_recess | side3_recess_1 to side3_recess_7 (see sequence listing) |
| 387 to 393 | side3_protrusion | side3_protrusion_1 to side3_ protrusion _7 (see sequence listing) |

| | **T=1 (FRET)** | |
|---|---|---|
| 394 to 548 | core | core_1 to core_155 (see sequence listing) |
| 549 to 555 | side1_recess | side1_recess_1 to side1_recess_7 (see sequence listing) |
| 556 to 562 | side1_protrusion | side1_protrusion_1 to side1_ protrusion _7 (see sequence |
| | | listing) |
| 563 to 569 | side2_recess | side2_recess_1 to side2_recess_7 (see sequence listing) |
| 570 to 576 | side2_protrusion | side2_protrusion_1 to side2_ protrusion _7 (see sequence listing) |
| 577 to 583 | side3_recess | side3_recess_1 to side3_recess_7 (see sequence listing) |
| 584 to 590 | side3_protrusion | side3_protrusion_1 to side3_ protrusion _7 (see sequence listing) |

| | **T=1 (-5°)** | |
|---|---|---|
| 591 to 746 | core | core_1 to core_156 (see sequence listing) |
| 747 to 753 | side1_recess | side1_recess_1 to side1_recess_7 (see sequence listing) |
| 754 to 760 | side1_protrusion | side1_protrusion_1 to side1_ protrusion _7 (see sequence |
| | | listing) |
| 761 to 767 | side2_recess | side2_recess_1 to side2_recess_7 (see sequence listing) |
| 768 to 774 | side2_protrusion | side2_protrusion_1 to side2_ protrusion _7 (see sequence listing) |
| 775 to 781 | side3_recess | side3_recess_1 to side3_recess_7 (see sequence listing) |
| 782 to 788 | side3_protrusion | side3_protrusion_1 to side3_ protrusion _7 (see sequence listing) |

| | **T=1 (+5°)** | |
|---|---|---|
| 789 to 940 | core | core_1 to core_152 (see sequence listing) |
| 941 to 947 | side1_recess | side1_recess_1 to side1_recess_7 (see sequence listing) |
| 948 to 954 | side1_protrusion | side1_protrusion_1 to side1_ protrusion _7 (see sequence |
| | | listing) |
| 955 to 961 | side2_recess | side2_recess_1 to side2_recess_7 (see sequence listing) |
| 962 to 968 | side2_protrusion | side2_protrusion_1 to side2_ protrusion _7 (see sequence listing) |
| 969 to 985 | side3_recess | side3_recess_1 to side3_recess_7 (see sequence listing) |
| 976 to 982 | side3_protrusion | side3_protrusion_1 to side3_ protrusion _7 (see sequence listing) |

| | **T=3** | |
|---|---|---|
| 983 to 1145 | core | core_1 to core_163 (see sequence listing) |
| 1146 to 1153 | side1_protrusion | side1_protrusion_1 to side1_ protrusion _8 (see sequence listing) |
| 1154 to 1161 | side2_recess | side2_recess_1 to side2_recess_8 (see sequence listing) |
| 1162 to 1168 | side3_recess | side3_recess_1 to side3_recess_7 (see sequence listing) |
| 1169 to 1175 | side3_protrusion | side3_protrusion_1 to side3_ protrusion _7 (see sequence listing) |

| | **T=4 (iso)** | |
|---|---|---|
| 1176 to 1343 | core | core_1 to core_168 (see sequence listing) |
| 1344 to 1351 | side1_protrusion | side1_protrusion_1 to side1_ protrusion _8 (see sequence listing) |
| 1352 to 1359 | side2_recess | side2_recess_1 to side2_recess_8 (see sequence listing) |
| 1360 to 1367 | side3_protrusion | side3_protrusion_1 to side3_protrusion _8 (see sequence listing) |
| | **T=4 (equi)** | |
| 1368 to 1538 | core | core_1 to core_171 (see sequence listing) |
| 1539 to 1545 | side1_recess | side1_recess_1 to side1_recess_7 (see sequence listing) |
| 1546 to 1552 | side2_recess | side2_recess_1 to side2_recess_7 (see sequence listing) |
| 1553 to 1559 | side3_recess | side3_recess_1 to side3_recess_7 (see sequence listing) |
| | **T=9 (pent)** | |
| 1560 to 1705 | core | core_1 to core_146 (see sequence listing) |
| 1706 to 1713 | side1_protrusion | side1_protrusion_1 to side1_ protrusion _8 (see sequence listing) |
| 1714 to 1721 | side2_recess | side2_recess_1 to side2_recess_8 (see sequence listing) |
| 1722 to 1728 | side3_protrusion | side3_protrusion_1 to side3_protrusion _7 (see sequence listing) |
| | **T=9 (hex1)** | |
| 1729 to 1891 | core | core_1 to core_163 (see sequence listing) |
| 1892 to 1899 | side1_protrusion | side1_protrusion_1 to side1_ protrusion _8 (see sequence listing) |
| 1900 to 1907 | side2_recess | side2_recess_1 to side2_recess_8 (see sequence listing) |
| 1908 to 1915 | side3_protrusion | side3_recess_1 to side3_recess_8 (see sequence listing) |
| | **T=9 (hex1) broken** | |
| 1916 to 2078 | core | core_1 to core_163 (see sequence listing) |
| 2079 to 2086 | side1_protrusion | side1_protrusion_1 to side1_ protrusion _8 (see sequence listing) |
| 2087 to 2094 | side2_recess | side2_recess_1 to side2_recess_8 (see sequence listing) |
| 2095 to 2102 | side3_protrusion | side3_recess_1 to side3_recess_8 (see sequence listing) |
| | **T=9 (hex2)** | |
| 2103 to 2266 | core | core_1 to core_164 (see sequence listing) |
| 2267 to 2274 | side1_protrusion | side1_protrusion_1 to side1_ protrusion _8 (see sequence listing) |
| 2275 to 2282 | side2_recess | side2_recess_1 to side2_recess_8 (see sequence listing) |
| 2283 to 2290 | side3_recess/protru sion | side3_recess/protrusion_1 to side3_recess/protrusion_8 (see sequence listing) |

| | **T_octa (half)** | |
|---|---|---|
| 2291 to 2436 | core | core_1 to core_146 (see sequence listing); core to be combined with option A1 or A2 and option B1 or B2 |
| 2437 to 2454 | A1: antibody attachment | AB_attach_1 to AB_attach_18 (see sequence listing); AB_attach_18 (SEQ ID NO: 2454) to be used instead of side3_protrusion_4 (SEQ ID NO: 2496 or 2525) |
| 2455 to 2471 | A2: no antibody attachment | no_AB_attach_1 to no_AB_attach_17 (see sequence listing) |
| 2472 to 2478 | B1: stacking contacts all active: side1_protrusion | side1_protrusion_1 to side1_protrusion_7 (see sequence listing) |
| 2479 to 2486 | B1: stacking contacts all active: side2_recess | side2_recess_1 to side2_recess_8 (see sequence listing) |
| 2487 to 2492 | B1: stacking contacts all active: side3_recess | side3_recess_1 to side3 (see sequence listing) |
| 2493 to 2500 | B1: stacking contacts all active: | side3_protrusion_1 to side3_protrusion_8 (see sequence listing) |
| | side3_protrusion | |
| 2501 to 2507 | B2: stacking contact passivated with polyT: side1_protrusion | side1_protrusion_1 to side1_protrusion_7 (see sequence listing) |
| 2508 to 2515 | B2: stacking contact passivated with polyT: side2_recess | side2_recess_1 to side2_recess_8 (see sequence listing) |
| 2516 to 2521 | B2: stacking contact passivated with polyT: side3_recess | side3_recess_1 to side3_recess_6 (see sequence listing) |
| 2522 to 2529 | B2: stacking contact passivated with polyT: side3_protrusion | side3_protrusion_1 to side3 (see sequence listing) |

| | **T_ pent T=1 (2 or 3 triangles)** | |
|---|---|---|
| 2530 to 2691 | core | core_1 to core_162 (see sequence listing); core to be combined with option A1 or A2 and option B1, B2 or B3 |
| 2692 to 2702 | A1: antibody attachment | AB_attach_1 to AB_attach_11 (see sequence listing) |
| 2703 to 2713 | A2: no antibody attachment | no_AB_attach_1 to no_AB_attach_11 (see sequence listing) |
| 2714 to 2720 | B1: stacking contacts all active: side1 _protrusion | side1_protrusion_2 to side1_protrusion_8 (see sequence listing) |
| 2721 to 2728 | B1: stacking contacts all active: side2_recess | side2_recess_1 to side2_recess_8 (see sequence listing) |
| 2729 to 2735 | B1: stacking contacts all active: side3_recess | side3_recess_1 to side3_recess_7 (see sequence listing) |
| 2736 to 2742 | B2: stacking contact passivated with polyT; or B3: stacking contact sticky overhangs for T_ring_T=1 (2 triangles) : side1_protrusion | side1_protrusion_2 to side1_protrusion_8 (see sequence listing) |
| 2743 to 2750 | B2: stacking contact passivated with polyT; or B3: stacking contact sticky overhangs for T_ring_T=1 (2 triangles) : side2_recess | side2_recess_1 to side2_recess_8 (see sequence listing) |
| 2751 to 2757 | B2: stacking contact passivated with polyT: side3_recess | side3_recess_1 to side3_recess_7 (see sequence listing) |
| 2758 to 2765 | B3: stacking contact with sticky overhangs for T_ring_T=1 (2 triangles) : side3_recess | side3_recess_1 to side3_recess_8 (see sequence listing); side3_recess_5 (SEQ ID NO: 2762), side3_recess_7 (SEQ ID NO: 2764) and side3_recess_8 (SEQ ID NO: 2765) to be used instead of core_129 (SEQ ID NO: 2658) and core_142 (SEQ ID 2671) |

| | **T_ring T=1 (2 triangles)** | |
|---|---|---|
| 2766 to 2914 | core | core_1 to core_149 (see sequence listing); core to be combined with option A1 or A2 and option B1, B2 or B3 |
| 2915 to 2929 | A1: antibody attachment | AB_attach_1 to AB_attach_15 (see sequence listing) |
| 2930 to 2943 | A2: no antibody attachment | no_AB_attach_1 to no_AB_attach_14 (see sequence listing) (plus AB_attach_15) |
| 2944 to 2950 | B1: stacking contacts all active: side1_recess | side1_recess_1 to side1_recess_7 (see sequence listing) |
| 2951 to 2957 | B1: stacking contacts all active: side1_protrusion | side1_protrusion_1 to side1_protrusion_7 (see sequence listing) |
| 2958 to 2964 | B1: stacking contacts all active: side2_recess | side2_recess_1 to side2_recess_7 (see sequence listing) |
| 2965 to 2971 | B1: stacking contacts all active: side2_protrusion | side2_protrusion_1 to side2_protrusion_7 (see sequence listing) |
| 2972 to 2979 | B1: stacking contacts all active: side3_protrusion | side3_protrusion_1 to side3_protrusion_8 (see sequence listing) |
| 2980 to 2986 | B2: stacking contact passivated polyT; or B3: stacking contact with 3 bases removed for T_pent T=1 (2 or 3 triangles): side1_recess | side1_recess_1 to side1_recess_7(see sequence listing) |
| 2987 to 2993 | B2: stacking contact passivated polyT; or B3: stacking contact with 3 bases removed for T_pent T=1 (2 or 3 triangles): side1_protrusion | side1_protrusion_1 to side1_protrusion_7 (see sequence listing) |
| 2994 to 3000 | B2: stacking contact passivated polyT; or B3: stacking contact with 3 bases removed for T_pent T=1 (2 or 3 triangles): side2_recess | side2_recess_1 to side2_recess_7 (see sequence listing) |
| 3001 to 3007 | B2: stacking contact passivated polyT; or B3: stacking contact with 3 bases removed for T_pent T=1 (2 or 3 triangles): side2_protrusion | side2_protrusion_1 to side2_protrusion_7 (see sequence listing) |
| 3008 to 3015 | B2: stacking contact passivated with polyT: side3_protrusion | side3_protrusion_1 to side3_protrusion_8 (see sequence listing) |
| 3016 to 3023 | B3: stacking contact with 3 bases removed for T_pent T=1 (2 or 3 triangles) : side3_protrusion | side3_protrusion_1 to side3_protrusion_8 (see sequence listing) |

| | **T_ring1 T=1 (3 triangles)** | |
|---|---|---|
| 3024 to 3190 | core | core_1 to core_167 (see sequence listing); core to be combined |
| | | with option A1 or A2 and option B1 or B2 |
| 3191 to 3199 | A1: antibody attachment | AB_attach_1 to AB_attach_9 (see sequence listing) |
| 3200 to 3208 | A2: no antibody attachment | no_AB_attach_1 to no_AB_attach_9 (see sequence listing) |
| 3209 to 3215 | B1: stacking contacts all active: side1_protrusion | side1_protrusion_1 to side1_protrusion_7 (see sequence listing) |
| 3216 to 3223 | B1: stacking contacts all active: side2_recess | side2_recess_1 to side2_recess_8 (see sequence listing) |
| 3224 to 3231 | B1: stacking contacts all active: side3_protrusion | side3_protrusion_1 to side3_protrusion_8 (see sequence listing) |
| 3232 to 3238 | B2: stacking contact passivated with polyT: side1_protrusion | side1_protrusion_1 to side1_protrusion_7 (see sequence listing) |
| 3239 to 3246 | B2: stacking contact passivated with polyT: side2_recess | side2_recess_1 to side2_recess_8 (see sequence listing) |
| 3247 to 3254 | B2: stacking contact passivated with polyT: side3_protrusion | side3_protrusion_1 to side3_protrusion_8 (see sequence listing) |

| | **T_ring2 T=1 (3 triangles)** | |
|---|---|---|
| 3255 to 3421 | core | core_1 to core_167 (see sequence listing); core to be combined with option A1 or A2 and option B1 or B2 |
| 3422 to 3429 | A1: antibody attachment | AB_attach_1 to AB_attach_8 (see sequence listing) |
| 3430 to 3437 | A2: no antibody attachment | no_AB_attach_1 to no_AB_attach_8 (see sequence listing) |
| 3438 to 3446 | B1: stacking contacts all active: side1**_**recess | side1_recess_1 to side1_recess_8 (see sequence listing) |
| 3447 to 3454 | B1: stacking contacts all active: side2_protrusion | side2_protrusion_1 to side2_protrusion_8 (see sequence listing) |
| 3455 to 3462 | B1: stacking contacts all active: | side3_protrusion_1 to side3_protrusion_8 (see sequence listing) |
| | side3_protrusion | |
| 3463 to 3470 | B2: stacking contact passivated with polyT: side1**_**recess | side1_recess_1 to side1_recess_8 (see sequence listing) |
| 3471 to 3478 | B2: stacking contact passivated with polyT: side2_protrusion | side2_protrusion_1 to side2_protrusion_8 (see sequence listing) |
| 3479 to 3486 | B2: stacking contact passivated with polyT: side3_protrusion | side3_protrusion_1 to side3_protrusion_8 (see sequence listing) |

| | **triangular brick** | |
|---|---|---|
| 3487 to 3630 | Tail core | core_1 to core_144 (see sequence listing) |
| 3631 to 3663 | PolyT back | back_1 to back_33 (see sequence listing) |
| 3664 to 3684 | 3T PolyT front | front_1 to front_21 (see sequence listing) |
| 3685 to 3696 | attach PolyT f TailStick | tailstick_1 to tailstick_12 (see sequence listing) |
| | **T=1 for triangular brick** | |
| 3697 to 3754 | core side 1 | core_1 to core_58 (see sequence listing) |
| 3755 to 3812 | core side 2 | core_1 to core_58 (see sequence listing) |
| 3813 to 3870 | core side 3 | core_1 to core_58 (see sequence listing) |
| 3871 to 3888 | attach core T1 sticky | sticky_1 to sticky_18 (see sequence listing) |
| 3689 to 3892 | 3T connect side1 | side1_I1 h51; side1_I2 h45; side1_O1_h13; side1_O2_h18 (see sequence listing) |
| 3893 to 3896 | 3T connect side2 | side2_I1_h43; side2_I2_h37; side2_O1_h25; side2_O2_h30 (see sequence listing) |
| 3897 to 3900 | 3T connect side3 | side3_I1_h59; side3_I2_h53; side3_O1_h1; side3_O2_h6 (see sequence listing) |

| | **octahedron for in vitro condition** | |
|---|---|---|
| 3901 to 4028 | core | core_1 to core_128 (see sequence listing) |
| 4029 to 4036 | hubble S1 | S1_1 to S1_8 (see sequence listing) |
| 4037 to 4044 | hubble S2 | S2_1 to S2_8 (see sequence listing) |
| 4045 to 4052 | hubble S3 | S3_1 to S3_8 (see sequence listing) |
| 4053 to 4058 | hole S1 | S1_1 to S1_6 (see sequence listing) |
| 4059 to 4064 | hole S2 | S2_1 to S2_6 (see sequence listing) |
| 4065 to 4070 | hole S3 | S3_1 to S3_6 (see sequence listing) |
| 4071 to 4073 | as feature | feature_1 to feature_3 (see sequence listing) |
| 4074 to 4079 | hd h11 1 | S1 to S3; S1_buddy to S3_buddy (see sequence listing) |
| 4080 to 4083 | hd h11 2 | S1_buddy to S3_buddy (see sequence listing) |
| 4083 to 4088 | hd h12 | S1 to S3; S1_buddy to S3_buddy (see sequence listing) |
| 4089 to 4094 | hd h22 | S1 to S3; S1_buddy to S3_buddy (see sequence listing) |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### EXAMPLES

### Introduction

We report the successful design of a suite of molecular building blocks inspired by nature that self-assemble into a wide family of virus-sized icosahedral shells. The blocks, constructed from DNA origami, are designed according to symmetry principles that dictate the minimal number of distinct bonds per icosahedral shell, and have programmable lock-and-key interaction patterns and strengths. These programmable shells could provide a novel route to virus deactivation, premised on the concept of trapping whole viruses in pre-designed macromolecular shells for effectively blocking molecular interactions between virus and host cells. The shells form robustly, with few defects and at high yields, as we validated by cryo electron microscopy and electrophoretic mobility shift assays. We used hepatitis B virus core particles to demonstrate the feasibility of our virus-trapping and interaction-blocking concept. Realizing the promise of our novel methods for fighting viral infections holds potential for reducing disease, health care costs, and lowering economic burden caused by currently untreatable viral diseases. In addition, our methods hold particular potential for treating emerging and as-yet undescribed viruses, because our method is not dependent on any particular viral structure or host-virus interaction. We show examples for further expanding the functionality of the shells to create "smart" antigen-responsive agents. In addition to antiviral agents, potential applications of these shells include building compartmentalized systems to use as antigen-carriers for vaccination, drug delivery vehicles, protective storage containers and synthetic organelles.

To develop our antiviral concept we considered how the mammalian immune defense can protect us against invading viral pathogens. On the one hand, neutralizing antibodies can emerge that bind to specific antigens on viruses and then can block infection, for example by inhibiting interactions of the virus with a host-cell receptor, or by binding to a viral capsid in order to inhibit uncoating of the genome. Unfortunately, most of the antibodies developed in our body in response to a pathogen do not have neutralizing activity. In addition, it takes time for the host to produce effective neutralizing antibodies. During this period the virus is replicating unchecked and can spread or even establish persistence and may be transmitted to other individuals. Some viral invaders have even evolved mechanisms to escape antibody neutralization. For example, HIV shields sensitive surface regions by flexible protein chains, and HCV engulfs itself into lipids (7, 8).

Antibodies are much smaller than viruses and generally only cover small patches on the surface of the virus per antibody. On the other hand, mammalian cells also produce interferons and (retro-)viral restriction factors that can inhibit invading viruses, as exemplified by the natural restriction factor TRIM5***α***, which assembles into a hexagonal shell surrounding entire HIV-1 retroviral capsids (9, 10). The TRIM5***α*** example shows that virus encapsulation in principle could be an effective therapeutic strategy, but the natural TRIM5***α*** lattice is highly specific and cannot be readily modified and adapted to different viruses.

Our route to virus deactivation is premised on re-creating and integrating synthetically the functional aspects of neutralizing antibodies and those of lattice-forming natural restriction factors such as TRIM5***α***. We envision trapping the viruses within *de novo* designed macromolecular shells to cover large areas of viral surfaces to effectively block molecular interactions between virus and host cells. We envision shells whose interiors allow modular, highly multivalent attachment of virus-recognizing moieties in virus-commensurate symmetries. The same type of shell "platform" could then be used modularly to target a variety of viruses. Because affinity increases exponentially with number of bonds formed (11), the multivalent attachment allows exploiting avidity to effectively trap viruses in shells functionalized with individually weakly virus-binding molecules. As virus binding molecules to be attached we envision antibodies, antibody fragments or aptamers, all of which can be made in principle without detailed knowledge about the target virus, and none of which need to have any a priori virus-neutralizing function. This is because in our concept the shell material, rather than the moieties directly contacting the virus, will prevent access to viral surfaces. Hence, with our concept any virus binder, including non-neutralizing antibodies, weakly binding aptamers, or other molecules such as heparin that binds a broad variety of different virus classes (12), can be utilized as an interior coating on the shell to create an effective antiviral agent.

Here, we report design principles for creating thick and robust macromolecular shells with user-defined sizes that are capable of accommodating entire viruses. We accomplished the construction of such artificial virus-sized macromolecular shells with high structural quality and high production yield. We have further modified our designs to build defined partial shells that feature openings for engulfing target viruses. We used hepatitis B virus (HBV) core particles to demonstrate the feasibility of our virus-trapping and interaction-blocking concept. This paper also describes routes for further expanding the functionality of the shells to create "smarter" antigen-responsive agents.

Briefly, construction of our macromolecular shells relies on several theoretical concepts. First, self-assembly of molecular complexes, such as virus capsids, proceeds typically without external guidance or energy input. Therefore, all information about the desired target shell must be encoded by the shape of the shell subunits and by the local interactions between them (13). Protein designers have previously succeeded in creating artificial macromolecular cages (14-17) by combining and modifying natural non-viral protein scaffolds that display suitable oligomerization symmetries. However, these artificially designed protein-cages are much smaller than the vast majority of natural viruses and cannot be easily modified, and thus are not suitable for our purposes. DNA nanoengineers previously created a variety of polyhedral structures based on DNA origami methods by first building vertices, programmed to assemble into different wireframe polyhedra by varying the vertex angle (18). Yields were low, however, because flexibility of the vertices limited specificity for the target structure. Subsequently, we built blocks which we sequentially assembled into more rigid vertices and larger higher-order wireframe polyhedra (19). While the yields for the blocks and the resulting polyhedra were now higher, the sequential stepwise assembly was tedious and the resulting wireframe polyhedra were still far too skeletal-like, that is too full of holes to be effective for covering the surfaces of viruses. In this work, we have finally succeeded building artificial DNA-based shells that demonstrate exquisite controllability, virus-commensurate sizes and symmetries, and high yield of assembly, thus providing a powerful platform to interface with large natural macromolecular assemblies such as viruses.

### Example 1: Shell design principles

Caspar and Klug elucidated the geometric principles that govern the structure of natural viral capsids in 1962 (20) by mapping a 2D triangulated net of protein positions to a 3D surface with positive curvature, through systematic replacement of 12 six-fold vertices with five-fold vertices. According to Casper and Klug theory - which has been expanded recently (21) - the number of distinct environments occupied by proteins within an icosahedral capsid is described by its triangulation number (T-number), which can be computed by a triangular net projection of the arrangement of pentamers and hexamers within an icosahedral capsid (T=h2+hk+k2, Fig. 1A). The total number of proteins required to build a natural capsid is T times sixty. This is because natural protein subunits are, by default, asymmetric and homo-trimerization is minimally required to construct a three-fold symmetric subunit that can assemble into an icosahedral shell with twenty triangular faces (Fig. 1B, left: T=1 minute virus of mice (22)). To build larger capsids for accommodating a larger genome, viruses use more than one capsid protein or capsid proteins that can adopt different conformations. For example, the cowpea chlorotic mottle virus capsid (23) assembles from a total of 180 identical-sequence proteins into a T=3 capsid in which the protein occurs in three different conformations (Fig. 1B, right).

To accommodate the required local symmetries for icosahedral shells, we created rigid pseudo-symmetric triangular subunits (Fig. 1C, left) using the methods of DNA origami (24-29). DNA origami is a biomolecular design method that can create megadalton-scale discrete objects with structurally well-defined 3D shapes (30, 31). The resulting objects can be assembled via hybridization into higher-order polyhedra (18), and into lattices (32-35). Our subunits are designed to self-assemble into higher-order objects through lateral edge-to-edge interactions using base pair stacking (36) instead of hybridization, thereby mimicking lock-and-key interactions in proteins. The lateral edge-to-edge base pair stacking interactions can be modularly activated and de-activated, a capability we exploited for constructing partial shells. Every side of the triangular subunits we created is the equivalent of one protein subunit of a natural capsid. The overall shell shape is controlled by geometric instructions provided by the triangular subunits. These instructions are given by the choice of the particular length of each triangular edge, by a unique topological pattern per edge for controlling the pairwise edge-to-edge interactions within a set of different triangle units, and by a particular choice of the bevel angle of the edges, which will control the effective curvature of the shell. We realized the target bevel angles by tuning the helical connectivity of the triangle edges in the vertices (Fig. 5), which required deviating substantially from default multi-layer DNA origami connectivity rules. We used iterative design with caDNAno (37) paired with elastic-network-guided molecular dynamics simulations (38) to produce candidate design solutions that fulfilled desired geometrical specifications (Fig. 1C).

Since in our system each triangular edge represents one protein, the Casper and Klug triangulation number now gives the number of unique triangular edges required to build a particular shell. Hence, T=1 and T=3 shells may both be built with a single triangle, with three identical edges for T=1 (Fig. 1D, right) and three different edges for a T=3 shell (Fig. 1E, left). A T=4 shell requires two separate triangular subunits, for example, one triangle with three unique edges and another with three identical edges (Fig. 1E, middle). A T=9 shell requires three different triangles, each having three unique edges (Fig. 1E, right). The greater the T number, the greater the overall number of triangles per target shell, given by 20T. We used design solutions in which all triangle bevel angles for a particular target shell were the same. While T=9 was the largest shell we set out to build, we also designed triangular subunits for a smaller octahedral container ("O") (Fig. 1D, left).

### Example 2: Subunit and shell structures

We encoded different triangle designs in DNA sequences (see methods below), produced the corresponding sets of oligonucleotides, and self-assembled the triangle variants in one-pot mixtures according to previously described procedures (39). Gel-electrophoretic folding quality analysis demanded some design iterations to improve triangular subunit assembly yields (Fig. 5). To validate the 3D structures of the designed triangles, we studied all triangle subunits using cryo transmission electron microscopy (cryo-EM) single particle analysis (Fig. 2). The resulting 3D electron maps had resolutions ranging from 13 to 22 Angstroms (Figs 6-13), which allowed us to evaluate the overall 3D shapes, the bevel angles, the correct positioning of all helices, the correct shape of the designed lateral protrusions and recesses for edge-to-edge docking, and the occurrence of systematic folding defects. For instance, one of the triangles for the T=9 shell (Thex1) had a systematic structural defect at one of its vertices which decreased its ability to form lateral edge-to-edge interactions (Fig. 14). Based on the cryo-EM data, we refined the design and eliminated the defect.

We then successfully assembled the iteratively improved triangle variants into closed shells having expected dimensions, as confirmed by direct imaging with cryo-EM (Fig. 2A, see Figs 15-19 for additional images). Inspection of individual particles (Fig. 2A) and of 2D class averages (Fig. 2B-F) revealed particles displaying the designed symmetries. For example, the three symmetry axes of the octahedron (4-fold, 3-fold, 2-fold, Fig. 2B) and T=1 shell (5-fold, 3-fold, 2-fold, Fig. 2C) can be clearly seen. The particles of the higher-T-number shells had a more circular appearance due to the higher number of triangles per particles, and the underlying triangular net predicted from the Caspar-and-Klug representation became clearly visible (Fig. 2D-F). Since the individual particles displayed the designed symmetry, we reconstructed 3D maps of the shells by imposing the respective symmetry (Fig. 2B-E). The resulting maps had resolutions ranging from 20 to 40 Angstrom. The resolutions of the octahedron and T=1 shell maps were sufficient to discern individual DNA double helices. For the T=1 shell we also computed a 3D map without imposing any a priori symmetry, and this map was fully icosahedral and superimposed well with the sibling that we reconstructed with imposed symmetry, albeit at less resolution. This work successfully proved the concept of icosahedral symmetry (Fig. 16E).

To elucidate effects of orientational specificity of subunit-subunit interactions, we varied the bevel angle of the T=1 subunits from the ideal geometry (a=20.9°). We designed two additional variants of the T=1 triangle whose bevel angles deviated by +5° or -5° from the icosahedral ideal. The decrease or increase of the bevel angle caused the appearance of larger shell-like structures in addition to T=1 shells or octahedra, respectively (Fig. 20). Based on these data we conclude that the correct target bevel angle must be matched within a range of +-5°.

We also successfully constructed octahedron cryo EM maps with and without imposed symmetry (Fig. 15E). Shell maps that lacked one or multiple triangles were reconstructed separately (Figs 15D, 16D), allowing for quantitative assessment of target quality and yield, and also displayed the designed overall symmetry. We examined the largest T=9 shells with negative stain EM tomography (Fig. 2F, Fig. 21E). Sections through tomograms of assembled T=9 shells show fully closed shells as well as the correct arrangement of pentamers according to the designed T-number (arrows in Fig. 2F and Fig. 21E). The set of shells shown have internal cavities ranging from ∼50 nm to ∼280 nm. This size range corresponds to the dimensions of many viral pathogens.

### Example 3: Shell yield and stability

Low-density gel electrophoretic mobility analysis (Fig. 3A, Fig. 22) revealed that shell assembly proceeded by disappearance of the triangular monomers, appearance of a smear indicating the presence of oligomeric species, followed by emergence of a dominant high intensity band, which in all cases corresponded to the fully formed shell. Octahedron and T=1 shells formed within 15 and 60 minutes, respectively, which is sufficiently fast to enable self-assembly of these shells directly during the one-pot triangle-folding reaction (Fig. 24). Octahedra formed with a final complete shell yield of ∼95%. The T=1 shells formed with up to 70% yield. The T=3 and T=4 shells formed with about 40% yield (Fig. 3A). Overall, with molecular masses ranging from 43 to 925 Megadaltons (8 to 180 triangle subunits, depending on the design), our shell assemblies were not only substantially larger than any other previously reported one-pot-assembled artificial macromolecular assembly with defined size, but our shells also formed with orders-of-magnitude improved yields compared to previously built nucleic acid polyhedra (18, 19).

Subunit-exchange experiments with fluorescently labeled subunits revealed that under shell-favoring conditions triangles that are incorporated in closed shells do not exchange with solution (Fig. 3B, Fig. 23A,B). Under equilibrium conditions (Fig. 3B, Fig. 23C), triangles do exchange. For the intended application, shells should be stable without subunit turnover in physiological fluids, which we achieved through post-assembly stabilization. For example, we first assembled shells at high ionic strength conditions where the base pair stacking is effective, followed by covalent photo-crosslinking of the edge-to-edge stacking contacts using strategically placed cyanovinylcarbazole (CNVK) moieties located on the triangle subunit edges, which form covalent inter-triangle crosslinks upon illumination with 365 nm light (Fig. 3C) (40). Gel-electrophoretic mobility analysis of UV-illuminated and CNVK modified shells revealed that non-illuminated shells fell apart under the low-salt electrophoresis conditions, whereas illuminated shells remained stable, thus confirming the covalent inter-triangle bonding (Fig. 3C). In order to move to physiological conditions, we also successfully tested stabilizing the shell triangle subunits with internal covalent bonds via UV point welding as previously described (41). Additionally coating the shells with a mixture of oligolysine and PEG oligolysine (42) allowed us to transfer the shells into mouse serum, where the shells remained intact (Fig. 3D).

### Example 4: Trapping and neutralizing viruses

We envision multiple strategies for using our shell system to block viral surfaces from potential virus-cell interactions. One strategy consists in self-assembling protective shells from triangular building blocks on the surface of viruses. A second strategy is to coordinate and engulf a single virus by two opposing half-shells, while the virus is in the middle. A third strategy consists in swallowing viruses within pre-made shells featuring sufficiently large entry portals, similar to how a pitcher plant captures its prey. We demonstrate the latter two strategies. To accomplish this task, we designed new variants of the triangle subunits to steer self-assembly toward forming user-defined shell fragments, including half octahedron (Fig. 4A), half T=1 shells (Fig. 4B), and "trap" T=1 shells with a missing pentagon vertex (Fig. 4C). In order to prepare half instead of complete octahedron, we deactivated the base-pair stacking contacts on one of the triangular edges of the corresponding subunit by either strand shortening or by adding unpaired thymidines (Fig. 4A, see Fig. 24A,D). To create half T=1 shells, two triangular subunits are required, one that forms a closed pentamer, and another that docks onto the edges of the pentamer (Fig. 4B, see Fig. 24B,E). To build the T=1 shell variant with a missing pentagon vertex, three triangular subunit variants are required (Fig. 4C, see Fig. 24C,F). The modified shells self-assembled successfully from the triangular building blocks, as validated by cryo EM solution structures (Fig. 4D-F, see Figs. 25-27). The resulting 2D class-averages and the 3D electron density maps revealed the expected structural features. We note that we already included nine sites per triangular edge for anchoring virus binding moieties onto the modified shell subunits, which manifested as "spikes" in the cryo EM maps (Fig. 4D,E, arrows).

To demonstrate the virus trapping capability, we trapped hepatitis B virus core particles (Fig 4G) within each of the above described shell variants. To confer specificity, we conjugated anti-HBc 17H7 (Isotype IgG-2b) antibodies that bind and recognize the particulate viral capsid to the shell interior by hybridization of ssDNA-labeled antibodies to a set of anchor points on the triangle subunits, which we predicted would render the interior of the shells sticky specifically for the target virus. Indeed, when we incubated HBV core particles with the thus-prepared DNA shells in approximately 1:1 stoichiometry, all free virus particles were effectively taken up by our shell particles, as seen by negative-staining TEM imaging (Fig. 28). We did not observe any HBV binding in the absence of HBV antibodies conjugated to the shell interior (Fig. 29A), nor in the presence of antibodies specific for other targets (Fig. 29B). We performed cryo electron microscopy to convince ourselves of the proper capture of the target virus in our shells and reconstructed 3D maps of octahedral and T=1 shells with trapped HBV core particles (Fig. 4H,I, see Figs. 30, 31). These maps in our opinion beautifully demonstrate the feasibility of our proposed virus-trapping system.

For the half-octahedral variant, the majority of particles consisted of two half octahedra coordinating a single HBV core particle (Fig. 4H). We observe the two half-octahedral shells in a variety of relative conformations on the shared HBV core particle (Fig. 30). The cryo-EM map also reveals signatures of the antibodies that connect the DNA shell to the trapped HBV core particle (Fig. 4H, left). Similar antibody signatures may be found in the map with the half-T=1 shell-HBV complex (Fig. 4I, left), albeit at higher density threshold. We did not reconstruct a cryo-EM map of HBV core particles trapped in the T=1 shells missing a pentagon vertex, because the HBV core particle positions varied too much. Instead, negative-stain TEM images demonstrate the capability of these "pitcher-plant"-like shell variants to swallow even more than one HBV core particle (Fig. 4J, see Fig. 28E,F).

Finally, as a proof-of-concept of the virus surface interaction-inhibiting capacity of our shells, we performed in vitro virus blocking assays with HBV-binding antibodies immobilized on a solid phase mimicking a cell surface (Fig. 4K, Fig. 32). We evaluated the extent of HBV core particle binding to the solid phase via binding of an orthogonal HBV core-specific reporter antibody coupled to horseradish peroxidase (HRP). As in conventional enzyme-linked immunosorbent assays, residual HBV core particles that are bound to the surface and that are accessible for binding will be detected via HRP catalyzed production of a colorimetric signal. In the presence of our virus-engulfing shells (half T=1 shells), virus interactions with the solid phase were blocked up to 99%, thus confirming strong binding of the shell to the HBV core and the desired interaction-inhibiting capacity of our shells. Controls of shells without HBV trapping antibody (anti-HBc 17H7), and HBV core particles directly incubated with the trapping antibody (anti-HBc 17H7) without shells all resulted in minimal virus blocking compared to the signal generated by naked HBV core particles that represent 0% virus blocking. Notably, with as few as 4 capture antibodies per shell a virus blocking efficiency of >80% is achieved, demonstrating the potency of our shells to shield the virus from its exterior by steric occlusion.

### Example 5: Expanding shell functionality

Our current shell variants add a -15 nm thick DNA layer around a trapped virus particle (not counting the internal spacing created by the layer of virus-binders), and it is hard to imagine how cell-virus interactions could still take place through such thick envelopes. However, our current designs do feature several apertures, notably the cavities in the triangular subunits, which may permit residual interactions. As a demonstration for a route toward sealing the cavities in the triangular subunits we built a DNA brick having a triangular cross-section roughly corresponding to the dimensions of the triangular cavity in the shell subunits. We anchored the brick via multiple attachment points to the outer surface of a T=1 shell triangle (Fig. 33 for design details, see Fig. 34 for a cryo-EM solution structure of the brick) and partially plugged it into the cavities in the shell subunits. The brick may also act as a spacer that further separates cell surfaces from those of viruses trapped in shells by another 40 nm. The brick can be added to already assembled shells or, alternatively, the triangles and brick units can also first be dimerized, followed by triggering shell assembly. Both routes yield fully assembled "spiky" T=1 shells under the same conditions as for the unmodified T=1 shell. We solved a structure of the spiky T=1 shell using cryo-EM single particle analysis (Fig. 5A, Fig. 35). The resulting map readily overlaps with those of the unmodified T=1 shell, but the central opening of the triangle shell subunits is now blocked by the added channel module (Fig. 35E). The fact that the cavity-plugging with the DNA brick readily worked demonstrates the robustness and structural modularity of our shells. The brick may also be considered as a mimic for previously described DNA-based membrane channels (43) or for any other functional module that one wishes to attach to a shell.

In our virus-trapping experiments (Fig. 4) we mounted up to nine antibodies per triangle; that is, a half T=1 shell could potentially feature 90 virus-binding moieties. While it would be possible to create even more attachment sites, 90 is already a very high number. Some of these sites could also be used to mount additional virus-binding moieties to enhance specificity, or for attaching molecules with proteolytic activity such as trypsin or proteinase K, which could endow our shells with additional virucidal activity by shaving off surface proteins of trapped viruses.

### Discussion

We have successfully experimentally demonstrated a symmetry-based approach for self-assembly of virus-like shells of various sizes with high yield and fidelity. This capability is enabled by rigid building blocks that correspond to patchy particles (44) featuring specific lock-and-key interactions whose geometry can be designed and realized with sub-nanometer precision. The exquisite programmability of the shell building blocks furthermore allows building defined-size partial shells, such as half-shells or shells with entry portals, which we propose as virus-neutralizing agents for trapping and inactivating viruses. We tested this concept with HBV core particles as a model virus particle, and in our experiments, we achieved up to 99% inactivation by engulfing HBV in a surrounding shell.

In our concept all recognition elements are integrated on the nanoparticle rather than relying on cascaded reactions between multiple diffusing components. This makes our concept insensitive to dilution, which is a crucial requirement for potential future biomedical applications. Beyond the proposed application as virus-neutralizing agents for trapping and inactivating viruses, variants of our shells could also be used as antigen-carriers for vaccination, as DNA or RNA carriers for gene therapy or gene modification, as drug delivery vehicles, as protective storage containers, or to build compartmentalized systems such as synthetic organelles. Our shells cover a wide range of internal cavity dimensions and may also offer opportunities as alternative gene delivery vectors that can accommodate genomic information much larger as those that can be delivered with e.g. adeno associated viruses (AAV) (45, 46), which are popular gene therapy vectors. The nucleic acids to be delivered could be combined with proteins or protein complexes rendering e.g. CRISPR/Cas based gene silencing or gene modification approaches safer and more efficient.

Whereas natural viruses assemble from protein subunits, we constructed icosahedral shells that consist purely of DNA, thereby taking viral construction principles from protein chemistry into the realm of nucleic acids. Nucleic acids are durable, available commercially, and easily functionalized and modified as we also showed in this work. By using nucleic acids to engulf viral pathogens, we have opened up a new realm for developing drugs to combat viral threats. Using nucleic-acid based agents potentially confers the advantage of avoiding neutralization, phagocytosis and degradation by pathways of the innate and adaptive immune system targeting protein structures. Recently, methods to produce DNA-based objects at scale (47) have emerged. These methods may help transform our concept for fighting viral diseases into candidate therapeutics for clinical use.

### Methods

### 1. Self-assembly of shell subunits

All self-assembly experiments were performed in standardized "folding buffers" containing x mM MgCl₂ in addition to 5 mM Tris Base, 1 mM EDTA, 5 mM NaCl at pH 8 (FoBx). Single-scaffold-chain DNA origami objects were self-assembled in one-pot folding reactions containing 50 nM scaffold DNA and 200 nM of each staple strand. DNA origami objects containing multiple scaffolds were self-assembled using each scaffold DNA chain at 10 nm concentration and 200 nM of each staple strand. The individual scaffolds were sequence-orthogonal and designed and produced as described previously (48, 49). Folding buffer (FoB20) was used with x = 20 mM MgCl₂. All reaction mixtures were subjected to thermal annealing ramps as detailed in Table 2 in Tetrad (Bio-Rad) thermal cycling devices. Staple strands were purchased from IDT (Integrated DNA Technologies).

**TABLE 2: Temperature ramps and scaffold molecules used for self-assembly of shell building blocks.**

| **Object** | **Denaturation phase temperature (15 min) (°C)** | **Temperature ramp (1°C/1h)** | **Storage temperature (°C)** | **Scaffold** |
|---|---|---|---|---|
| T_octa | 65 | 60-56°C | 20 | M13 8064 |
| T=1 | 65 | 58-54°C | 20 | M13 8064 |
| T=1 -5° | 65 | 58-54°C | 20 | M13 8064 |
| T=1 +5° | 65 | 58-54°C | 20 | M13 8064 |
| T=3 | 65 | 54-52°C | 20 | M13 8064 |
| T=4_iso | 65 | 56-54°C | 20 | M13 8064 |
| T=4_equi | 65 | 58-54°C | 20 | M13 8064 |
| T=9_pent | 65 | 56-52°C | 20 | M13 7249 |
| T=9_hex1 | 65 | 56-52°C | 20 | M13 8064 |
| T=9_hex2 | 65 | 58-54°C | 20 | M13 8064 |
| T_octa_half | 65 | 58-54°C | 20 | M13 8064 |
| T_pent T=1 (2/3 triangles) | 65 | 58-54°C | 20 | M13 8064 |
| T_ring T=1 (2 triangles) | 65 | 56-52°C | 20 | M13 8064 |
| T_ring1 T=1 (3 triangles) | 65 | 56-52°C | 20 | M13 8064 |
| T_ring2 T=1 (3 triangles) | 65 | 56-52°C | 20 | M13 8064 |
| Triangular brick | 65 | 60-44°C | 20 | M13 8064 |

| | | | | |
|---|---|---|---|---|
| (for scaffold/template sequences see Table 1; for staple sequences see Table 1 and Sequence Listing). | | | | |

### 2. Purification of shell subunits and self-assembly of shells

All shell subunits were purified using gel purification and, if necessary, concentrated with ultrafiltration (Amicon Ultra 500 µl with 100 kDa molecular weight cutoff) before self-assembling the subunits into shells. Both procedures were performed as previously described (39) with the following alterations: for gel purification, we used 1.5% agarose gels containing 0.5x TBE and 5.5 mM MgCl₂. For ultrafiltration, the same filter was filled with gel-purified sample multiple times (about 2-5 times, ∼400 µl every step) in order to increase the concentration of objects that are recovered from the filter. Before putting the filter upside down in a new filter tube we performed two washing steps with 1xFoB5 (∼400 µl) to achieve well-defined buffer conditions for the shell assembly. To assemble the purified (and concentrated) shell subunits into shells we adjusted the subunit and MgCl₂ concentrations by adding 1xFoB5 and 1.735 M MgCl₂ in suitable amounts. Typical subunit concentrations were in the range of 5 nM and up to 100 nM (for cryo-EM measurements, see Table 3). Typical MgCl₂ concentrations for shell self-assembly were in the range of 10-40 mM. Shell self-assembly was performed at 40°C. Reaction times were varied depending on the shell type (see Fig. 3A).

### 3. Half shells and HBV core binding

Nine staples on the inside of the triangles were modified with handles with 26 single-stranded bases at the 5' ends (seq.: 'GCAGTAGAGTAGGTAGAGATTAGGCA-oligonucleotide', for design details see Figs. 40, 41, Table 1 and Sequence Listing). The triangles were purified and assembled as described above. Oligonucleotides complementary to the handle-sequence and modified with a thiol group at the 3' end were coupled to the HBcore 17H7 antibody using a Sulfo-SMCC (Sulfosuccinimidyl-4-[N-maleimidomethyl]cyclohexane-1-carboxylate) cross-linker. The product was subsequently purified using the *proFIRE*® from *Dynamic Biosensors.* The DNA modified antibodies were added to the assembled shells and incubated over night at 25°C. HBV core particles were incubated with the modified shells for 1-4 hours at 25°C.

### 4. Octahedron oligolysine stabilization

The octahedral shells were assembled at 35mM MgCl₂ and UV cross-linked as described in (41) for 1h using the Asahi Spectra *Xenon Light source 300W MAX-303.* The shells were incubated in a 0.6:1 ratio with a mixture of K₁₀ oligolysine and K₁₀-PEG_{5K} oligolysine (1:1) for 1h at room temperature as similarly described in (42).

### 5. T=1 shell exterior modification

The T=1 triangle and the triangular brick (Fig. 5A) were dimerized using single stranded DNA sticky ends protruding from the T=1 triangle. The protruding sequences contained three thymidines for flexibility plus 7 base long sequence motifs that were directly complementary to single stranded scaffold domains of the brick (Fig. 42). Dimerization reactions were performed at room temperature overnight using a monomer concentration of 40 nM in the presence of 11 mM MgCl₂.

### 6. Gel electrophoresis

The size distribution of folding reactions or shell assemblies was investigated using agarose gel electrophoresis. For solutions including only shell subunits, we used 1.5% agarose gels containing 0.5xTBE Buffer (22.25 mM Tris Base, 22.25 mM Boric Acid, 0.5 mM EDTA) and 5.5 mM MgCl₂. For solutions including oligomeric assemblies such as shells, an agarose concentration of 0.5% was used. The gel electrophoresis was performed in 0.5xTBE buffers supplemented with the same MgCl₂ concentration as the solutions in which the shells were incubated in. For MgCl₂ concentration larger than 15 mM, a surrounding ice-water bath was used for cooling the gel. The gel electrophoresis was performed for 1.5 to 2 hours at 90 V bias voltage. The agarose gels were then scanned with a Typhoon FLA 9500 laser scanner (GE Healthcare) with a pixel size of 50 µm/pix.

### 7. Negative-staining TEM

Samples were incubated on glow-discharged collodion-supported carbon-coated Cu400 TEM grids (in-house production) for 30 to 120 s depending on structure and MgCl₂ concentration. The grids were stained with 2% aqueous uranyl formiate solution containing 25 mM sodium hydroxide. Imaging was performed with magnifications between 10000x to 42000x. T=3 triangles were imaged on a Phillips CM100 equipped with a AMT 4Mpx CCD camera. All other negative staining data was acquired at a FEI Tecnai T12 microscope operated at 120 kV with a Tietz TEMCAM-F416 camera. TEM micrographs were high-pass filtered to remove long-range staining gradients and the contrast was auto-leveled (Adobe Photoshop CS6). To obtain detailed information on individual particles and investigate successful encapsulation negative stain EM tomography was used as a visualization technique. The grids were prepared as described above, and the tilt series acquired with magnifications between 15000x and 30000x using the FEI Tecnai 120. The stage was tilted from -50° to 50° and micrographs were acquired in 2° increments.

All tilt series were subsequently processed with IMOD (50) to acquire tomograms. The micrographs were aligned to each other by calculating a cross correlation of the consecutive tilt series images. The tomogram is subsequently generated using a filtered back-projection. The Gaussian-Filter used a cutoff between 0.25 and 0.5 and a fall-off of 0.035.

### 8. Cryo electron microscopy

The DNA origami concentrations used for preparing the cryo-EM grids are summarized in Table 3. Samples with concentrations higher than 100 nM were applied to glow-discharged C-flat 1.2/1.3 or 2/1 thick grids (Protochip). Samples containing shells with less than 30 nM monomer concentrations were incubated on glow-discharged grids with an ultrathin carbon film supported by a lacey carbon film on a 400 mesh copper grid (Ted Pella). The concentration of all single triangles was increased above 500 nM with PEG precipitation (39). 1 ml of folding reaction (∼50 nM monomer concentration) was mixed with 1 ml of PEG, centrifuged at 21k rcf for 25 min and re-suspended in 50 to 100 µl 1xFoB5. The DNA origami triangles used for assembling the shells were all gel purified and concentrated with ultrafiltration as described above before increasing the MgCl₂ concentration. Plunge freezing in liquid ethane was performed with a FEI Vitrobot Mark V with a blot time of 1.5 to 2 s, a blot force of -1 and a drain time of 0 s at 22°C and 95% humidity. The samples with less than 100 nM monomer concentrations were incubated on the support layer for 60 to 90 s before blotting. All cryo-EM images were acquired with a Cs-corrected Titan Krios G2 electron microscope (Thermo Fisher) operated at 300 kV and equipped with a Falcon III 4k direct electron detector (Thermo Fisher). We used the EPU software for automated single particle acquisition. See Table 3 for microscope settings for all individual datasets. The defocus for all acquisitions was set to -2 µm. The image processing was done at first in RELION-2 (51) and then later in RELION-3 (52). The recorded movies were subjected to MotionCor2 (53) for movie alignment and CTFFIND4.1 (54) for CTF estimation. After reference-free 2D classification the best 2D class averages, as judged by visual inspection, were selected for further processing. A subset of these particles was used to calculate an initial model. After one to two rounds of 3D classification, the classes showing the most features or completely assembled shells were selected for 3D auto-refinement and post-processing. For the corresponding shells octahedral (O) or icosahedral (I1) symmetry was used for the last two steps.

**TABLE 3: Cryo-EM imaging conditions.**

| **Object** | **Concentration (nM)** | **# of particles** | **# of fractions** | **Dose (e/A^2)** | **Pixel size (A/pix)** | **Resolution of resulting 3D map (A)** | **Symmetry** |
|---|---|---|---|---|---|---|---|
| Octa monomer | 700 | 16524 | 5 | 42.57 | 2.28 | 18.69 | C1 |
| T=1 monomer | 500 | 9496 | 7 | 51.16 | 2.28 | 20.27 | C1 |
| T=3 monomer | 500 | 11080 | 7 | 53.17 | 2.28 | 19.09 | C1 |
| T=4_iso | 500 | 16904 | 7 | 48.53 | 2.28 | 17.22 | C1 |
| T=4_equi | 500 | 34288 | 7 | 48.26 | 2.28 | 21.21 | C1 |
| T=9_pent | 800 | 25053 | 8 | 47.9 | 1.79 | 14.92 | C1 |
| T=9_hex1 | 800 | 38498 | 13 | 36.85 | 1.79 | 12.92 | C1 |
| T=9_hex2 | 800 | 11481 | 8 | 48 | 1.79 | 15.04 | C1 |
| Octa shell (17.5mM) | 130 | 3384 | 11 | 42.71 | 2.28 | 19.64 | O |
| T=1 shell (20 mM) | 110 | 2578 | 10 | 51.11 | 2.28 | 21 | I1 |
| T=1 shell (25 mM) | 50 (lacey carbon grid) | 720 | 7 | 31.26 | 2.28 | 22.21 | I1 |
| T=3 shell (20 mM) | 20 | 612 | - | 22.96 | 3.71 | 36.15 | I1 |
| T=4 shell (25 mM) | 21 (T_iso) 7 (T_equi) | 255 | - | 25 | 3.71 | 47.87 | I1 |
| Octa_half shell (30mM) | 180 | 6801 | 10 | 40.44 | 2.9 | 20.41 | C4 |
| Octa_half shell (30mM) + HBV core | 40 | 2707 | 7 | 44.38 | 2.9 | 23 | C1 |
| T=1 (FRET)_half shell (2 triangles, 30mM) | 180 | 8725 | 10 | 40.44 | 2.9 | 15.16 | C1 |
| T=1 (FRET)_half shell + HBV core (2 triangles, 30mM) | 50 | 1770 | 7 | 44.79 | 2.9 | 23 | C5 |
| T=1 (FRET)_15mer shell (3 triangles, 30mM) | 210 | 3194 | 10 | 29.17 | 2.9 | 22.3 | C5 |
| spiky shell (22.5mM) | 150 | 3847 | 8 (dataset1) 11 (dataset 2) | 25.76 (dataset1) 30.00 (dataset 2) | 3.76 | 22 | I1 |
| triangular brick | 1000 | 38132 | 7 | 78.6 | 2.28 | 11.9 | C1 |

### 9. In vitro virus blocking ELISA

Various concentrations of assembled half-T1 shells were incubated overnight at room temperature with 2 nM oligonucleotide-conjugated capture antibody (anti-HBc 17H7, Isotype IgG-2b) in FoB30-T (FoB30 + 0.05% Tween-20). The next day the pre-incubated mixtures were added to 5 pM HBV core particles and incubated overnight at room temperature, yielding 1 nM capture antibody, 2.5 pM HBV core particle and 0-200 pM half-T=1 shells. A flat-bottom transparent 96 well microplate (Nunc MaxiSorp) was treated overnight at 4 °C with 100 µl/well anti-CAgHB antibody (1 µg/ml in PBS). After washing 4 times with 200 µl/well PBS-T (PBS + 0.05% Tween-20) the well surface was blocked by incubating with 200 µl/well 5% bovine serum albumin in PBS for 2 hours at room temperature. After washing 4 times with 200 µl/well FoB30-T, 90 µl of the pre-incubated samples were added to the wells and incubated for 2 hours at room temperature, followed by washing and subsequent incubation for 1 hour with 100 µl/well horseradish peroxidase conjugated detection antibody (anti-CAgHB-HRP in FoB30-T). After washing with FoB30-T, 100 µl/well HRP substrate (3,3',5,5'-Tetramethylbenzidine, Life Technologies) was added and product formation was monitored in time by measuring the absorbance at 650 nm with a 60 s interval in a plate reader pre-equilibrated to 30 °C (CLARIOstar, BMG LABTECH). HRP activity was calculated by fitting linear regression slopes to the linear regime of the kinetic data (typically the first 5 min). Virus blocking efficiency was calculated relative to a control of HBV core particles only and blank measurements. All experiments were performed in triplicates.

Antibodies used for the ELISA were kindly provided by Centro De Ingenieria Genetica y Biotecnologia de sancti spiritus in Cuba.

### 10. Helium Ion Microscopy (HIM)

Imaging was performed with negative-stained TEM grids coated with a 5 nm layer of AuPd using a Quorum Q150T sputter coater in ORION Nanofab (Zeiss). We used an acceleration voltage of 30 kV and a beam current of 0.3 to 0.4 pA. The images were acquired in scanning mode with an Everhart-Thornley 2k detector.

### 11. Production of HBV core particles

Hepatitis B virus core particles of genotype D (subtype ayw2) were produced recombinantly in *E. coli* K802 and BL21 cells (purchased from the Latvian Biomedical Research and Study Centre, Riga, Latvia). Briefly, particles were obtained by sonication and clarification from bacterial protein extracts and purified by ammonium sulphate precipitation and subsequent anion exchange and size exclusion chromatography as described (55). Final preparations were constantly kept at 4°C in the dark in conventional PBS (including 0.05% NaN₃, 1 mM DTT).

### 12. Production of Anti-HBc antibody

Anti-HBV core (anti-HBc) antibody 17H7 (Isotype IgG-2b) was produced by the Monoclonal Antibody Core Facility at Helmholtz Zentrum München in Munich (HMGU). Briefly, mouse HBc-recognizing B cells were generated by common hybridoma technology. The mice were challenged with the peptide NLEDPASRDLVVC (aa 75-86 of HBV core). Mouse hybridoma clones were selected, and secreted antibodies were analyzed by immune staining and precipitation of HBcAg and ELISA for native antigen recognition and by Western Blot analysis for detection of denatured antigen. Final 17H7 preparations were purified via standard affinity chromatography using a protein A/G column and concentrated to 0.8 mg/ml (5.33 µM) of protein and kept in conventional PBS (137 mM NaCl, 10 mM Phosphate, 2.7 mM KCI, pH 7.4) at 4°C in the dark.

### REFERENCES

1. C. Gortazar et al., Crossing the interspecies barrier: opening the door to zoonotic pathogens. PLoS pathogens 10, e1004129 (2014).
2. S. S. Morse et al., Prediction and prevention of the next pandemic zoonosis. Lancet 380, 1956-1965 (2012).
3. J. J. O'Brien, D. M. Campoli-Richards, Acyclovir. An updated review of its antiviral activity, pharmacokinetic properties and therapeutic efficacy. Drugs 37, 233-309 (1989).
4. J. LaBonte, J. Lebbos, P. Kirkpatrick, Enfuvirtide. Nat Rev Drug Discov 2, 345-346 (2003).
5. W. L. Davies et al., Antiviral Activity of 1-Adamantanamine (Amantadine). Science 144, 862-863 (1964).
6. Y. K. Gupta, M. Meenu, P. Mohan, The Tamiflu fiasco and lessons learnt. Indian J Pharmacol 47, 11-16 (2015).
7. J. M. Steichen et al., A generalized HIV vaccine design strategy for priming of broadly neutralizing antibody responses. Science 366, (2019).
8. K. O. Saunders et al., Targeted selection of HIV-specific antibody mutations by engineering B cell maturation. Science 366, (2019).
9. B. K. Ganser-Pornillos, O. Pornillos, Restriction of HIV-1 and other retroviruses by TRIM5. Nat Rev Microbiol 17, 546-556 (2019).
10. K. A. Skorupka et al., Hierarchical assembly governs TRIM5alpha recognition of HIV- 1 and retroviral capsids. Sci Adv 5, eaaw3631 (2019).
11. M. Mammen, S. K. Choi, G. M. Whitesides, Polyvalent Interactions in Biological Systems: Implications for Design and Use of Multivalent Ligands and Inhibitors. Angew Chem Int Ed Engl 37, 2754-2794 (1998).
12. V. Cagno, E. D. Tseligka, S. T. Jones, C. Tapparel, Heparan Sulfate Proteoglycans and Viral Attachment: True Receptors or Adaptation Bias? Viruses 11, (2019).
13. F. H. Crick, J. D. Watson, Structure of small viruses. Nature 177, 473-475 (1956).
14. J. B. Bale et al., Accurate design of megadalton-scale two-component icosahedral protein complexes. Science 353, 389-394 (2016).
15. N. P. King et al., Accurate design of co-assembling multi-component protein nanomaterials. Nature 510, 103-108 (2014).
16. Y. T. Lai et al., Structure of a designed protein cage that self-assembles into a highly porous cube. Nature chemistry 6, 1065-1071 (2014).
17. G. L. Butterfield et al., Evolution of a designed protein assembly encapsulating its own RNA genome. Nature 552, 415-420 (2017).
18. R. linuma et al., Polyhedra self-assembled from DNA tripods and characterized with 3D DNA-PAINT. Science 344, 65-69 (2014).
19. K. F. Wagenbauer, C. Sigl, H. Dietz, Gigadalton-scale shape-programmable DNA assemblies. Nature 552, 78-83 (2017).
20. D. L. Caspar, A. Klug, Physical principles in the construction of regular viruses. Cold Spring Harbor symposia on quantitative biology 27, 1-24 (1962).
21. R. Twarock, A. Luque, Structural puzzles in virology solved with an overarching icosahedral design principle. Nature communications 10, 4414 (2019).
22. A. L. Llamas-Saiz et al., Structure determination of minute virus of mice. Acta Crystallogr D Biol Crystallogr 53, 93-102 (1997).
23. J. A. Speir, S. Munshi, G. Wang, T. S. Baker, J. E. Johnson, Structures of the native and swollen forms of cowpea chlorotic mottle virus determined by X-ray crystallography and cryo-electron microscopy. Structure 3, 63-78 (1995).
24. P. W. K. Rothemund, Folding DNA to create nanoscale shapes and patterns. Nature 440, 297-302 (2006).
25. S. M. Douglas et al., Self-assembly of DNA into nanoscale three-dimensional shapes. Nature 459, 414-418 (2009).
26. C. E. Castro et al., A primer to scaffolded DNA origami. Nature methods 8, 221-229 (2011).
27. R. Veneziano et al., Designer nanoscale DNA assemblies programmed from the top down. Science 352, 1534 (2016).
28. E. Benson et al., DNA rendering of polyhedral meshes at the nanoscale. Nature 523, 441-444 (2015).
29. K. E. Dunn et al., Guiding the folding pathway of DNA origami. Nature 525, 82-86 (2015).
30. X. C. Bai, T. G. Martin, S. H. Scheres, H. Dietz, Cryo-EM structure of a 3D DNA-origami object. Proceedings of the National Academy of Sciences of the United States of America 109, 20012-20017 (2012).
31. J. J. Funke, H. Dietz, Placing molecules with Bohr radius resolution using DNA origami. Nature nanotechnology 11, 47-52 (2016).
32. R. Jungmann et al., DNA origami-based nanoribbons: assembly, length distribution, and twist. Nanotechnology 22, 275301 (2011).
33. W. Liu, H. Zhong, R. Wang, N. C. Seeman, Crystalline two-dimensional DNA-origami arrays. Angewandte Chemie 50, 264-267 (2011).
34. Y. Suzuki, M. Endo, H. Sugiyama, Lipid-bilayer-assisted two-dimensional self-assembly of DNA origami nanostructures. Nature communications 6, 8052 (2015).
35. Y. Ke et al., DNA brick crystals with prescribed depths. Nature chemistry 6, 994-1002 (2014).
36. T. Gerling, K. F. Wagenbauer, A. M. Neuner, H. Dietz, Dynamic DNA devices and assemblies formed by shape-complementary, non-base pairing 3D components. Science 347, 1446-1452 (2015).
37. S. M. Douglas et al., Rapid prototyping of 3D DNA-origami shapes with caDNAno. Nucleic acids research 37, 5001-5006 (2009).
38. C. Maffeo, J. Yoo, A. Aksimentiev, De novo reconstruction of DNA origami structures through atomistic molecular dynamics simulation. Nucleic acids research 44, 3013-3019 (2016).
39. K. F. Wagenbauer et al., How we make DNA origami. Chembiochem : a European journal of chemical biology, (2017).
40. T. Gerling, H. Dietz, Reversible Covalent Stabilization of Stacking Contacts in DNA Assemblies. Angewandte Chemie 58, 2680-2684 (2019).
41. T. Gerling, M. Kube, B. Kick, H. Dietz, Sequence-programmable covalent bonding of designed DNA assemblies. Sci Adv 4, eaau1157 (2018).
42. N. Ponnuswamy et al., Oligolysine-based coating protects DNA nanostructures from low-salt denaturation and nuclease degradation. Nature communications 8, 15654 (2017).
43. M. Langecker et al., Synthetic lipid membrane channels formed by designed DNA nanostructures. Science 338, 932-936 (2012).
44. N. Kern, D. Frenkel, Fluid-fluid coexistence in colloidal systems with short-ranged strongly directional attraction. J Chem Phys 118, 9882-9889 (2003).
45. M. F. Naso, B. Tomkowicz, W. L. Perry, 3rd, W. R. Strohl, Adeno-Associated Virus (AAV) as a Vector for Gene Therapy. BioDrugs 31, 317-334 (2017).
46. D. Wang, P. W. L. Tai, G. Gao, Adeno-associated virus vector as a platform for gene therapy delivery. Nat Rev Drug Discov 18, 358-378 (2019).
47. F. Praetorius et al., Biotechnological mass production of DNA origami. Nature 552, 84-87 (2017).
48. F. A. S. Engelhardt et al., Custom-Size, Functional, and Durable DNA Origami with Design-Specific Scaffolds. ACS nano, (2019).
49. B. Kick, F. Praetorius, H. Dietz, D. Weuster-Botz, Efficient Production of Single-Stranded Phage DNA as Scaffolds for DNA Origami. Nano letters, (2015).
50. J. R. Kremer, D. N. Mastronarde, J. R. McIntosh, Computer visualization of three-dimensional image data using IMOD. Journal of structural biology 116, 71-76 (1996).
51. D. Kimanius, B. O. Forsberg, S. H. Scheres, E. Lindahl, Accelerated cryo-EM structure determination with parallelisation using GPUs in RELION-2. Elife 5, (2016).
52. J. Zivanov et al., New tools for automated high-resolution cryo-EM structure determination in RELION-3. Elife 7, (2018).
53. S. Q. Zheng et al., MotionCor2: anisotropic correction of beam-induced motion for improved cryo-electron microscopy. Nature methods 14, 331-332 (2017).
54. A. Rohou, N. Grigorieff, CTFFIND4: Fast and accurate defocus estimation from electron micrographs. Journal of structural biology 192, 216-221 (2015).
55. Sominskaya I, Skrastina D, Petrovskis I, Dishlers A, Berza I, et al. (2013) A VLP library of Cterminally truncated Hepatitis B core proteins: correlation of RNA encapsidation with a Th1/Th2 switch in the immune responses of mice. PLoS One 8: e75938.
56. P. S. Kwon et al., Designer DNA architecture offers precise and multivalent spatial pattern-recognition for viral sensing and inhibition. Nat. Chem. 12, 26-35 (2020).

## Claims

1. A macromolecule-based nanostructure having a molecular mass of at least 1 MDa, encasing a cavity with a diameter of at least 20 nm for encapsulating a virus or viral particle, in particular wherein said macromolecule-based nanostructure is a DNA-based nanostructure.

2. The macromolecule-based nanostructure of claim 1, wherein said macromolecule-based nanostructure is a DNA-based nanostructure formed by self-assembling DNA-based building blocks, in particular wherein each of said self-assembling DNA-based building blocks is formed by a single-stranded DNA template strand and a set of oligonucleotides complementary to said single-stranded DNA template, wherein each of said oligonucleotides is either complementary to one contiguous DNA sequence stretch or to at least two non-contiguous DNA sequence stretches on said single-stranded DNA template.

3. The macromolecule-based nanostructure of claim 1 or 2, which is a closed three-dimensional geometric shape, in particular a closed three-dimensional geometric shape selected from a sphere, a spherocylinder, and a polyhedron, in particular a tetrahedron, an octahedron or an icosahedron, formed in situ in the presence of said virus or viral particle to be encapsulated, in particular formed in situ from said self-assembling DNA-based building blocks in the presence of said virus or viral particle to be encapsulated.

4. The macromolecule-based nanostructure of claim 1 or 2, which is a shell with an opening for accessing said cavity.

5. The macromolecule-based nanostructure of claim 1 or 2, which is a combination of a first and a second shell with an opening to access a first and a second inner cavity, respectively, wherein said first and said second inner cavity together form said cavity, in particular wherein said first and said second shell are connected by at least one linker.

6. The macromolecule-based nanostructure of any one of claims 1 to 5, which is based on an icosahedral structure.

7. The macromolecule-based nanostructure of claim 6, wherein said macromolecule-based nanostructure is a DNA-based nanostructure formed by self-assembling DNA-based building blocks, wherein each of said self-assembling DNA-based building blocks is a triangular and/or a rectangular prismoid, particularly a triangular prismoid.

8. The macromolecule-based nanostructure of claim 7,
wherein each said triangular and/or a rectangular prismoid is formed by m triangular, or rectangular, respectively, planes, wherein m is an integer independently selected from 4, 5, 6, 7 and 8, in particular independently selected from 5, 6 and 7, more particularly wherein said integer is 6,
the three, or four, respectively, edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4,
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes, and
wherein at least two of the three, or four, respectively, side trapezoids comprises a specific pattern of recesses and/or extrusions formed by missing or additional DNA double helical stretches for specific interaction with a complementary pattern on the side trapezoid of another one of said self-assembling DNA-based building blocks.

9. The macromolecule-based nanostructure of claim 8, wherein for at least part of said self-assembling DNA-based building blocks the length of at least one edge of each of said m planes is decreasing from the first to the m^{th} plane, so that bevel angles results between planes perpendicular to said first plane and each of the trapezoid planes formed by said m edges.

10. The macromolecule-based nanostructure of claim 8 or 9 comprising at least one set of self-assembling DNA-based building blocks, wherein all three, or four, respectively, side trapezoids comprises a specific pattern of recesses and/or extrusions formed by missing or additional DNA double helical stretches for specific interaction with a complementary pattern on the side trapezoid of another one of said self-assembling DNA-based building blocks.

11. The macromolecule-based nanostructure of claim 8 or 9, wherein the side trapezoids of the self-assembling DNA-based building blocks forming the rim of said shell, or of said first and second shell, respectively, do not comprises a specific pattern of recesses and/or extrusions formed by missing or additional DNA double helical stretches for specific interaction with a complementary pattern on the side trapezoid of another one of said self-assembling DNA-based building blocks.

12. The macromolecule-based nanostructure of any one of claims 1 to 11, wherein said macromolecule-based nanostructure is a DNA-based nanostructurere, which is a shell selected from
(a) a half octahedron, which consists of a set of four copies of a triangular frustum, wherein the base-pair stacking contacts on one of the triangular edges of the triangular frustum are inactivated by either strand shortening or by adding unpaired thymidines;
(b) a half T=1 shell, which consists of two sets of in each case five copies of two different triangular frusta, wherein the five copies of the first set form a closed pentamer, and the five copies of the second set dock onto the edges of said pentamer; and
(c) a "trap" T=1 shell with a missing pentagon vertex, which consists of three sets of in each case five copies of three different triangular frusta, wherein the five copies of the first set form a closed pentamer, the five copies of the second set dock onto the edges of said pentamer the five copies of the second set dock onto the edges of said pentamer, and the five copies of the third set dock into the gaps between the five copies of said second set.

13. The macromolecule-based nanostructure of any one of claims 7 to 12, further comprising
(a) one or more types of DNA brick constructs, each type of such DNA brick constructs being **characterized by** one or more interaction sites for specific interaction by edge-to-edge stacking contacts with one or more complementary interaction sites present on the plane of a triangular or rectangular frustum on the outer surface of said DNA-based nanostructure, wherein said DNA brick constructs cover the free space between the three, or four, respectively, edges of said plane;
(b) one or more cross-linkages within one of said triangular or rectangular prismoids, and/or between two of said triangular and/or rectangular prismoids; and/or
(c) at least one moiety specifically interacting with said virus or viral particle.

14. A composition comprising a virus or viral particle encapsulated by a macromolecule-based according to any one of claims 1 to 13.

15. A method for encapsulating a virus or viral particle, comprising the steps of: providing a macromolecule-based nanostructure according to any one of claims 1 to 13, and contacting said macromolecule-based nanostructure with a medium comprising, or suspected to comprise, said virus or viral particle.
